# EUROPEAN PATENT SPECIFICATION

(11) **EP 2 192 199 B1**
(45) Date of publication and mention of the grant of the patent: **14.03.2012**
(21) Application number: 09015797.5
(22) Date of filing: 08.12.2006
(51) Int. Cl.: C12Q 1/70, C12Q 1/68

(54) **METHOD OF DETECTING HUMAN PAPILLOMA VIRUS BY USING NUCLEIC ACID AMPLIFICATION METHOD AND NUCLEIC ACID CHAIN-IMMOBILIZED CARRIER**
VERFAHREN ZUR ERKENNUNG DES MENSCHLICHEN PAPILLOMA-VIRUS DURCH VERWENDUNG DES NUKLEINSÄURE-AMPLIFIKATIONSVERFAHRENS UND NUKLEINSÄURENTRÄGER MIT IMMOBILISIERTEN KETTEN
PROCEDE DE DETECTION DU PAPILLOMAVIRUS HUMAIN PAR LE PROCEDE D'AMPLIFICATION D'ACIDE NUCLEIQUE ET SUPPORT IMMOBILISE DE CHAINE D'ACIDE NUCLEIQUE

(30) Priority: 08.12.2005 JP 2005354826; 07.07.2006 JP 2006187871; 15.11.2006 WO PCT/JP2006/323261
(43) Date of publication of application: 02.06.2010
(62) Divisional of application: 06834753.3
(73) Proprietor: Kabushiki Kaisha Toshiba, Minato-ku, Tokyo 105-8001 (JP); Sekisui Medical Co., Ltd., Tokyo 103-0027 (JP)
(72) Inventor: Hashimoto, Koji, Tokyo 105-8001 (JP); Ito, Keiko, Tokyo 105-8001 (JP); Nakamura, Naoko, Tokyo 105-8001 (JP); Horiuchi, Hideki, Tokyo 105-8001 (JP); Hashimoto, Michie, Tokyo 105-8001 (JP); Sato, Osamu, Tokyo 103-0027 (JP)
(74) Representative: Lord, Hilton David

(56) References cited:
- WO-A-2005/056839
- NAGAMINE KENTARO ET AL: "Isolation of single-stranded DNA from loop-mediated isothermal amplification products" BIOCHEMICAL AND BIOPHYSICAL RESEARCH COMMUNICATIONS, ACADEMIC PRESS INC. ORLANDO, FL, US LNKD- DOI:10.1006/BBRC.2001.6334, vol. 290, no. 4, 1 February 2002 (2002-02-01), pages 1195-1198, XP002312265 ISSN: 0006-291X
- NAGAMINE K ET AL: "Accelerated reaction by loop-mediated isothermal amplification using loop primers." MOLECULAR AND CELLULAR PROBES JUN 2002, vol. 16, no. 3, June 2002 (2002-06), pages 223-229, XP004471001 ISSN: 0890-8508
- TAKAHASHI MASAYOSHI ET AL: "CONSTRUCTION OF AN ELECTROCHEMICAL DNA CHIP FOR SIMULTANEOUS GENOTYPING OF SINGLE NUCLEOTIDE POLYMORPHISMS" ANALYST, ROYAL SOCIETY OF CHEMISTRY, LONDON, GB, vol. 130, no. 5, May 2005 (2005-05), pages 687-693, XP009082052 ISSN: 0003-2654
- HAGIWARA MASANORI ET AL: "Loop-mediated isothermal amplification method for detection of human papillomavirus type 6, 11, 16, and 18." JOURNAL OF MEDICAL VIROLOGY MAY 2007, vol. 79, no. 5, May 2007 (2007-05), pages 605-615, XP002429535 ISSN: 0146-6615

## Description

### Technical Field

The present invention relates to a nucleic acid primer sequence, a kit, and a nucleic acid chain-immobilized carrier for detection of human papilloma virus and identification of its genotype, and a method of detecting human papilloma virus by using a nucleic acid amplification method.

### Background Art

Human papilloma virus (HPV) infection was reported as a cause of uterine cervical cancer in the 1980's, and in particular, the relationship between cancer malignancy and HPV genotype is attracting attention. HPV is also considered to be the cause of cancers other than uterine cervical cancer such as cancers of the genital organs and oral mucosa, and there has been a demand for a rapid and accurate method of detecting HPV. Hitherto known were a method of detecting a malignant or benign genotype by using a DNA/RNA-recognizing antibody, and a method of amplifying a region containing a sequence characteristic to a genotype in polymerase chain reaction (PCR) and identifying the genotype finally by using a genotype-specific probe. However, the former method, which does not identify the genotype, is not applicable to the test for vaccine administration currently under development. Alternatively, the latter method of using the PCR method had disadvantages such as complicated procedure of pretreatment for example nucleic acid extraction, demand for a complex temperature-regulating device such as thermal cycler, and longer reaction period of two hours or more. In addition, the PCR method has a possibility that, if an incorrect complementary strand happens to be synthesized, the product may be used as a template in amplification, consequently leading to incorrect judgment. Actually, it is difficult to control specific amplification only with a difference of one nucleotide at the terminal of a primer.

For detection by using a DNA chip, gene products amplified by the PCR method are generally double-stranded chains. Thus, there emerged a problem that the complementary strands became competitors to the probe, lowering hybridization efficiency and detection sensitivity in the hybridization reaction with a probe. Accordingly, for example, a method of decomposing or separating the complementary strand is employed to make the target gene product into a single strand. However, these methods still have problems such as the higher cost and complicated procedure because of the use of enzymes or magnetic beads, and there exists a need for a new method replacing such conventional methods.

### Disclosure of Invention

An object of the present invention is to provide a nucleic acid primer for detection of an HPV nucleotide sequence present in LAMP amplification products when principle of a LAMP method allowing simple and rapid detection of nucleic acids is applied, and an HPV-detection method using the nucleic acid primer.

The inventions employed a method different from the PCR method, i.e., LAMP method, for identification of the HPV genotype.

Furthermore, the additional use of Loop primers, as described by Nagamine et al. (Molecular and cellular probes [2002]), has provided an accelerated Lamp reaction. Thus, it is possible to identify the genotype easily. However, the LAMP products, which have complicated high-order structures, cause physical hindrance with the probe-bound support during hybridization, which in turn lead to deterioration of the hybridization efficiency (see, for example, JP-A 2005-095043(KOKAI)). Accordingly, in the present invention, the primer is so designed that the human papilloma virus-derived target sequence becomes located in the single-stranded loop region of the LAMP product, differently from before.

The invention is defined in the accompanying claims.

### Brief Description of Drawings

FIG. 1 is a schematic chart showing an amplification method in a conventional LAMP method;
FIG. 2 is a schematic chart showing amplification products obtained by the conventional LAMP method;
FIG. 3 is a schematic chart showing an amplification method for producing a nucleic acid for measurement according to the present invention;
FIG. 4 is a schematic chart showing nucleic acids for measurement according to the present invention;
FIG. 5 is a schematic view illustrating an example of a DNA chip for identification of HPV genotype;
FIG. 6 is a schematic view illustrating another example of the DNA chip for identification of HPV genotype;
FIG. 7 is a chart showing an example of electrophoretic photographs after LAMP amplification;
FIG. 8 is a chart showing another example of the electrophoretic photographs after LAMP amplification;
FIG. 9 includes charts showing examples of results detected by a current-detecting DNA chip;
FIG. 10 include charts showing examples of the electrophoretic photographs after LAMP amplification; and
FIG. 11 is a chart showing an HPV sequence and regions usable as a primer or probe according to the present invention.

### Best Mode for Carrying Out the Invention

An amplification method used in the present invention, the "LAMP method", is a kind of isothermal polymerase chain reaction, and uses 4, 5 or 6 kinds of primers. The LAMP method is reported to be higher in amplification efficiency than the PCR method and also resistant to the influence by impurities in a sample. It is thus possible to detect human papilloma virus in a smaller amount easily with simple pretreatment of the sample.

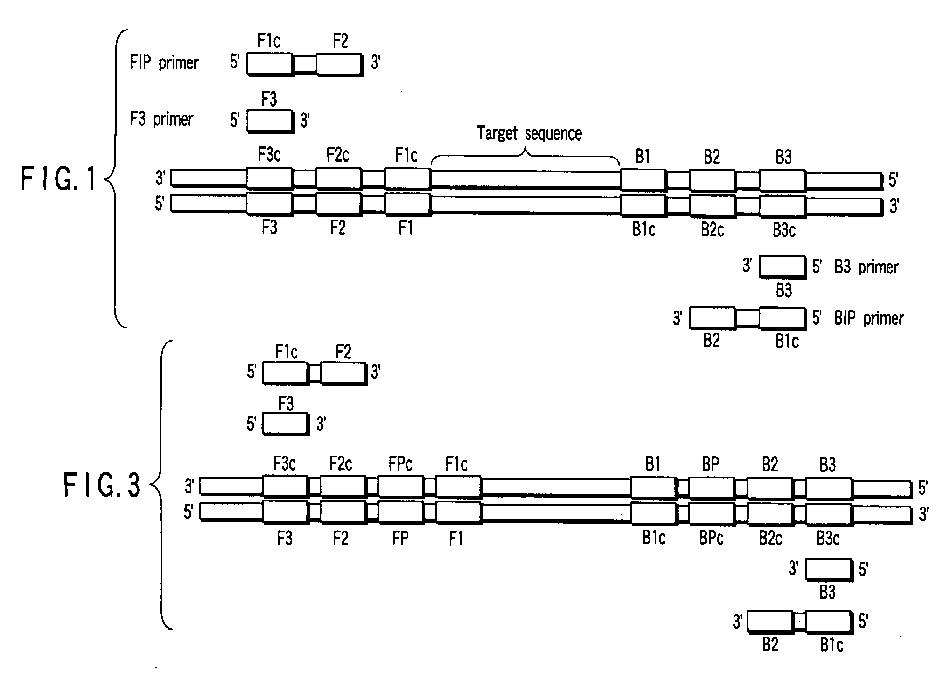

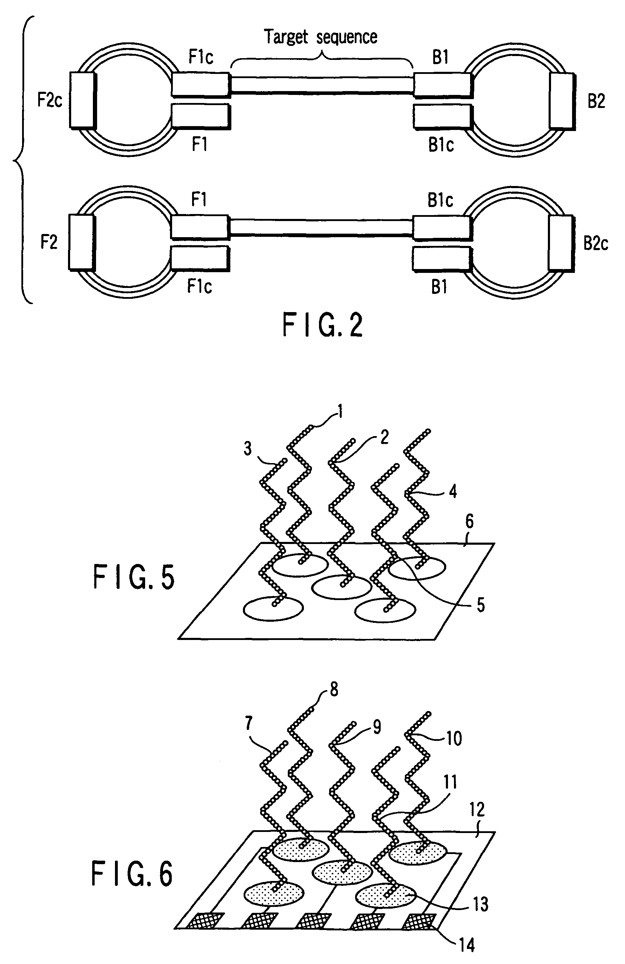

The primer design and amplification products obtained in the LAMP method will be described with reference to FIGS. 1 and 2. FIG. 1 shows a double strand DNA to be detected. Conventionally, a target sequence has been located in the center of a stem-and-loop structure of LAMP amplification products (FIG. 2). In amplifying and detecting the target sequence, a total of four kinds of primer sequences (FIP, F3, BIP, and B3 primers) are determined from the sequences located at both sides of the target sequence. The FIP and BIP primers each contain two regions (FIP = F1c + F2, BIP = B2 + B1c). A total of six regions used in these primers will be called primer regions below. LAMP amplification by using the four kinds of primers gives amplification products with the dumbbell-shaped stem-and-loop structure shown in FIG. 2, each of them being complementary to each strand of the DNA shown in FIG. 1. The amplification mechanism is not described here, but may be referred, for example, to in JP-A 2002-186781(KOKAI).

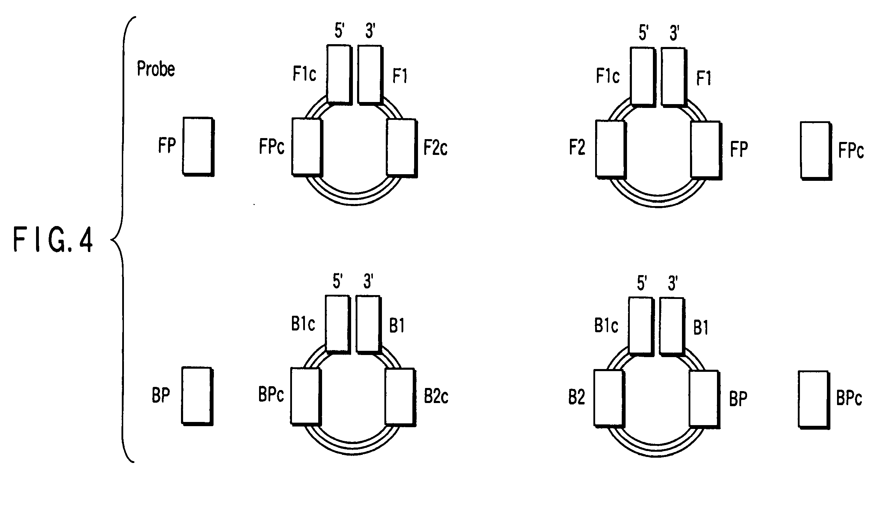

On the other hand, in the present invention, the primers are designed such that the target sequence is placed in the single-stranded loop region, unlike the conventional target-sequence site shown in FIG. 2. Specifically as shown in FIG. 3, in the invention, six primer regions are so placed that the target sequence (any one of FPc, FP, BP, and BPc in FIG. 3) is located between primer regions F1 and F2 (including F2 region), between primer regions F2c and F1c (including F2c region), between primer regions B1 and B2 (including B2 region), and/or between primer regions B2c and B1c (including B2c region). The target sequence may be placed in any one of the single-stranded loop regions formed between the regions above, and thus, the loop region between primer regions F1 and F2 includes the F2 region. A part of LAMP amplification products, which is shown in FIG. 4, are obtained by preparing four kinds of primers according to the six primer regions thus determined and performing LAMP amplification by using these primers. In the LAMP amplification products, target sequences FPc, FP, BP, and BPc are located in the single-stranded loops in the dumbbell structure of the amplification products. On the other hand, primer regions F1c and F1, and B1c and B1, have sequences complementary to each other, and thus, form double strands by selfhybridization. Some of the target sequences contained in the amplification products are in the single stranded state as shown in FIG. 4. For this reason, it is possible to detect the target sequences by specific hybridization to probe nucleic acids (FP, FPc, BP, and BPc) complimentary to respective target sequences without denaturation processing, as shown in the figure. The term "specific hybridization" means that it is possible to detect a slight difference caused by single nucleotide polymorphism (SNP) or mutation if present.

The present invention detects the genotype of HPV virus by applying such a primer structure to HPV virus.

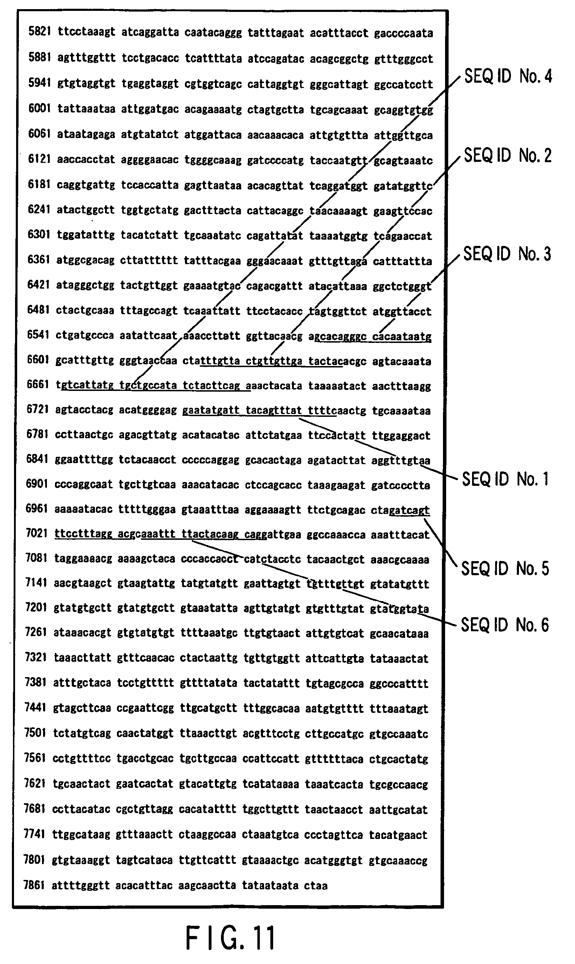

A sequence shown in FIG. 11 is an HPV virus sequence. A region containing SEQ ID Nos. 1, 2 and 3 in the figure is known to be preserved among many HPV viruses. Alternatively, the SEQ ID No. 4 shows a region where it is known that there is polymorphism between malignant and benign tumors. In detecting the HPV viral genotype, polymorphism is detected, for example, by using a sequence of the region corresponding to the SEQ ID No. 4 as a target sequence. In such a case, for example, sequences selected from first sequence group (Table 1) and second sequence group (Table 2), or for example sequences selected from first sequence group and third sequence group (Table 3), are used as the sequence corresponding to the primer regions F1 and F2. The first, second, and third sequence groups are the sequence groups shown in the following Tables 1 to 3, and respectively correspond to the regions of SEQ ID No. 1, 2, and 3 in FIG. 11. The sequence in these regions varies according to its viral type, and is not always identical with the sequence shown in FIG. 11. In addition, a primer set consisting of BIP and B3 primers is also needed in actual LAMP amplification. It is possible to use the sequences of SEQ ID Nos. 5 and 6 in FIG. 11 or the complementary sequences thereof. The primer according to the invention for use is preferably a primer having, on the same chain in the direction from 5' to 3', a sequence complementary to a sequence selected from those in the first sequence group and a sequence selected from those in the second sequence group bound to each other, a sequence selected from those in the second sequence group and a sequence complementary to a sequence selected from those in the first sequence group bound to each other, a sequence complementary to a sequence selected from those in the first sequence group and a sequence selected from those in the third sequence group bound to each other, a sequence selected from those in the third sequence group and a sequence complementary to a sequence selected from those in the first sequence group bound to each other, a sequence complementary to a sequence selected from those in the second sequence group and a sequence selected from those in the third sequence group bound to each other, a sequence selected from those in the third sequence group and a sequence complementary to a sequence selected from those in the second sequence group bound to each other, a sequence selected from those in the fourth sequence group (Table 4), or a sequence complementary to a sequence selected from those in the fourth sequence group. The complementary sequences include strictly complementary sequences and also sequences that can hybridize under a condition stringent to the sequence groups above. Generally under such a condition, a sequential homology of 90 to 95% seems to be sufficient for progress of the reaction. Such a stringent condition would be obvious for those skilled in the art, and is, for example, a temperature in the range of 20°C to 65°C, 2 x SSC buffer solution, and 0.1% w/v SDS. Particularly favorable is a highly stringent condition at a temperature of at least 65°C, 0.1 x SSC buffer solution, and 0.1% w/v SDS. Alternatively, the sequence may be sequences of at least one of the strands of SEQ ID Nos. 1, 2 and 3 or the complementary strands thereof that have one or more nucleotides (e.g., 1 to 5 nucleotides) thereof substituted, deleted, or inserted. However, these substituted, deleted, or inserted sequences are sequences that can hybridize respectively with the complementary strands of unsubstituted, undeleted, or uninserted sequences under the stringent condition. In addition, at least one of SEQ ID Nos. 1, 2 and 3 and the complementary strands thereof may be a mixed-nucleotide sequence of 1 to 5 nucleotides, or at least one of SEQ ID Nos. 1, 2 and 3 and the complementary strands thereof may be a sequence of 1 to 5 nucleotides bring a universal nucleotide. Examples of the universal nucleotides for use include deoxyinosine (dI), and 3-Nitropyrrole, 5-Nitroindole, deoxyribofuranosyl (dP), deoxy-5'-dimethoxytrityl-D-ribofuranosyl (dK) available from Gren Research. It would be obvious for those skilled in the art that these primer regions may be bound to each other directly or via a spacer in the primer according to the invention. A sequence (spacer) of about 1 to 100 nucleotides, preferably 2 to 30 nucleotides, may be present between the sequences or at the terminal of the primer. The length of the nucleic acid primer is about 15 to 200 nucleotides, preferably 20 to 100 nucleotides, and more preferably 40 to 60 nucleotides.

It is possible to amplify the polymorphic region only by using sequences in combination of those in the sequence groups 1 to 3 or in the sequence group 4 as the sequence corresponding to the primer regions F1 and F2, or B1 and B2 in the primer for detection of HPV viral genotype according to the present invention. It is thus possible to detect the polymorphism present in SEQ ID No. 4 contained in LAMP amplification product with the probe. Accordingly, the target sequence FPc, FP, BP, or BPc located in the single-stranded loop of the dumbbell structure of the product amplified with the LAMP primer correspond to the sequence of SEQ ID No. 4.

As described in FIG. 4, it is possible to obtain an HPV-derived target sequence (i.e., sequence in the region corresponding to SEQ ID No. 4) contained in a single-stranded loop structure of a amplification product having a stem-and-loop structure, by amplification by the LAMP method of an HPV-containing sample with the above-mentioned primers in the structure having primer regions F1 and F2.

The amplification reaction may be carried out by using one primer set per tube or multiple primer sets for various genotypes per tube. It is more efficient to use the latter method for identification of multiple genotypes at the same time.

The amplification products are detected, for example, by using probe nucleic acids (FP, FPc, BP, and BPc) having a sequence complementary to the SEQ ID No. 4. Homogeneous hybridization is achieved by using nucleic acid probe labeled by such as fluorochrome (Fluorescein, Rhodamine, FITC, FAM, TET, JOE, VIC, MAX, ROX, HEX, TAMRA, Cy3, Cy5, TexasRed, etc.), quencher(TAMRA, Eclipse, Dabcyl, Au colloid, etc.), electron spin material and metal complex(Ruthenium, Cobalt, Iron, etc.). For example molecular beacon, fluorescence resonance energy transfer (FRET) and electron spin resonance (ESR) technologies are often used for homogeneous hybridization using labeled probe. Invader and pyrosequenching technologies are also used for homogeneous hybridization without labeling. Homogeneous hybridization assay for LAMP products is not particularly limited. The probe nucleic acids may be immobilized on the surface of a solid support for heterogeneous hybridization, and typically, a DNA chip is used, but a probe on another microarray may be used. As described above, the region of the SEQ ID No. 4 is a polymorphic region, and thus, the probe nucleic acids for detection of amplified products may be altered according to the polymorphism to be detected.

The nucleic acid probe sequence for use in the present invention is preferably a nucleic acid probe having a sequence containing a sequence selected from those in the fifth sequence group (Table 5) or a sequence complementary to a sequence selected from those in the fifth sequence group, or the sequence selected from those in the fifth sequence group or the sequence of the complementary to a sequence thereof of which one or more nucleotides are substituted, deleted or insertion. It is also possible to use the sequences described in Kleter et al., J. Clin. Microbiol., 37, 2508-17 (1999); Vernon et al., BMC Infectious Diseases, 3:12 (2003); JP-A 09-509062(KOKAI) and others. The structure of the nucleic acid probe is also not particularly limited, and DNA, RNA, PNA, LNA, methyl phosphonate-skeleton nucleic acid, and other synthetic nucleic acid chains may also be used. In addition, the chimeric nucleic acids thereof may also be used. It is also possible to introduce a functional group such as amino group, thiol group, or biotin, for immobilization of the nucleic acid probe on a solid support, and a spacer may also be introduced additionally between the functional group and the nucleotide. The kind of the spacer used herein is not particularly limited, and, for example, an alkane or ethylene glycol skeleton may be used. Examples of the universal nucleotides for use in the present invention include deoxyinosine (dI) and 3-Nitropyrrole, 5-Nitroindole, deoxyribofuramsyl (dP), and deoxy-5'-dimethoxytrityl-D-ribofuranosyl (dK) available from Gren Research, and the like.

The detection method for use in the invention is not particularly limited, and examples thereof include optical methods of using turbidity, visible light, fluorescence, chemiluminescence, electrochemiluminescence, chemifluorescence, fluorescent energy transfer, ESR, or the like, and electrical methods of using an electrical property such as electrical current, voltage, frequency, conductivity, or resistance.

The support for immobilizing the nucleic acid probe for use in the invention is not particularly limited, and examples thereof include particles (e.g., resin beads, magnetic beads, metal fine particles, and gold colloid), plates (e.g., microtiter plate, glass plate, silicon plate, resin plate, electrode plate, and membrane), and the like.

The raw material for the support for use in the invention is not particularly limited, and examples thereof include permeable materials such as a porous material and membrane and non-permeable materials such as glass and resin. Typical examples of the support materials include inorganic insulation materials such as glass, quartz glass, alumina, sapphire, forsterite, silicon carbide, silicon oxide, and silicon nitride, and organic materials such as polyethylene, ethylene, polypropylene, polyisobutylene, polymethyl methacrylate, polyethylene terephthalate, unsaturated polyesters, fluorine-containing resins, polyvinyl chloride, polychlorinated vinylidene, polyvinyl acetate, polyvinylalcohol, polyvinyl acetal, acrylic resins, polyacrylonitrile, polystyrene, acetal resins, polycarbonate, polyamide, phenol resins, urea resins, epoxy resins, melamine resins, styrene-acrylonitrile copolymers; acrylonitrile butadiene styrene copolymers, silicone resins, polyphenyleneoxide, polysulfone, polyethylene glycol, agarose, acrylamide, nitrocellulose, nylon, and latex.

The support surface on which the nucleic acid chain is immobilized may be formed, for example, with an electrode material. The electrode material is not particularly limited, but examples thereof include pure metals such as gold, gold alloys, silver, platinum, mercury, nickel, palladium, silicon, germanium, gallium, and tungsten, and the alloys thereof; carbon materials such as graphite and glassy carbon; and the oxides and compounds thereof. Other examples include semiconductor compounds such as silicon oxide, and various semiconductor devices such as CCD, FET, and CMOS. The electrode can be produced by plating, printing, sputtering, vapor deposition, or the like. An electrode film may be formed in vapor deposition by resistance heating, high-frequency heating, or electron beam heating. When in sputtering, the electrode film may be formed by DC bipolar sputtering, bias sputtering, asymmetric AC sputtering, getter sputtering, or high-frequency sputtering. It is also possible to use an electrolytic-polymerization membrane such as polypyrrole or polyaniline or a conductive polymer. The material for insulating the area other than the electrode is not particularly limited, but preferably, a photopolymer or a photoresist material. Examples of the resist materials for use include photoresists for light irradiation, photoresists for far-ultraviolet light, photoresists for X-ray irradiation, and photoresists for electron beam irradiation. Examples of the photoresists for light irradiation include photoresists containing cyclized rubber, polycinnamic acid or novolak resin as the main raw material. A cyclized rubber, a phenol resin, polymethylisopropenylketone (PMIPK), polymethyl methacrylate (PMMA), or the like is used as the far-ultraviolet photoresist. Any one of COP, metal acrylate, as well as the substances described in the Thin Film Handbook (published by Ohmsha) may be used for the X-ray resist. Further, the substances described in the literature above such as PMMA may be used for the electron-beam resist. The resist for use desirably has a thickness of 100Å or more and 1 mm or less. It is possible to make the area constant by covering the electrode with a photoresist and performing lithography. It is thus possible to uniformize the amount of DNA probe immobilized between electrodes and to make the measurement favorable in reproducibility. The resist material has been generally removed finally, but it is possible to use the resist material as a part of the electrode for gene detection without removal. In such a case, a substance higher in water resistance is needed to be used as the resist material. Materials other than the photoresist materials may be used for the insulation layer formed over the electrode. Examples thereof include oxides, nitrides, and carbides of metals such as Si, Ti, Al, Zn, Pb, Cd, W, Mo, Cr, Ta, and Ni and the alloys thereof. After a thin film is formed on the material, for example, by sputtering, vapor deposition, or CVD, the electrode-exposed regions are patterned to an area adjusted to a particular value by photolithography. It is possible to prepare an electrode allowing tests on several kinds of targets by configuring several electrode units and immobilizing different probes thereon on a single chip. It is also possible to test multiple samples at the same time by configuring several electrode units and immobilizing the same probe thereon on a single chip. In such a case, multiple electrodes are patterned on a substrate previously by photolithography. It is effective then to form an insulation film separating individual electrodes for prevention of contact of neighboring electrodes. The thickness of the insulation film is preferably about 0.1 to 100 micrometers.

The sample to be analyzed in the present invention is not particularly limited, and examples thereof include blood, serum, leukocyte, urine, feces, semen, saliva, vaginal fluid, tissue, biopsy sample, oral mucosa, cultured cell, sputum, and the like. Nucleic acid components are extracted from these samples. The extracting method is not particularly limited, and examples thereof include liquid-liquid extraction, for example with phenol-chloroform, and solid-liquid extraction by using a carrier. Commercial nucleic acid-extracting kits such as QIAamp (manufactured by QIAGEN), or Sumai test (manufactured by Sumitomo Metal Industries) may be used instead. The extracted nucleic acid components are amplified by LAMP methods, and the amplified product is hybridized with the probe immobilized on the electrode for gene detection. The reaction is carried out in a buffer solution at an ionic strength in the range of 0.01 to 5 and at a pH in the range of 5 to 10. Other additives such as hybridization accelerator dextran sulfate, salmon sperm DNA, bovine thymic DNA, EDTA, and surfactant may be added to the solution. The amplified product is added thereto. Alternatively, hybridization may be performed by dropping the solution on the substrate. The reaction may be accelerated, for example, by agitation or shaking during the reaction. The reaction temperature is preferably in the range of 10°C to 90°C, and the reaction period is about 1 minute or more to overnight. After hybridization reaction, the electrode is separated and washed. A buffer solution at an ionic strength of 0.01 to 5 and a pH in the range of 5 to 10 is used for washing.

The extracted nucleic acid sample can be detected, by labeling with a fluorescent dye such as FITC, Cy3, Cy5, or rhodamine; biotin, hapten, an enzyme such as oxidase or phosphatase, or an electrochemically active substance such as ferrocene or quinone, or by using a second probe previously labeled with the substance described above.

For example with an electrochemically active DNA-binding substance, nucleic acid components are analyzed in the following manner. A substrate is first cleaned, a DNA-binding substance selectively binding to the double-stranded region formed on the electrode surface is allowed to react, and the substrate is analyzed electrochemically. The DNA-binding substance for use is not particularly limited, and examples thereof include Hoechst 33258, acridine orange, quinacrine, daunomycin, metallointercalators, bisintercalators such as bisacridine, trisintercalators, and polyintercalators. In addition, these intercalators may be modified with an electrochemically active metallocomplex such as ferrocene or viologen. The concentration of the DNA-binding substance may vary according to the kind thereof, but is generally in the range of 1 ng/ml to 1 mg/ml. A buffer solution at an ionic strength in the range of 0.001 to 5 and a pH in the range of 5 to 10 is used then. The electrode after reaction with the DNA-binding substance is washed and analyzed electrochemically. The electrochemical measurement is performed in a three-electrode analyzer including reference, counter, and action electrodes or in a two-electrode analyzer including counter and action electrodes. During measurement, a voltage high enough to cause electrochemical reaction of the DNA-binding substance is applied, and the reaction current derived from the DNA-binding substance is determined. The voltage may be varied linearly, or may be applied in the pulse shape or at a constant voltage. The current and voltage during measurement are controlled by using a device such as a potentiostat, a digital multimeter, or a function generator. The concentration of the target gene is calculated from the measured electric current with a calibration curve. The gene-detecting device using the gene-detecting electrode includes a gene-extracting unit, a gene-reacting unit, a DNA-binding substance-reacting unit, an electrochemical measurement unit, a washing unit, and others.

It is possible to diagnose human papilloma virus infection by using the method according to the present invention.

Thus, provided is a method of diagnosing human papilloma viral infection, comprising
obtaining a sample from human;
extracting nucleic acid components from the sample;
a step of amplifying the nucleic acid chains in the sample in LAMP reaction by using multiple primers including at least one primer selected from the nucleic acid primers described above; and
a step of analyzing whether there are amplification products after the amplification reaction, wherein
presence of the amplification products indicates infection to human papilloma virus.

Also provided is a method of diagnosing human papilloma virus infection, comprising
obtaining a sample from human;
extracting nucleic acid components from the sample;
a step of amplifying the nucleic acid chains in the sample in LAMP reaction by using multiple primers including at least one primer selected from the nucleic acid primers described above; and
a step of analyzing whether there are amplification products or identifying the genotype of the virus after the amplification reaction, wherein
presence of the amplification products leads to diagnosis of infection to human papilloma virus.

In addition, the present invention provides a LAMP-amplification kit for use in the detection of human papilloma virus and identification of its genotype. The LAMP-amplification kit contains a nucleic acid primer selected from following (a)-(f) and additionally any other components needed for the LAMP amplification reaction such as polymerase, dNTPs, betaine, buffer, positive control DNA, and sterilized water:
(a) a nucleic acid primer containing, on the same chain, a sequence complementary to a sequence selected from those in a first sequence group listed in Table 1 and a sequence selected from those in a second sequence group listed in Table 2;
(b) a nucleic acid primer containing, on the same chain, a sequence complementary to a sequence selected from those in the first sequence group and a sequence selected from those in a third sequence group listed in Table 3;
(c) a nucleic acid primer containing, on the same chain, a sequence complementary to a sequence selected from those in the second sequence group and a sequence selected from those in the third sequence group;
(d) a nucleic acid primer containing a sequence that differs from a selected sequence of one of the nucleic acid primers (a), (b), and (c), by insertion, deletion, or substitution of one or more bases, and capable to hybridize with a nucleic acid chain having a sequence complementary to the selected sequence of one of the nucleic acid primers (a), (b), and (c);
(e) a nucleic acid primer containing a sequence selected from those in a fourth sequence group listed in Table 4 or a sequence complementary thereto; and
(f) a nucleic acid primer containing a sequence that differs from a selected sequence of one of the nucleic acid primers (e), by insertion, deletion, or substitution of one or more bases, and capable to hybridize with a nucleic acid chain having a sequence complementary to the selected sequence of one of the nucleic acid primers (e).

In addition, the present invention provides a detection kit for use in the detection of human papilloma virus and identification of its genotype. The detection kit includes the above-mentioned LAMP-amplification kit and a support carrying a nucleic acid chain immobilized thereon for detection of the human-papilloma-virus LAMP amplification products amplified by using the LAMP-amplification kit. The immobilized nucleic acid chain is;
(g) a nucleic acid probe containing a sequence selected from those in a fifth sequence group listed in Table 5 or a sequence complementary thereto, or
(h) a nucleic acid probe containing a sequence that differs from a selected sequence of one of the nucleic acid probe (g), by insertion, deletion, or substitution of one or more bases, and capable to hybridize with a nucleic acid chain having a sequence complementary to the selected sequence of one of the nucleic acid probe (g). The nucleic acid probe may be immobilized on the surface of a support, and the support and the support surface are made of the material described above.

### Examples

Hereinafter, typical examples of the sequences corresponding to the primer regions in the first, second, third, and fourth sequence groups are shown in the following Tables.

**Table 1 Representative nucleic acid primer sequence**

| Sequence group | SEQ ID No. | Sequence (5' → 3') |
|---|---|---|
| First sequence group | 16 | TGATTTACAGTTTATTTTTC |
| | 17 | GAATATGATTTACAGTTTATTTTTC |
| | 18 | GAAGAATATGATTTACAGTTTATTTTTC |
| | 19 | GAGGAATATGATTTACAGTTTATTTTTC |
| | 20 | GAGTATGATTTACAATTTATTTTTC |
| | 21 | GAGTTTGATTTACAGTTTATTTTTC |
| | 22 | GAATTTGATTTACAATTTATTTTTC |
| | 23 | GAATATGATTTACAGTTTATTTTTC |
| | 24 | GAATATGATTTGCAGTTTATTTTTC |
| | 25 | GAATATGAATTACAGTTTGTGTTTC |
| | 26 | GAATTTGATTTACAATTTATATTTC |
| | 27 | GAATATGATATACAGTTTATATTTC |
| | 28 | GAATATGATCTACAGTTTGTTTTTC |
| | 29 | GAGTATGACCTGCAGTTTGTGTTTC |
| | 30 | GAATATGATTTACAGTTTATTTTTC |
| | 31 | GAGTATGATTTACAATTTATATTTC |
| | 32 | GAGTTTGATTTGCAGTTTATTTTTC |
| | 33 | GAATATGATGTGCAATTTATATTTC |
| | 34 | GAATATGATTTACAGTTTATTTTTC |
| | 35 | GAGTATGAATTGCAATTTATTTTTC |
| | 36 | GAATTTGATTTACAATTTATTTTTC |
| | 37 | GAATATGAATTACAATTTGTGTTTC |
| | 38 | GAATATGAATTACAATTTGTTTTTC |
| | 39 | GAATATGACTTACAGTTTGTTTTTC |
| | 40 | GAGTTTGATTTGCAATTTATTTTTC |
| | 41 | GAATATGAACTACAGTTTGTGTTTC |
| | 42 | GAATATGATTTGCAATTTATATTTC |
| | 43 | GAAAHATAAAYTGYAADTCATAYTC |

**Table 2**

| Sequence group | SEQ ID No. | Sequence (5' → 3') |
|---|---|---|
| Second sequence group | 44 | TTTGTTACTGTGGTAGATAC |
| | 45 | TTTGTTACTGTGGTAGATACTAC |
| | 46 | TTTGTTACTGTGGTAGATACCAC |
| | 47 | TTTGTTACTGTGGTAGATACCAC |
| | 48 | TTTGTTACTGTAGTTGATACTAC |
| | 49 | TTTGTTACTGTTGTTGATACTAC |
| | 50 | TTTGTTACTGTGGTAGATACCAC |
| | 51 | TTTGTTACTGTTGTGGACACCAC |
| | 52 | TTTGTTACTGTGGTAGATACCAC |
| | 53 | TTTCTAACTGTTGTGGATACTAC |
| | 54 | TTTGTTACTGTGGTAGATACCAC |
| | 55 | TTTTTAACTGTTGTAGATACTAC |
| | 56 | TTTGTTACTGTAGTTGATACAAC |
| | 57 | TTTCTTACTGTTGTGGACACTAC |
| | 58 | TTTGTTACAGTTGTAGACACCAC |
| | 59 | TTTTTAACTGTGGTTGATACTAC |
| | 60 | TTTGTTACTGTAGTGGACACTAC |
| | 61 | TTTATTACCTGTGTTGATACTAC |
| | 62 | TTTGTCACAGTTGTGGATACCAC |
| | 63 | TTTGTAACTGTTGTGGATACCAC |
| | 64 | TTTGTTACTGTAGTAGATACTAC |
| | 65 | TTTGTTACCGTGGTTGATACCAC |
| | 66 | TTTGTAACCGTTGTGGATACCAC |
| | 67 | TTTGTTACTGTTGTGGATACTAC |
| | 68 | TTTCTTACTGTTGTGGATACCAC |
| | 69 | TTTKTTACHGTKGTDGATACYAC |

**Table 3**

| Sequence group | SEQ ID No. | Sequence (5' → 3') |
|---|---|---|
| Third sequence group | 70 | GCACAGGGCCACAATAATGG |
| | 71 | GCACAGGGACATAACAATGG |
| | 72 | GCGCAGGGCCACAATAATGG |
| | 73 | GCACAGGGACATAATAATGG |
| | 74 | GCCCAGGGCCACAACAATGG |
| | 75 | GCTCAGGGTTTAAACAATGG |
| | 76 | GCTCAGGGTTTAAACAATGG |
| | 77 | GCCCAGGGACATAACAATGG |
| | 78 | GCTCAGGGACATAACAATGG |
| | 79 | GCCCAGGGACACAATAATGG |
| | 80 | GCACAGGGTCATAACAATGG |
| | 81 | GCACAGGGTCATAATAATGG |
| | 82 | GCACAGGGACACAATAATGG |
| | 83 | GCTCAGGGACACAATAATGG |
| | 84 | GCACAAGGTCATAATAATGG |
| | 85 | GCCCAGGGACAAAACAATGG |
| | 86 | GCACAAGGCCATAATAATGG |
| | 87 | GCCCAGGGCCACAACAATGG |
| | 88 | GCCCAGGGCCATAAGAATGG |
| | 89 | GCACAAGGACACAATAATGG |
| | 90 | GGACATAATAATGG |
| | 91 | GCGCAGGGCCACAATAATGG |
| | 92 | GCCCAGGGCCATAACAATGG |
| | 93 | GCGCAGGGTCACAATAATGG |
| | 94 | GCGCAGGGCCACAATAATGG |
| | 95 | GCCCAGGGACATAATAATGG |
| | 96 | GCCCAGGGTCAAAACAATGG |
| | 97 | GCGCAGGGCCACAATAATGG |
| | 98 | GCCCAAGGCCATAATAATGG |
| | 99 | GCACAAGGTCATAACAATGG |
| | 100 | GCTCAGGGTTTAAACAATGG |
| | 101 | GCCCAGGGCCACAACAATGG |
| | 102 | GCACAGGGTCATAATAATGG |
| | 103 | GCACAGGGACATAACAATGG |
| | 104 | GCACAGGGTCATAATAATGG |
| | 105 | GCCCAGGGACATAACAATGG |
| | 106 | GCACAGGGACACAACAATGG |
| | 107 | GCACAGGGACATAACAATGG |
| | 108 | GCCCAGGGAACTAATAATGG |
| | 109 | GCCCAGGGTCATAATAATGG |
| | 110 | GCACAGGGTCATAATAATGG |
| | 111 | GCGCAAGGCCACAATAATGG |
| | 112 | GCACAGGGACATAATAATGG |
| | 113 | GCCCAGGGACATAATAATGG |
| | 114 | GCGCGGGGTCATAACAATGG |
| | 115 | GCMCAGGGWCATAAYAATGG |
| | 103 | GCACAGGGACATAACAATGG |
| | 104 | GCACAGGGTCATAATAATGG |
| | 105 | GCCCAGGGACATAACAATGG |
| | 106 | GCACAGGGACACAACAATGG |
| | 107 | GCACAGGGACATAACAATGG |

**Table 4**

| Sequence group | SEQ ID No. | Sequence (5' → 3') |
|---|---|---|
| Fourth sequence group | 116 | GCGTGTAGTATCAACAACAGT |
| | 117 | CCTTATTGGTTACAACGAGCACAACAAATAGTTGGTTACCCCA |
| | 118 | GGTGAAAATGTACCAGACGAT |
| | 119 | AGAGGTAACCATAGAACCACTAGGGTCTACTGCAAATTTAGCCA |
| | 120 | AGTAGATATGGCAGCACAT |
| | 121 | ACAGGGCCACAATAATGGCATGACATATTTGTACTGCGTGT |
| | 122 | CATTAAAGGCTCTGGGTCTA |
| | 123 | GCATCAGAGGTAACCATAGAACCACTGCAAATTTAGCCAGTTCA |
| | 124 | TTCCAGTCCTCCAAAATAGTGG |
| | 125 | ATACTACACGCAGTACAAATATGTCTGTCATAACGTCTGCAGTTAAGG |
| | 126 | CAAATTATTTTCCTACACCTAGTGG |
| | 127 | GTTGGTTACCCCAACAAATGCCTCTATGGTTACCTCTGATGCCC |
| | 128 | GTCATAACGTCTGCAGTTAAGG |
| | 129 | GTCGTAGGTACTCCTTAAAGTTAG |
| | 130 | ATACTACACGCAGTACAAATATGTCTCCCCATGTCGTAGGTACTCC |
| | 131 | CTACACGCAGTACAAATATGTCTCCCCATGTCGTAGGTACTCC |
| | 132 | CACGCAGTACAAATATGTCACCCCATGTCGTAGGTACTCC |
| | 133 | CTACACGCAGTACAAATATGTCGTAGTTTCTGAAGTAGATATGGCA |
| | 134 | CTACACGCAGTACAAATATGTCTATGTAGTTTCTGAAGTAGATATG |
| | 135 | GTGGCCCTGTGCTCGTTGTTCTATGGTTACCTCTGATGCCC |
| | 136 | GTGGCCCTGTGCTCGTTGTCTATGGTTACCTCTGATGCC |
| | 137 | GTGCTGCCATATCTACTTCAGAAAC |
| Fourth sequence group | 138 | GCTGCCATATCTACTTCAGAAACTACA |
| | 139 | AACCAATAAGGTTTATTGAATATTT |
| | 140 | CCAATAAGGTTTATTGAATATTTGG |
| | 141 | TTATGCAGCAAATGCAGGTGTGGCCCCTATAGGTGGTTTGCAACC |
| | 142 | CCCTATAGGTGGTTTGCAAC |
| | 143 | GTACATGGGGATCCTTTGCC |
| | 144 | ACAGAAAATGCTAGTGCTTATGCAGCCAATTGTGTTTGTTTGTAATCCATAG |
| | 145 | CACAGAAAATGCTAGTGCTTATTGTGTTTGTTTGTAATCCATAG |
| | 146 | ATAAAGGATGGCCACTAATGCCCGTGTAGGTGTTGAGGTAGGTCG |
| | 147 | CCTGACACCTCATTTTATAATCCAG |
| | 148 | ATCCAGATACACAGCGGCTG |
| | 149 | CCACACCTAATGGCTGACCACACACAGCGGCTGGTTTG |
| | 150 | GCCACTAATGCCCACACCTAATGACACAGCGGCTGGTTTG |
| | 151 | CCAACAGTACCAGCCCTAT |
| | 152 | TGGTGTCAGAACCATATGGCGACAAACATTTGTTCCCTTCG |
| | 153 | CACAGTTATTCAGGATGGTGAT |
| | 154 | GGAACTTCACTTTTGTTAGCCTGTTGGTTCATACTGGCTTTGG |
| | 155 | CCCTATAGGTGGTTTGCAAC |
| | 156 | ACAGAAAATGCTAGTGCTTATGCAGCCAATTGTGTTTGTTTGTAATCCATAG |
| | 157 | CCTGACACCTCATTTTATAATCCAG |
| | 158 | CCACACCTAATGGCTGACCACACACAGCGGCTGGTTTG |
| | 159 | GCCACTAATGCCCACACCTAATGACACAGCGGCTGGTTTG |
| Fourth sequence group | 160 | AAGTTCCAATCCTCTAAAATACTGC |
| | 161 | ACCACTCGCAGTACCAATTTAACTGAATATAGGACATAACATCTGCAG |
| | 162 | TGTATTCTCCCTCTCCAAGTG |
| | 163 | TAATTGATTATGCCAGCAAACACCCTCTATTGTTACCTCTGACTCCC |
| | 164 | GCCAGCAAACACCATTGTTACTCTATTGTTACCTCTGACTCCC |
| | 165 | GCCAGCAAACACCATTGTTATGCTCTATTGTTACCTCTGACTCCC |
| | 166 | GAATATAGGACATAACATCTGCAG |
| | 167 | ACCACTCGCAGTACCAATTTAACCCTCAACATGTCTGCTATACTGC |
| | 168 | CCACTCGCAGTACCAATTTAACCCTCAACATGTCTGCTATACTGC |
| | 169 | CCACTCGCAGTACCAATTTAACCTCAACATGTCTGCTATACTG |
| | 170 | ACCCTGTGCCTTATGTAACC |
| | 171 | GATGACACTGAAAGTTCCCATGCGCCCAAAATACATAACTGTGTCTGC |
| | 172 | GATGACACTGAAAGTTCCCATGCCCCAAAATACATAACTGTGTCTGC |
| | 173 | AGTGTTCCCCAATAGCAGG |
| | 174 | CAGTGTTCCCCAATAGCAGG |
| | 175 | GACACTGAAAGTTCCCATGCCGCTGTGTCTGCTTATAATCTACAGACAC |
| | 176 | TAAAATGGATGCCCACTAAGGCCTGCTGGAGTGGAAATTGGCCG |
| | 177 | CCTGAAACACAACGTTTAGTG |
| | 178 | CACAACGTTTAGTGTGGGCC |
| | 179 | ACCTGATACTAGTATTTATAATCCTGA |
| | 180 | GATGCCCACTAAGGCCAACACGCCTGTGCTGGAGTGGA |
| | 181 | CACTAAGGCCAACACCTAAAGGCAACACAACGTTTAGTGTGGG |
| Fourth sequence group | 182 | AGTGTTCCCCAATAGCAGG |
| | 183 | GACACTGAAAGTTCCCATGCCGCTGTGTCTGCTTATAATCTACAGACAC |
| | 184 | CCTGAAACACAACGTTTAGTG |
| | 185 | ACCTGATACTAGTATTTATAATCCTGA |
| | 186 | GATGCCCACTAAGGCCAACACGCCTGTGCTGGAGTGGA |
| | 187 | CACTAAGGCCAACACCTAAAGGCAACACAACGTTTAGTGTGGG |
| | 188 | CCAATCTTCCAAAATAGCAGGATTC |
| | 189 | ACCACACGTAGTACCAATATGTCCTGTGAATATATGTCATTATGTCTGCAG |
| | 190 | CATACTTTCCTACACCTAGCG |
| | 191 | AACTGATTGCCCCAACAAATACCCTCCATGGTTACTTCAGATGCAC |
| | 192 | CCATTATTGTGTCCCTGAGCACCTCCATGGTTACTTCAGATGCAC |
| | 193 | CTGATTGCCCCAACAAATACCTCCATGGTTACTTCAGATGC |
| | 194 | CTGATTGCCCCAACAAATACCCATGGTTACTTCAGATGCAC |
| | 195 | GTGAATATATGTCATTATGTCTGCAG |
| | 196 | ACCACACGTAGTACCAATATGTCTTCCTCACCATGTCTTAAATACTC |
| | 197 | ACCACACGTAGTACCAATATGTCATTCCTCACCATGTCTTAAATACTC |
| | 198 | CACACGTAGTACCAATATGTCTGATTCCTCACCATGTCTTAAATACTC |
| | 199 | CCACACGTAGTACCAATATGTCCCTCACCATGTCTTAAATACTC |
| | 200 | CCACACGTAGTACCAATATGTCCTCACCATGTCTTAAATACTC |
| | 201 | CACGTTGCATCCAATATGGT |
| | 202 | CACGTTGCATCCAATATGG |
| | 203 | CACTGAAAACTCTAATAGATATGCCGGTGCAACCAAGTAAACACAGTTGTG |
| Fourth sequence group | 204 | CCAATAGGTGGTTTGCAAC |
| | 205 | CCTTTACCCCAATGCTCTCC |
| | 206 | CACTGAAAACTCTAATAGATATGCCGGAGTTGTGTTTGTTTATAATCCATTG |
| | 207 | CTGAAAACTCTAATAGATATGCCTGTGTTTGTTTATAATCCATTG |
| | 208 | GGATGACCACTAATACCTACACCCTGTGTTGGTTTAGAGGTAGGTC |
| | 209 | TCCTGATACATCTTTTTATAATCCTG |
| | 210 | AACTCAACGCTTAGTTTGGGC |
| | 211 | CCACTAATACCTACACCTAATGGCTGCAAACTCAACGCTTAGTTTGGGC |
| | 212 | CACTAATACCTACACCTAATGGCCTCAACGCTTAGTTTGGG |
| | 213 | GACCTACCTCTAAACCAACACAG |
| | 214 | TAATGGCTGCCCGCGA |
| | 215 | CCAATAGGTGGTTTGCAAC |
| | 216 | CACTGAAAACTCTAATAGATATGCCGGAGTTGTGTTTGTTTATAATCCATTG |
| | 217 | TCCTGATACATCTTTTTATAATCCTG |
| | 218 | CCACTAATACCTACACCTAATGGCTGCAAACTCAACGCTTAGTTTGGGC |
| | 219 | GGAGGTGTTAAACCAAATTGCC |
| | 220 | ACCACTCGCAGTACTAATATGACTATGTCATAACTTCTGCAGTTAAGG |
| | 221 | ACCACTCGCAGTACTAATATGACCTTCTGCAGTTAAGGTAACTTTGC |
| | 222 | GCTTTTTTTCCCACTCCTAGTG |
| | 223 | CCTGATTGCCCCAACAAATACCGATCAATGGTTACTTCCGAATCTC |
| | 224 | CCTGATTGCCCCAACAAATACCAGCCATATTGGCTACAACGTGC |
| | 225 | ATGTCATAACTTCTGCAGTTAAGG |
| Fourth sequence group | 226 | ACCACTCGCAGTACTAATATGACTTCTTCAACATGTCTTATATATTC |
| | 227 | ATACCACTCGCAGTACTAATATGTTCTTCAACATGTCTTATATATTC |
| | 228 | ACCACTCGCAGTACTAATATGACATTCTTCAACATGTCTTATATATTCT |
| | 229 | ATACCACTCGCAGTACTAATATGATTCTTCAACATGTCTTATATATTCT |
| | 230 | CCTGGACAACCGGGTGCTGTTGGAGGCTTACATCCAAG |
| | 231 | TATCCTGGACAACCGGGTGCTCCTGTTGGAGGCTTACATCCAAG |
| | 232 | GGAGGCTTACATCCAAGTAAAC |
| | 233 | GTACAAGCAACACCTTTACCCC |
| | 234 | GACACTGAAACCGGTAACAAGTATCCACTGTGTTTGTTTATAATCCATGG |
| | 235 | GGATGACCACTTATGCCAACGCCACAACGATTAGTATGGGCATGTG |
| | 236 | CCTGACACCTCCTTTTATAACCCT |
| | 237 | CCTGACACCTCCTTTTATAACCC |
| | 238 | CACTTATGCCAACGCCTAATGGGATACACAACGATTAGTATGGGC |
| | 239 | GGATGACCACTTATGCCAACGCACAACGATTAGTATGGGCATG |
| | 240 | GCTGCCCTCTACCTATTTCAAGG |
| | 241 | CACCTTTACCCCAATGTTCC |
| | 242 | GTCATATTAGTACTGCGAGTGG |
| | 243 | CCGGGTGCTGATAATAGGGACCTGTTGGAGGCTTACATCC |
| | 244 | CCATATTGGCTACAACGTGCTACCTGATTGCCCCAACA |
| | 245 | CCATATTGGCTACAACGTGCACCAAATACCTGATTGCCCC |
| | 246 | GTGCACAAGGTCATAATAATGG |
| | 247 | AGTATGGGCATGTGTAGGC |
| Fourth sequence group | 248 | AGGGCTGGTACATTAGGAGA |
| | 249 | TGTTCCCGATGACCTGTAC |
| | 250 | CTTGTTACCGGTTTCAGTGTCAGCAGCCATTAGGCGTTG |
| | 251 | GCACTGCTTTGAATAGAGGCACTGTTCCCGATGACCTG |
| | 252 | GTAACCATTGATCCACTAGGAGTGAAAGGTTCAGGAACTACTGCC |
| | 253 | GTAGTTCCTGAACCTTTAATGTACAG |
| | 254 | GCACTGCTTTGAATAGAGGCA |
| | 255 | CAGTAGTTCCTGAACCTTTAATGTACA |
| | 256 | GGAGGCTTACATCCAAGTAAAC |
| | 257 | GACACTGAAACCGGTAACAAGTATCCACTGTGTTTGTTTATAATCCATGG |
| | 258 | CCTGACACCTCCTTTTATAACCCT |
| | 259 | CACTTATGCCAACGCCTAATGGGATACACAACGATTAGTATGGGC |
| | 260 | CAAATCCTGTGTCTACCATG |
| | 261 | GGCACACCTTGTAATGCTAACTCCCCGTCTTGTAGTACAGTG |
| | 262 | ATGTTGGTAACTCTGGTAACTC |
| | 263 | GGAGGCCTACAACCTATTAAACAGGTACAGATAACAGGGAATGC |
| | 264 | AATTGTGTTTGTTTATAATCCATAG |
| | 265 | TGCACCAAATCCTGTGTC |
| | 266 | GCTAACCAGGTAAAAGCAGGATACCATGTCCCCGTCTTG |
| | 267 | AACTCTGGTAACTCTGGTACAG |
| | 268 | GGAGGCCTACAACCTATTAAACAACAGGGAATGCATTTCTATGG |
| | 269 | CAATTGTGTTTGTTTATAATCCATAG |
| Fourth sequence group | 270 | GATATTTGCAAATGGAACTG |
| | 271 | GACGGGGACATGGTAGACACATATATCTAGGGGAACATCAC |
| | 272 | GTGTTTAATAGGTTGTAGGCC |
| | 273 | CTCCTGCTTTTACCTGGTTAGCTCCTATAGGTGAACATTGGG |
| | 274 | ATTACAAGGTGTGCCTTTTC |
| | 275 | GGATATTTGCAAATGGAACTG |
| | 276 | GGGACATGGTAGACACAGGACATATATCTAGGGGAACATCAC |
| | 277 | CCTATAGGTGAACATTGGG |
| | 278 | GTTTAGTAACTCCAAAGGAGGACAAAGGCACACCTTGTAATGC |
| | 279 | ATTCTCCTGCTTTTACCTGG |
| | 280 | CATTCTCCTGCTTTTACCTGGT |
| | 281 | ATCCTCTAAAATGGACGGGTTC |
| | 282 | CAACCCGTAGTACAAATATGTCTGATATGTCATAACATCTGCTGTTAGTG |
| | 283 | CTAGTTATTTTCCTACTCCTAGTGG |
| | 284 | CAATTGGTTACTCCAACAAATACCCTCTATGGTAACCTCCGATGCAC |
| | 285 | CCATTATTATGGCCTTGTGCACGCTCTATGGTAACCTCCGATGCAC |
| | 286 | ATGGCCTTGTGCACGTTGCAACCTCTATGGTAACCTCCGATGCAC |
| | 287 | GGCCTTGTGCACGTTGCCTATGGTAACCTCCGATGCAC |
| | 288 | GCCTTGTGCACGTTGCACTATGGTAACCTCCGATGCAC |
| | 289 | ATGTCATAACATCTGCTGTTAGTG |
| | 290 | ATGTCATAACATCTGCTGTTAGTG |
| | 291 | CAACCCGTAGTACAAATATGTCTGTTCTTCACCATGCCTTAAATATTCC |
| Fourth sequence group | 292 | CCCGTAGTACAAATATGTCTGCACCATGCCTTAAATATTCC |
| | 293 | CCCGTAGTACAAATATGTCTGTTCACCATGCCTTAAATATTCC |
| | 294 | CCCGTAGTACAAATATGTCTGCTTCACCATGCCTTAAATATTCC |
| | 295 | CCAATATGGTTTATTAAATATTTG |
| | 296 | CAATATGGTTTATTAAATATTTGTG |
| | 297 | CAATATGGTTTATTAAATATTTGTGC |
| | 298 | GTTGGTAACTCTGGTAACTCTGGGGAGGCCTACAACCTATTAAACAC |
| | 299 | GTTGGTAACTCTGGTAACTCTGGGAGGCCTACAACCTATTAAACAC |
| | 300 | CCCAATGTTCACCTATAGGAGG |
| | 301 | GCCTTTTCCCCAATGTTCACC |
| | 302 | TGGTAACTCTGGTAACTCTGGTACAGCCTACAACCTATTAAACACAATTGTG |
| | 303 | GGATGACCACTAATACCTACTCCGTTTGGTTTGGGCCTGTACAGG |
| | 304 | GGATGACCACTAATACCTACTCCGTTTGGTTTGGGCCTGTACAG |
| | 306 | CCAGACACATCATTTTATGATCC |
| | 306 | TTATGATCCCTGCCTCCAGC |
| | 307 | GACCACTAATACCTACTCCTAATGGCCTGCCTCCAGCGTTTGG |
| | 308 | AGCATTACAAGGTGTGCC |
| | 309 | CAGATAACAGGGAATGCATTTCAATGTTCACCTATAGGAGGCC |
| | 310 | AAGTAGGTCGTGGTCAGC |
| | 311 | CCAGAGTTACCAGAGTTACCAACGGAGTAGGTATTAGTGGTCATCC |
| | 312 | GATTTTCAGTATCATCCAATTTAT |
| | 313 | CCCAATGTTCACCTATAGGAGG |
| Fourth sequence group | 314 | TGGTAACTCTGGTAACTCTGGTACAGCCTACAACCTATTAAACACAATTGTG |
| | 315 | CCAGACACATCATTTTATGATCC |
| | 316 | GACCACTAATACCTACTCCTAATGGCCTGCCTCCAGCGTTTGG |
| | 317 | CCAATTGTCCAATATAGAGGAATTC |
| | 318 | ACTACCCGTAGTACCAACTTTACGACATAACATCAGTTGTTAATGTGAC |
| | 319 | GTTCTGTATACTGCCCCTCTC |
| | 320 | AACATATACCATTGTTGTGGCCCTTCCATGGTAACCTCTGATTCCC |
| | 321 | GCCTTATGTAGCCAATAAGGCCCAGCGGTTCCATGGTAACC |
| | 322 | GCCTTATGTAGCCAATAAGGCGCGGTTCCATGGTAACCTCTG |
| | 323 | GCCAACATATACCATTGTTGTCATGGTAACCTCTGATTCCC |
| | 324 | GACATAACATCAGTTGTTAATGTGAC |
| | 325 | ACTACCCGTAGTACCAACTTTACTCCACGTGCCTGGTATATTCC |
| | 326 | ACTACCCGTAGTACCAACTTTACTCCACGTGCCTGGTATATTCCT |
| | 327 | CTACCCGTAGTACCAACTTTACCCACGTGCCTGGTATATTCC |
| | 328 | CCTTATGTAGCCAATAAGGC |
| | 329 | GGCCTTATGTAGCCAATAAGGC |
| | 330 | CAGTAGGGATAATGTGTCTGTGGATGCCTTTCCCTTACCCCAGTG |
| | 331 | AATGGCGGGAACACAGC |
| | 332 | CCGTAGATACATTATTGGGCTTGC |
| | 333 | CTGAAAACTCACCATTTTCATCAACCCACAACTGTGTCTGTTTATAATCCAC |
| | 334 | GGTGAGTTTTCAGTATCATCCTGTCCCATTGGGTGTTGGTATTAGTGG |
| | 335 | CCAGATGCATCCTTATATAATCCA |
| Fourth sequence group | 336 | GTTTAGTATGGGCTTGTGTAGG |
| | 337 | ATGGGTGTCCACTAATACCAACACGTTTAGTATGGGCTTGTGTAGGG |
| | 338 | AATGGCGGGAACACAGC |
| | 339 | CTGAAAACTCACCATTTTCATCAACCCACAACTGTGTCTGTTTATAATCCAC |
| | 340 | CCAGATGCATCCTTATATAATCCA |
| | 341 | ATGGGTGTCCACTAATACCAACACGTTTAGTATGGGCTTGTGTAGGG |
| | 342 | TGGTGGAGGGACACCAAAATTC |
| | 343 | TTCCAATTTTCTAATATACTACTATTC |
| | 344 | CACTACCCGCAGTACTAATTTAACATATGACATAACCTCTGCAGTTAAAG |
| | 345 | CACTACCCGCAGTACTAATTTAACGGATATATGACATAACCTCTGCAG |
| | 346 | CACTACCCGCAGTACTAATTTAACCCTCTGCAGTTAAAGTAATAGTGC |
| | 347 | CACTACCCGCAGTACTAATTTAACCTATGGATATATGACATAACCTCTGC |
| | 348 | TGTATTCCCCTTCTCCCAG |
| | 349 | GTGAAACCCCTGGCAGTTG |
| | 350 | AAATACCATTGTTATGGCCCTGGGGCTCTATTATTACTTCTGATTCTC |
| | 351 | ATACCATTGTTATGGCCCTGGGTGTGTATTCCCCTTCTCCCAG |
| | 352 | ATACCATTGTTATGGCCCTGGGTGTATTCCCCTTCTCCCAGTG |
| | 353 | ATACCATTGTTATGGCCCTGGGGGCTCTATTATTACTTCTGATTCTC |
| | 354 | CAACTGATTATGCCAACAAATACCAGCCATATTGGTTACATAAGGCC |
| | 355 | TATGACATAACCTCTGCAGTTAAAG |
| | 356 | CACTACCCGCAGTACTAATTTAACCCTCCACATGTCTACTATACTGC |
| | 357 | CACTACCCGCAGTACTAATTTAACACTATACTGCTTAAACTTAGTAGGG |
| Fourth sequence group | 358 | GGATGATACAGAAAGTGCTCATGCCCTAAAATACACAGCTGTGTTTGC |
| | 359 | GGATGATACAGAAAGTGCTCAAAATACACAGCTGTGTTTGC |
| | 360 | CACCAATAGCAGGTACACAACC |
| | 361 | GTGCTCACCAATAGCAGGTAC |
| | 362 | GGATGATACAGAAAGTGCTCATGCAGCTGTTTGCTTATAATCAACTGACACA |
| | 363 | TAAAATGGATGGCCACTTAGGCCGGTATGGAAATTGGTCGTGGGC |
| | 364 | CCACTTAGGCCAATACCTAAGTGTAGGTATGGAAATTGGTCG |
| | 365 | CCTGAAACACAACGTTTGGTT |
| | 366 | GAAACACAACGTTTGGTTTGGGC |
| | 367 | GCCACTTAGGCCAATACCTAAAGGCATGTGTAGGTATGGAAATTGG |
| | 368 | GCCACTTAGGCCAATACCTAAAGTGGGCATGTGTAGGTATGGAA |
| | 369 | AGGCTGCCCACGACC |
| | 370 | CAATACCTAAAGGCTGCC |
| | 371 | CACCAATAGCAGGTACACAACC |
| | 372 | GGATGATACAGAAAGTGCTCATGCAGCTGTTTGCTTATAATCAACTGACACA |
| | 373 | CCTGAAACACAACGTTTGGTT |
| | 374 | GCCACTTAGGCCAATACCTAAAGGCATGTGTAGGTATGGAAATTGG |
| | 375 | GCCACTTAGGCCAATACCTAAAGTGGGCATGTGTAGGTATGGAA |
| | 376 | CTGTTCAAGAATGGTAGGATCC |
| | 377 | TCCACTGTTCAAGAATGGTAGG |
| | 378 | TAACTATTAGCACTGCCACTGCATAAGCCATTACCTCTGTAGTTAAAG |
| | 379 | TAACTATTAGCACTGCCACTGCGTGTGTAAATAAGCCATTACCTCTG |
| Fourth sequence group | 380 | ATATACTCTGCTACTCCCAGTG |
| | 381 | GGCCGTGACCCTATAGAAAG |
| | 382 | GCTGATTGTTCCAGCAAATGCCGGTCTATGATAACATCTGATTCTC |
| | 383 | ATTATTGTGACCCTGCGCACGGATACTCTGCTACTCCCAGTGGG |
| | 384 | TAAGCCATTACCTCTGTAGTTAAAG |
| | 385 | TAACTATTAGCACTGCCACTGCCTTCCCCATGCCTAATATATTGC |
| | 386 | ATACTACCAGAAGTACAAATTTAACCTTCCCCATGCCTAATATATTGC |
| | 387 | GCACAACAAGATGTTAGAGATAACACCCAATAGGTGGAGCACAGCCT |
| | 388 | TTGCATGTAGTGCCAATACCC |
| | 389 | GCATGTAGTGCCAATACCCC |
| | 390 | TGCACAACAAGATGTTAGAGATAACACAATAGGTGGAGCACAGCCT |
| | 391 | GCACAACAAGATGTTAGAGATAACAATAGGTGGAGCACAGCC |
| | 392 | GCACAACAAGATGTTAGAGATAAATAGGTGGAGCACAGCC |
| | 393 | GCACAACAAGATGTTAGAGATCAATAGGTGGAGCACAGCC |
| | 394 | TGCTATGCGTGAATTTTCTGTGTCTTGGTGTTGGCCTTAGTGGTCA |
| | 395 | GTTGAGGTGGGCAGAGGAC |
| | 396 | CCAGACACAGATAGGTTGGTG |
| | 397 | CTATGCGTGAATTTTCTGTGTCATCCCTTGGTGTTGGCCTTAGT |
| | 398 | CTATGCGTGAATTTTCTGTGCCCTTGGTGTTGGCCTTAG |
| | 399 | GCTATGCGTGAATTTTCTGTGCCCTTGGTGTTGGCCTTAG |
| | 400 | CATCATATTTATTAAATAAGGGATG |
| | 401 | TTTATTAAATAAGGGATGACCA |
| Fourth sequence group | 402 | CATATTTATTAAATAAGGGATGACCAC |
| | 403 | TTGCATGTAGTGCCAATACCC |
| | 404 | TGCACAACAAGATGTTAGAGATAACACAATAGGTGGAGCACAGCCT |
| | 405 | GTTGAGGTGGGCAGAGGAC |
| | 406 | CTATGCGTGAATTTTCTGTGTCATCCCTTGGTGTTGGCCTTAGT |
| | 407 | CCTCAGCACATAAAGTCATG |
| | 408 | GTACTGGTTACAACGTGCGCA GTGGTATCCACAACTGTGAC |
| | 409 | GGGTCTAACTCTGGCAAT |
| | 410 | GGATTCTGAGGTTACCATAGAACC ACTGCCACTGTACAAAGC |
| | 411 | CCTCAGCACATAAAGTCATG |
| | 412 | CAGGGCCACAATAATGGCAT ACGAGTGGTATCCACAAC |
| | 413 | GGGTCTAACTCTGGCAAT |
| | 414 | GGATTCTGAGGTTACCATAGAACCACTGCCACTGTACAAAGC |
| | 415 | GTCCTCTAAAATAGTGGCATCC |
| | 416 | GGGGTAAGGCCAAATTGCC |
| | 417 | CCACTCGTAGCACTAACATGACGTATGTCATAACATCAGCTGTTAATG |
| | 418 | CCACTCGTAGCACTAACATGACCCTCTAAAATAGTGGCATCCATC |
| | 419 | GCTTTTTTTCCTACTCCTAGTGG |
| | 420 | GCCACTGTACAAAGCAGTGC |
| | 421 | ACTGATTGCCCCAACATATGCCTTCTATGGTAACCTCAGAATCCC |
| | 422 | ATTATTGTGGCCCTGCGCACGTTCTATGGTAACCTCAGAATCCC |
| | 423 | CCCTGCGCACGTTGTAACTATGGTAACCTCAGAATCCC |
| Fourth sequence group | 424 | CCAACATATGCCATTATTGTGCTATGGTAACCTCAGAATCCC |
| | 425 | ACCACTCGTAGCACTAACATGACTCGCCATGACGAAGGTATTCCT |
| | 426 | CCACTCGTAGCACTAACATGGCCATGACGAAGGTATTCC |
| | 427 | CCACTCGTAGCACTAACATGACGCCATGACGAAGGTATTCC |
| | 428 | GTATGTCATAACATCAGCTGTTAATG |
| | 429 | TGAATGTATGTCATAACATCAGCTG |
| | 430 | CCACTCGTAGCACTAACATGACTCGCCATGACGAAGGTATTCC |
| | 431 | GCTGAGGTTAAAAAGGAAAGCACA |
| | 432 | GACTTTATGTGCTGAGGTTAAAAAGG |
| | 433 | CTTTATGTGCTGAGGTTAAAAAGGAAAG |
| | 434 | CCAGTACGGTTTATTAAATAATT |
| | 435 | GCGCACGTTGTAACCAGTAC |
| | 436 | GTTGTAACCAGTACGGTTTATTAAATA |
| | 437 | CGTTGTAACCAGTACGGTTTATTAAAT |
| | 438 | CACCTATAGGAGGTTTGCATCC |
| | 439 | TGATACTGAAACCAGTAACAAATATGCTAACTGAGTCTGCTTATAATCCATAG |
| | 440 | CCAGATACATCTTTTTATAACCCAG |
| | 441 | AAAGGATGCCCACTAATACCCACAAAACCCAAAGGTTGGTGTG |
| | 442 | CCTCAGCACATAAAGTCATG |
| | 443 | CAGGGCCACAATAATGGCAT AGTGGTATCCACAACTGTGA |
| | 444 | GCCACTGTACAAAGCAGT |
| | 445 | CACGTTGTAACCAGTACGGTTTTTCCTACTCCTAGTGGTTCT |
| Fourth sequence group | 446 | CACCTATAGGAGGTTTGCATCC |
| | 447 | TGATACTGAAACCAGTAACAAATATGCTAACTGAGTCTGCTTATAATCCATAG |
| | 448 | CCAGATACATCTTTTTATAACCCAG |
| | 449 | AAAGGATGCCCACTAATACCCACAAAACCCAAAGGTTGGTGTG |
| | 450 | GTCCTCCAGTAGGTTAGCATTC |
| | 451 | AGTACTAACATGACTATTAGTACTGCTGCCATAACCTCTGCAGACAAAG |
| | 452 | ATACTACTAGAAGTACTAACATGACTGCCATAACCTCTGCAGACAAAG |
| | 453 | GTATATGTTGCTACGCCTAGTG |
| | 454 | AATTGATTACCCCAGCAAATGCCGTCTATGATTACGTCTGAGGCAC |
| | 455 | CCCCAGCAAATGCCATTATTCTATGATTACGTCTGAGGCAC |
| | 456 | GCCATAACCTCTGCAGACAAAG |
| | 457 | ATACTACTAGAAGTACTAACATGACCCTCCACATGTCTAAGGTACTG |
| | 458 | GCACGTTGCAACCAATAAGG |
| | 459 | GGCTGGATGATACTGAAAGTTCCCACAACTGTGTTTGCTTGCCATC |
| | 460 | TCCAATGTTCACCCATAGC |
| | 461 | CCAATGTTCACCCATAGCGG |
| | 462 | GGATGATACTGAAAGTTCCAATTTAGCCACAACTGTGTTTGCTTGCC |
| | 463 | AAACAATGGATGGCCACTTAGCCATGTGTAGGTTTGGAGGTAGGC |
| | 464 | TTATAATCCGGACCAGGAACG |
| | 465 | ATAATCCGGACCAGGAACGG |
| | 466 | ACAATGGATGGCCACTTAGCC GCATGTGTAGGTTTGGAGGTAGG |
| | 467 | GGATGATACTGAAAGTTCCAATTTAGCCACAACTGTGTTTGCTTGCC |
| Fourth sequence group | 468 | TTATAATCCGGACCAGGAACG |
| | 469 | ACAATGGATGGCCACTTAGCCGCATGTGTAGGTTTGGAGGTAGG |
| | 470 | TCCAATGTTCACCCATAGC |
| | 471 | CAAAGTCCATGCATCCAAAC |
| | 472 | GCCTGTAACAATAATGCAGCTGCACCATGTCACCATCCTCA |
| | 473 | AACAGATATCCCGCACAG |
| | 474 | GTGGGAGGTTTACAGCCAATTAGGTCTGATAACAGGGAATGC |
| | 475 | CCATTGTTATGACCTTGTGC |
| | 476 | TCCAACTCCTAGTGGCTCTATAGCGCTGTAGCCAATAAGGC |
| | 477 | GACGTGAGCAGATGTTTGT |
| | 478 | GGATAACTGCAGTATTACCGGACCTAGGGCTGGAAAACTTGG |
| | 479 | GTTACCTCAGAATCACAATTATTTAATAAGCC |
| | 480 | CCTCAGAATCACAATTATTTAATAAGCC |
| | 481 | CAGTCCTCCAAAATATTGGAATCC |
| | 482 | AACCAAATTGCCAGTCCTCC |
| | 483 | TACCACTCGTAGCACTAATATGACATATGTCATTATCTCTGCAGTTAGTG |
| | 484 | ACCACTCGTAGCACTAATATGACTATGTCATTATCTCTGCAGTTAGTG |
| | 485 | ACCACTCGTAGCACTAATATGACATATGTCATTATCTCTGCAGTTAGTG |
| | 486 | ACCACTCGTAGCACTAATATGACGCAGTTAGTGTAATTTTGCAAAGCTG |
| | 487 | TACCACTCGTAGCACTAATATGACGCAGTTAGTGTAATTTTGCAAAGCTG |
| | 488 | GTAGTGCATTTTTTCCAACTCCTAG |
| | 489 | CTGCAGTTATCCAAAGTAGTGC |
| Fourth sequence group | 490 | CTGATTGCCCCAGCAAATGCCGCTCTATAGTTACCTCAGAATCAC |
| | 491 | CTGATTGCCCCAGCAAATGCCTTGGCTACAGCGTGCACAAGG |
| | 492 | CTGATTGCCCCAGCAAATGCCGGCTCTATAGTTACCTCAGAATCA |
| | 493 | ATGTCATTATCTCTGCAGTTAGTG |
| | 494 | ACCACTCGTAGCACTAATATGACTTCTTCAACATGACGTACATATTCC |
| | 495 | CTCACCAGTGGGAGGTTTAC |
| | 496 | CTGAAACCAGTAACAGATATCCCGGCCAATTAAACATAATTGTGTTTG |
| | 497 | TGAAACCAGTAACAGATATCCCGTCCATAGATAAGCATTCCCTG |
| | 498 | TGAAACCAGTAACAGATATCCCGAATCCATAGATAAGCATTCCC |
| | 499 | TAACCCTGATACACAACGTTTG |
| | 500 | AGGATGACCACTTACGCCAACACTCTGGGCATGTGTAGGCCTT |
| | 501 | CAATGGCTGTCCCCTACCTA |
| | 502 | ATGGCTGTCCCCTACCTATTTC |
| | 503 | CCTTTACCCCAATGCTCAC |
| | 504 | AGCCAGGGTCTGATAACAGGGGGAGGTTTACAGCCAATT |
| | 505 | AATAGGTAGGGGACAGCC |
| | 506 | GCGGGATATCTGTTACTGGTTTCAGCGTAAGTGGTCATCCTT |
| | 507 | CTCACCAGTGGGAGGTTTAC |
| | 508 | CTGAAACCAGTAACAGATATCCCGGCCAATTAAACATAATTGTGTTTG |
| | 509 | TAACCCTGATACACAACGTTTG |
| | 510 | AGGATGACCACTTACGCCAACACTCTGGGCATGTGTAGGCCTT |
| | 511 | CCAATCCTCCAAAATAGTGGTATTC |
| Fourth sequence group | 512 | CTACTCGCAGCACCAATCTTTCGTATGACATTACCTCTGTAGTTAATG |
| | 513 | CTACTCGCAGCACCAATCTTTCGAATGTATGACATTACCTCTGTAG |
| | 514 | CTACTCGCAGCACCAATCTTTCTGAATGTATGACATTACCTCTGTAG |
| | 515 | ACAATTGATTGTGCCAACATATACCGGTCTGTGGTTACTTCTGATTCAC |
| | 516 | TATATTCCCCTTCCCCAAGTG |
| | 517 | CAATTGATTGTGCCAACATATACCGGTCTGTGGTTACTTCTGATTCAC |
| | 518 | TTAAACCCTGAGCCTTGTGCAGGGTCTGTGGTTACTTCTGATTCAC |
| | 519 | GTATGACATTACCTCTGTAGTTAATG |
| | 520 | GAATGTATGACATTACCTCTGTAG |
| | 521 | TGAATGTATGACATTACCTCTGTAG |
| | 522 | CTACTCGCAGCACCAATCTTTCCCTCCACATGTCTGGCATATTC |
| | 523 | GCAGGTACACAGCCAATAATACAC |
| | 524 | GATGA CACTGAAAACTCTCATGTAGCGCTGAGTTTGTTTATAATCCACAG |
| | 525 | GATGACACTGAAAACTCTCATGCTGAGTTTGTTTATAATCCACAG |
| | 526 | CCAGATAACACAGTATATGATCCTAAC |
| | 527 | CACTGAGTCCTACCCCTAAAGGTTGTCTCAACGCTTGGTCTGG |
| | 528 | CACTGAGTCCTACCCCTAAAGGTCTCAACGCTTGGTCTGG |
| | 529 | GCTGTTGATACCAAAGATACACGTG |
| | 530 | CTGTTGATACCAAAGATACACGTGATA |
| | 531 | GATACCAAAGATACACGTGATAATG |
| | 532 | GCATCTGCTGTTGATACCAAAGATAC |
| | 533 | GATTTCAACACCTACACAGGC |
| Fourth sequence group | 534 | GCAGGTACACAGCCAATAATACAC |
| | 535 | GATGA CACTGAAAAC TCTCATGTAG CGCTGAGTTTGTTTATAATCCACAG |
| | 536 | CCAGATAACACAGTATATGATCCTAAC |
| | 537 | CACTGAGTCCTACCCCTAAAGGTTGTCTCAACGCTTGGTCTGG |
| | 538 | GTGCCTTGTGCAGCCAAT |
| | 539 | GTAGTTATGTATATGCCCCCTCGCCGCTTGTTAAATAACTGGGAGTCTGA |
| | 540 | ACGTAGGGAACAGTTATTTGCTAG |
| | 541 | TGTCACGTATGTCAGTGCCCTTAATGGAATAGAGGGGGCATGGT |
| | 542 | AATGGCAGGAACACAGCC |
| | 543 | TGAAAATTCCCCGTTTTCCTCCAACGCGTTTGTTTATAGTCCACTGAAAC |
| | 544 | TGATACGCAGCGATTGGTATG |
| | 545 | TGCCCACTAAGGCCAACACCGGCCTGTGTTGGTGTTGAA |
| | 546 | GTTAAATAACTGGGAGTCTGAGGAT |
| | 547 | TAAGGGCACTGACATACGTGACAGCCATAGACCCACTAGGCGAG |
| | 548 | CTGCAGATGTATATGGAGACAGTA |
| | 549 | AGTGTCCCCTACCATGCCCCACGTAGGGAACAGTTATTTGCT |
| | 550 | TGTGTCCCTGTGCCTTGT |
| | 551 | GTAGTTATGTATATGCCCCCTCGCCGCAGCCAATAGGGCTTGTT |
| | 552 | CGCAGTACCAATTTTACTTTGTCTACTACTACCTCAACATGCCTAATATATTCC |
| | 553 | ACTGACATACGTGACAGTCCTAG |
| | 554 | GATTATGCCAACAAATACCATTGTTGTTCCCAGTTATTTAACAAGCCC |
| | 555 | CAGTAAGAAATAATTGATTATGCCAACAAACAAGCCCTATTGGCTGC |
| Fourth sequence group | 556 | CCACTCGCAGTACCAATTTTACTTTGCCTCAACATGCCTAATATATTCC |
| | 557 | ATACCACTCGCAGTACCAATTTTACCCTCAACATGCCTAATATATTCC |
| | 558 | TCAGTGGACAATGTTATAGTACAC |
| | 559 | CCACTCGCAGTACAAATTTTACTTTGCCTCAACATGCCTAATATATTCC |
| | 560 | ATACCACTCGCAGTACAAATTTTACCCTCAACATGCCTAATATATTCC |
| | 561 | CATCAGTGGATAATGTTATAGTACAC |
| | 562 | GCGCAGTACTAATTTTACATTGTCCCCTCAACATGCCTAACATATTCC |
| | 563 | ATACAACGCGCAGTACTAATTTTACCCTCAACATGCCTAACATATTCC |
| | 564 | GATTTACCTTTGGCCCAGTG |
| | 565 | TAGATGATACTGAAAATTCCCCGTTGCCTATAATACATAGTTGCGTTTG |
| | 566 | CCTGAGTCTACATTATATAACCCTGA |
| | 567 | CCCACTAAGGCCAACACCTAATGTACGCAGCGATTGGTATGG |
| | 568 | CAGCTGATTCAGTAGTAGTAGACAA |
| | 569 | GGCACAGGGACACAACAATGGTAAATTGGTACTGCGAGTGGTA |
| | 570 | TGACATACGTGACAGTCCTAGT |
| | 571 | TTGTGCAGCCAATAGGGCTTGTTAAGTATATGCCCCCTCGCCTA |
| | 572 | CTCTATTCCAAAAATGCCTAGCA |
| | 573 | CCTAGTAGTTATGTATATGCCCCCTC |
| | 574 | TACAATTCAGTAGGTATAGTGTCCCCT |
| | 575 | GTGGGTCTATGGTATCCTCAGACTC |
| | 576 | CCACTCGCAGTACCAATTTTACTTTGCATCAGTGGACAATGTTATAGTACAC |
| | 577 | TACCACTCGCAGTACCAATTTTACCATCAGTGGACAATGTTATAGTACAC |
| Fourth sequence group | 578 | CCATTGTTGTGTCCCTGTGCCTCTATGGTATCCTCAGACTCCC |
| | 579 | CAACAAATACCATTGTTGTGTCCCTCTATGGTATCCTCAGACTCCC |
| | 580 | CCTCCAACAAAAATCCTAAGGACAGCCAATGGCAGGAACACAGCC |
| | 581 | CTCCAACAAAAATCCTAAGGACAGCCAATGGCAGGAACACAGCCT |
| | 582 | GATTTACCTTTGGCCCAGTGC |
| | 583 | TATAATGGATGCCCACTAAGGCCGCCTGTGTTGGTGTTGAAATAGG |
| | 584 | ACCCTGATACGCAGCGATTG |
| | 585 | GTTATGTATATGCCCCCTCG |
| | 586 | CAACGCGCAGTACTAATTTTACATTGCATCAGTGGATAATGTTATAGTACAC |
| | 587 | TACAACGCGCAGTACTAATTTTACCATCAGTGGATAATGTTATAGTACAC |
| | 588 | GTTATGTGTATGCCCCCTCG |
| | 589 | CAACAAATACCATTGTTGTGTCCCTCTATGGTGTCCTCTGACTCCC |
| | 590 | CCAATCATCCAAAATAGCAGGATTC |
| | 591 | TAGATGATACTGAAAATTCCCCGTTGCCTATAATACATAGTTGCGTTTG |
| | 592 | CCTGAGTCTACATTATATAACCCTGA |
| | 593 | CCCACTAAGGCCAACACCTAATGTACGCAGCGATTGGTATGG |
| | 594 | GATTTACCTTTGGCCCAGTG |

In addition, typical examples of the sequences in the fifth sequence group corresponding to the nucleic acid probe will be shown below.

**Table 5**

| Sequence group | SEQ ID No. | Sequence (5' → 3') |
|---|---|---|
| Fifth sequence group | 595 | ATTATGTGCTGCCATATCTACTTCAGAAACTAC |
| | 596 | AACCAATAAGGTTTATTGAATATTTGGGCATC |
| | 597 | ACCAATAAGGTTTATTGAATATTTGGGCATCAGA |
| | 598 | CAATAAGGTTTATTGAATATTTGGGCATCAG |
| | 599 | ATAAGGTTTATTGAATATTTGGGCATCAGAG |
| | 600 | AAGGTTTATTGAATATTTGGGCATCAGAGG |
| | 601 | AAATGCAGGTGTGGATAATAGAGAATGTAT |
| | 602 | AAATGCAGGTGTGGATAATAGAGAATGTA |
| | 603 | ATTATGTGCTGCCATATCTACTTCAGAAAC |
| | 604 | GTGCTGCCATATCTACTTCAGAAACTACAT |
| | 605 | TATGTGCTGCCATATCTACTTCAGAAACTACATA |
| | 606 | ACTTCAGAAACTACATATAAAAATACTAACTTTAA |
| | 607 | TTATGTGCTGCCATATCTACTTCAGAAACT |
| | 608 | CTACTTCAGAAACTACATATAAAAATACTAACTT |
| | 609 | TCAGAAACTACATATAAAAATACTAACTTTAAGGAG |
| | 610 | AACTACATATAAAAATACTAACTTTAAGGAGTACCTA |
| Fifth sequence group | 611 | ATGTGCTGCCATATCTACTTCAGAAACTACATATAAAA |
| | 612 | TGCCATATCTACTTCAGAAACTACATATAAAAATACT |
| | 613 | TCTACACAGTCTCCTGTACCTGGGCAATATG |
| | 614 | AATATGTGCTTCTACACAGTCTCCTGTACCT |
| | 615 | CTCCTGTACCTGGGCAATATGATGCTACCAA |
| | 616 | CACGTCTAATGTTTCTGAGGACGTTAGGGA |
| | 617 | GTCTAATGTTTCTGAGGACGTTAGGGA |
| | 618 | TAATGTTTCTGAGGACGTTAGGGA |
| | 619 | TAATGTTTCTGAGGACGTTAGGGACAATGTG |
| | 620 | TAATGTTTCTGAGGACGTTAGGGACAATG |
| | 621 | AATGTTTCTGAGGACGTTAGGGACAATGTG |
| | 622 | AATATGTGCTTCTACACAGTCTCCTGTACC |
| | 623 | TGCTTCTACACAGTCTCCTGTACCTGGGCA |
| | 624 | TGCTTCTACACAGTCTCCTGTACCTGGGCA |
| | 625 | ACCTGGGCAATATGATGCTACCAAATTTAA |
| | 626 | CCTGTACCTGGGCAATATGATGCTACCAAATTTAA |
| | 627 | TGGTCCTGGCACTGATAATAGGGAATGTATATCAATGG |
| Fifth sequence group | 628 | ATAATAGGGAATGTATATCAATGGATTATAAACAAACAC |
| | 629 | GGCACTGATAATAGGGAATGTATATCAATGGATTATAAA |
| | 630 | TGGTCCTGGCACTGATAATAGGGAATGTAT |
| | 631 | ATAATAGGGAATGTATATCAATGGATTATAAA |
| | 632 | ATAATAGGGAATGTATATCAATGGATTATAAAC |
| | 633 | ATAATAGGGAATGTATATCAATGGATTATAAACAAAC |
| | 634 | TGCTGCAATTGCAAACAGTGATACTACATT |
| | 635 | AACAGTGATACTACATTTAAAAGTAGTAATTTTAA |
| | 636 | TTATCCATGGATTATAAACAAACACAGTTATGTTT |
| | 637 | TTATCCATGGATTATAAACAAACACAGTTATGTTTAC |
| | 638 | TTATCCATGGATTATAAACAAACACAGTTATGTTTACTTGGA |
| | 639 | ACAAGGTCATAATAATGGTATTTGTTGGGG |
| | 640 | GTAGTTCCTGAACCTTTAATGTACAGGTCA |
| | 641 | CAAGGTCATAATAATGGTATTTGTTGGGGC |
| | 642 | ATGTTTATCCATGGATTATAAACAAACACAGTTAT |
| | 643 | TTATCCATGGATTATAAACAAACACAGTTA |
| | 644 | TTATCCATGGATTATAAACAAACACAGTTATGT |
| Fifth sequence group | 645 | TGGACAACCGGGTGCTGATAATAGGGAATG |
| | 646 | TGGACAACCGGGTGCTGATAATA |
| | 647 | GATAATAGGGAATGTTTATCCATGGATTATAAACAA |
| | 648 | AGGGAATGTTTATCCATGGATTATAAACAAACAC |
| | 649 | AGGGAATGTTTATCCATGGATTATAAACAA |
| | 650 | AATGTTTATCCATGGATTATAAACAAACACAGT |
| | 651 | ACTTTATGCACACAAGTAACTAGTGACAGT |
| | 652 | ACTAGTGACAGTACATATAAAAATGAAAATTTTAA |
| | 653 | ATGGATTTTACTACATTACAAGCTAATAAAA |
| | 654 | TTTGGTGCAATGGATTTTACTACATTACAAGCTA |
| | 655 | GTGCAATGGATTTTACTACATTACAAGCTAATA |
| | 656 | ATAACAGGGAATGCATTTCTATGGATTAT |
| | 657 | TTCTGCTGTGTCTTCTAGTGACAGTACATA |
| | 658 | TCTAGTGACAGTACATATAAAAATGACAATTTTAA |
| | 659 | TCTATAGAGTCTTCCATACCTTCTACATATGATCCT |
| | 660 | TGGGCCTTATGTAGCCAATAAGGCTTATTAAATAACTG |
| | 661 | GCCAATAAGGCTTATTAAATAACTGGGAATCAGAG |
| Fifth sequence group | 662 | CAATAAGGCTTATTAAATAACTGGGAATCA |
| | 663 | AATAAGGCTTATTAAATAACTGGGAATCAGA |
| | 664 | TCTTCCATACCTTCTACATATGATCCTTCTAA |
| | 665 | TCCATACCTTCTACATATGATCCTTCTAAGTTTAAGGAAT |
| | 666 | TTATCTACCTCTATAGAGTCTTCCATACCTTCTACA |
| | 667 | ATACCTTCTACATATGATCCTTCTAAGTTTAAG |
| | 668 | TTCTACATATGATCCTTCTAAGTTTAAGGAATATACC |
| | 669 | TATGTAGCCAATAAGGCTTATTAAATAACTGGGA |
| | 670 | ACCAATAAGGACAGTAGGGATAATGTGTCT |
| | 671 | ACCAATAAGGACAGTAGGGATAATGTGT |
| | 672 | ACCAATAAGGACAGTAGGGATAATGTG |
| | 673 | CCTCTATAGAGTCTTCCATACCTTCTACAT |
| | 674 | TCCATACCTTCTACATATGATCCTTCTAAGTTTAA |
| | 675 | GTTATTACGCAGGATGTTAGGGATAATGTG |
| | 676 | AGCTACAGCTGTTATTACGCAGGATGTTAGG |
| | 677 | ACGCAGGATGTTAGGGATAATGTGTCAGTTGAT |
| | 678 | CTACAGCTGTTATTACGCAGGATGTTAGGGATAATGTGTC |
| Fifth sequence group | 679 | ACAGCTGTTATTACGCAGGATGTTAGGGAT |
| | 680 | TGTTATTACGCAGGATGTTAGGGATAATGT |
| | 681 | TTACGCAGGATGTTAGGGATAATGTGTCAG |
| | 682 | TACACAAAATCCTGTGCCAAGTACATATGA |
| | 683 | CCTGTGCCAAGTACATATGACCCTACTAAGTTTAA |
| | 684 | GACAACAAACAGACTCAGTTATGTATAATAGGCTGTGC |
| | 685 | TCATCATATTTATTAAATAAGGGATGACCACT |
| | 686 | ACATCTGTTGACAACAAACAGACTCAGTTATGTA |
| | 687 | ATCATATTTATTAAATAAGGGATGACCACTAAGG |
| | 688 | TGTTGACAACAAACAGACTCAGTTATGTATAAT |
| | 689 | TATTTATTAAATAAGGGATGACCACTAAGGCCA |
| | 690 | TTGACAACAAACAGACTCAGTTATGTATAATAGGCT |
| | 691 | GCGGTTTCCCCAACATTTACTCCAAGTAACTTT |
| | 692 | TCCCCAACATTTACTCCAAGTAACTTTAAGC |
| | 693 | AAACAGACTCAGTTATGTATAATAGGCTGTG |
| | 694 | CAGACTCAGTTATGTATAATAGGCTGTGCT |
| | 695 | TCTGTTGACAACAAACAGACTCAGTTATGTATAATAGG |
| Fifth sequence group | 696 | TCTGTTGACAACAAACAGACTCAGTTATGTATAAT |
| | 697 | GTTGACAACAAACAGACTCAGTTATGTATAATAGG |
| | 698 | TGTTGACAACAAACAGACTCAGTTATGTATAATAGG |
| | 699 | TCTGTTGACAACAAACAGACTCAGTTATGTATAATAGGCT |
| | 700 | GCTGCGGTTTCCCCAACATTTACTCCAAGT |
| | 701 | GCGGTTTCCCCAACATTTACTCCAAGTAAC |
| | 702 | GCGGTTTCCCCAACATTTACTCCAAGTAACTTTAA |
| | 703 | TAATGGCATATGTTGGGGCAATCAGTTGTTTGTCACAG |
| | 704 | ACTAGGAGTAGGAAAAAAAGCACTGCTTTG |
| | 705 | GGGCCACAATAATGGCATATGTTGGGGCAATCAGTTGTT |
| | 706 | TATGTTGGGGCAATCAGTTGTTTGTCACAGTT |
| | 707 | TAGGAGTAGGAAAAAAAGCACTGCTTTGTA |
| | 708 | CACAATAATGGCATATGTTGGGGCAATCAGT |
| | 709 | ACAATAATGGCATATGTTGGGGCAATCAGTTGTTTGT |
| | 710 | TACGGTTTATTAAATAATTGGGATTCTGAG |
| | 711 | GAGGTTAAAAAGGAAAGCACATATAAAAATGAAAATTTTA |
| | 712 | TTTATGTGCTGAGGTTAAAAAGGAAAGCACA |
| Fifth sequence group | 713 | TTGTAACCAGTACGGTTTATTAAATAATTGGGA |
| | 714 | GTTAAAAAGGAAAGCACATATAAAAATGAAAAT |
| | 715 | TAAAAAGGAAAGCACATATAAAAATGAAAATTTTAAGGAA |
| | 716 | GGAAAGCACATATAAAAATGAAAATTTTAAGGAATACCTT |
| | 717 | TGTAACCAGTACGGTTTATTAAATAATTGGGATTCTGA |
| | 718 | TGTGCTGAGGTTAAAAAGGAAAGCACATATAAAAATGAAA |
| | 719 | CCAGTACGGTTTATTAAATAATTGGGATTC |
| | 720 | CTGAGGTTAAAAAGGAAAGCACATATAAAAA |
| | 721 | TTAAAAAGGAAAGCACATATAAAAATGAAAAAT |
| | 722 | CTGAGGTTAAAAAGGAAAGCACATATAAAAAT |
| | 723 | TGCTGAGGTTAAAAAGGAAAGCACATATAAA |
| | 724 | AAAAAGGAAAGCACATATAAAAATGAAAATTTTAAGGA |
| | 725 | TGCTGAGGTTAAAAAGGAAAGCACATATAAAAATGAAAA |
| | 726 | TGCTGAGGTTAAAAAGGAAAGCACATATAAAAAT |
| | 727 | TTTATGTGCTGAGGTTAAAAAGGAAAGCACATATAAAAATGAAAA |
| | 728 | TTTATGTGCTGAGGTTAAAAAGGAAAGCACATATAAAAAT |
| | 729 | TTTATGTGCTGAGGTTAAAAAGGAAAGCACATATA |
| Fifth sequence group | 730 | GGTAAACCTGGTATAGATAATAGGGAATGT |
| | 731 | TGGTAAACCTGGTATAGATAATAGGGAATGT |
| | 732 | TTTATGTGCTGAGGTTAAAAAGGAAAGCAC |
| | 733 | CCTTGGGCACGTTGCAACCAATAAGGTTTATTAAATAACT |
| | 734 | TTAGTACTGCTACAGAACAGTTAAGTAAATATGATGCACG |
| | 735 | GGCACGTTGCAACCAATAAGGTTTATTAAATAACTGTGCC |
| | 736 | CGTTGCAACCAATAAGGTTTATTAAATAACTGTGCCTC |
| | 737 | GCTACAGAACAGTTAAGTAAATATGATGCACGAAAAAT |
| | 738 | TTATTATGGCCTTGGGCACGTTGCAACCAATAAGGTTT |
| | 739 | ACAGAACAGTTAAGTAAATATGATGCACGAAAA |
| | 740 | ACCAATAAGGTTTATTAAATAACTGTGCCTCAGAC |
| | 741 | AGAACAGTTAAGTAAATATGATGCACGAAAAATTAATCAG |
| | 742 | TAACATGACTATTAGTACTGCTACAGAACAGTTAAGTAAA |
| | 743 | GTAAATATGATGCACGAAAAATTAATCAGTACCTTAG |
| | 744 | TGACTATTAGTACTGCTACAGAACAGTTAAGTAAATATGA |
| | 745 | GAACAGTTAAGTAAATATGATGCACGAAAAA |
| Fifth sequence group | 746 | TACTGCTACAGAACAGTTAAGTAAATATGATGCACG |
| | 747 | AATAATAATGTTATAGAAGATAGTAGGGAC |
| | 748 | ATAGTAGGGACAATATATCAGTTGATGGCA |
| | 749 | TATTAGTACTGCTACAGAACAGTTAAGTAA |
| | 750 | ACAGAACAGTTAAGTAAATATGATGCACGA |
| | 751 | GAACAGTTAAGTAAATATGATGCACGAAAAATTAA |
| | 752 | AGGTCATCCGGGACAGCCTCGCCAAGTTTT |
| | 753 | TTACCTCAGAATCACAATTATTTAATAAGCCT |
| | 754 | CTTTAATATAAAGGTCATCCGGGACAGCCTCG |
| | 755 | CAGAATCACAATTATTTAATAAGCCTTATT |
| | 756 | CCAATGGCTGTCCCCTACCTATTTCAAGGC |
| | 757 | CACAGCCAGGGTCTGATAACAGGGAATGCTT |
| | 758 | TGGCTGTCCCCTACCTATTTCAAGGCCTAC |
| | 759 | GGGTCTGATAACAGGGAATGCTTATCTATG |
| | 760 | CACAGCCAGGGTCTGATAACAGGGAATGCT |
| Fifth sequence group | 761 | CAGGGAATGCTTATCTATGGATTATAAACA |
| | 762 | CACTGAAGTAACTAAGGAAGGTACATATAA |
| | 763 | ATTATGCACTGAAGTAACTAAGGAAGGTAC |
| | 764 | ACTAAGGAAGGTACATATAAAAATGATAATTTTAA |
| | 765 | TGATACCAAAGATACACGTGATAATGTATCTG |
| | 766 | ATCTGCTGTTGATACCAAAGATACACGTGA |
| | 767 | GCCCCGACCGATTTCAACACCTACACAGGC |
| | 768 | GCCCCGACCGATTTCAACACCTACACAGGCCCAGACCAA |
| | 769 | ACCAAAGATACACGTGATAATGTATCTGTGGATTATA |
| | 770 | CCCGACCGATTTCAACACCTACACAGGCCCAGAC |
| | 771 | CTGTTGATACCAAAGATACACGTGATAATG |
| | 772 | GTTGATACCAAAGATACACGTGATAATGTATCTGTGGA |
| | 773 | CGATTTCAACACCTACACAGGCCCAGACCA |
| | 774 | ATCTGCTGTTGATACCAAAGATACACGTGA |
| | 775 | CTGCTGTTGATACCAAAGATACACGTGA |
| | 776 | TCTGCTGTTGATACCAAAGATACACGTGAT |
| Fifth sequence group | 777 | ATCTGCTGTTGATACCAAAGATACACGTGATAATG |
| | 778 | GTTGATACCAAAGATACACGTGATAATGTATCTGTGG |
| | 779 | TCTGCTGTTGATACCAAAGATACACGTGATAATGTATCTG |
| | 780 | TCTATTCCTAATGTATACACACCTACCAGT |
| | 781 | TCTATTCCTAATGTATACACACCTACCAGTTTTAA |
| | 782 | TCCTAGTAGTTATGTATATGCCCCCTCGCCT |
| | 783 | AGTAGTTATGTATATGCCCCCTCGCCTAGT |
| | 784 | AGTAGTTATGTGTATGCCCCCTCGCCTAGC |
| | 785 | TGAATCAGCTGTACCAAATATTTATGATCCT |
| | 786 | AGACTCTACTGTACCAGCTGTGTATGATTCT |
| | 787 | TTCCTCCAACAAAAATCCTAAGGACAGTAG |
| | 788 | TTCCTCCAACAAAAATCCTAAGGACAGTA |
| | 789 | TCCTAAGGACAGTAGGGAATATGTTTCAGT |
| | 790 | TCAGTGGACTATAAACAAACGCAACTATG |
| | 791 | TCCACTACTACAGACTCTACTGTACCAGCT |
| | 792 | GCTGTACCAAATATTTATGATCCTAATAAATTTAA |
| | 793 | TTTGTCTACTACTACTGAATCAGCTGTACCAAA |
| | 794 | ACTGTACCAGCTGTGTATGATTCTAATAAATTTAA |

Hereinafter, the method of detecting a nucleic acid according to the present invention will be described specifically with reference to Examples.

### (1) Synthetic oligonucleotide

A nucleic acid primer for use in the detection of human papilloma virus and identification of its genotype is prepared in combination of the sequences described in Table 1 above. More specifically, sequences in the first and the second sequence groups, sequences in the first and third sequence groups, or sequences complementary to the sequences in the second sequence groups and sequences in third sequence groups are bound to each other directly or via a spacer. Alternatively, a sequence in the fourth sequence group or a sequence complementary thereto is prepared. Any method known to those skilled in the art may be used in preparing the primers.

### (2) LAMP reaction solution

The LAMP reaction solution had the composition shown in the following Table 6. The template used was a plasmid DNA containing cloned HPV16.

**Table 6**

| Reagent | Volume |
|---|---|
| Sterilized ultrapure water | 1.5 µL |
| Bst DNA polymerase | 1 µL |
| Buffer | 12.5 µL |
| Tris HCl (pH8.0) | 40 mM |
| KCl | 20 mM |
| MgSO4 | 16 mM |
| (NH₄)₂SO₄ | 20 mM |
| Tween20 | 0.2 % |
| Betaine | 1.6 M |
| dNTP | 2.8 mM |
| F3-primer (10 µM) | 0.5 µL |
| B3-primer (10 µM) | 0.5 µL |
| FIP-primer (10 µM) | 4 µL |
| BIP-primer(10 µM) | 4 µL |
| Template | 1 µL |
| (cloned HPV16) | |
| Total | 25 µL |

In addition, the nucleic acid primer for LAMP amplification reaction shown in the following Table 7 was used as the same time.

**Table 7 Nucleic acid primer sequence for LAMP amplification**

| Title | SEQ ID No. | Sequence (5' → 3') |
|---|---|---|
| FIP primer | 12 | GAAAAATAAACTGTAAATCATATTCTTTGTTACTGTGGTAGATACTAC |
| F3 primer | 13 | GCACAGGGCCACAATAATGG |
| BIP primer | 14 | TTTTTGGGAAGTAAATTTAAAGGACGTCCTAAAGGAAACTGATC ' |
| B3 primer | 15 | CCTGCTTGTAGTAAAAATTT |

### (3) Nucleic acid amplification by LAMP method

The nucleic acid amplification is carried out at a temperature of 58°C for 1 hour. A sample added with sterilized water instead of the template is used as the negative control. Analysis of the amplified LAMP products by agarose gel electrophoresis reveals a ladder-shaped pattern characteristic to LAMP products. On the other hand, no amplification is observed with a sample containing no template DNA. It is thus possible to perform sequence-specific amplification of the papilloma virus by using a selected primer set.

### (4) Preparation of nucleic acid probe-immobilized slide glass (FIG. 5)

The DNA probes 1 - 5 were immobilized on the slide glass 6. The nucleic acid sequence of them is shown in Table 8-1.

**Table 8-1 Probe sequence**

| Title | SEQ ID No. | Complementary sequence (5' → 3') |
|---|---|---|
| HPV16 | 7 | TCTGAAGTAGATATGGCAGCACATAATGAC |
| HPV18 | 8 | TGCCCAGGTACAGGAGACTGTGTAGAAGCA |
| HPV26 | 9 | AGTGGATGCAGATGCTGCAGATAATGTACT |
| HPV31 | 10 | GTATCACTGTTTGCAATTGCAGCACAAACA |
| rDNA | 11 | CTGGACGAAGACTGACGCTC |

| | | |
|---|---|---|
| *The terminal is modified by amino group. | | |

HPV's 16 to 31 were used as sequences specific to the subtypes of HPV, while the rDNA's as negative controls. Each probe was modified with an amino group at the terminal, and was immobilized on a carbodiimide-treated slide glass substrate by spotting the probe solution thereon. Finally, the substrate was washed with ultrapure water and air-dried, to give a DNA chip.

### (5) Hybridization of LAMP products to nucleic acid probe

The LAMP products amplified in (3) above were used as a nucleic acid sample. The DNA chip prepared in (4) was hybridized by dipping it into the LAMP product solution containing 2 × SSC salt added and leaving it therein at 35°C for 60 minutes. Subsequently, Cy5-labelled nucleic acid of SEQ ID No. 7 was added thereto, and the mixture was left at 35°C for 15 minutes and washed with ultrapure water slightly. The fluorescence intensity was detected by using Tyhoon manufactured by Amersham.

### (6) Results

Fluorescence measurement showed significant emission only on HPV16 probe-immobilized spots, indicating that it was possible to detect nucleic acids amplified by LAMP reaction specifically to the sequence.

### (7) Preparation of nucleic acid probe-immobilized electrode (FIG. 6)

The DNA probes 7 - 11 were immobilized on the electrode 13 placed on the support 12. The nucleic acid sequence of them is shown in Table 8-2. Connection part 14 may be placed on the support 12.

**Table 8-2 Probe sequence**

| Title | SEQ ID No. | Complementary sequence (5'→ 3') |
|---|---|---|
| HPV16 | 7 | TCTGAAGTAGATATGGCAGCACATAATGAC |
| HPV18 | 8 | TGCCCAGGTACAGGAGACTGTGTAGAAGCA |
| HPV26 | 9 | AGTGGATGCAGATGCTGCAGATAATGTACT |
| HPV31 | 10 | GTATCACTGTTTGCAATTGCAGCACAAACA |
| rDNA | 11 | CTGGACGAAGACTGACGCTC |

| | | |
|---|---|---|
| *The terminal is modified by thiol group. | | |

HPV's 16 to 31 are used as sequences specific to the subtypes of HPV, while the rDNA's as negative controls. Each probe is modified with an amino group at the terminal, and is immobilized on a gold electrode by spotting the probe solution thereon. Finally, the substrate is washed with ultrapure water and air-dried, to give a DNA chip.

### (8) Hybridization of LAMP products to nucleic acid probe

The DNA chip prepared in (7) is hybridized by dipping it into the LAMP product solution containing added 2 x SSC salt and leaving it therein at 35°C for 60 minutes. The electrode is dipped into a phosphate buffer solution containing a 50 µM intercalating agent Hoechst 33258 for 15 minutes, and the oxidative current response of the Hoechst 33258 molecule is determined.

### (9) Result

Voltammetric analysis indicates that significant current signals are detectable only on the spots carrying the immobilized HPV16 probe. For this reason, it has been clear that it is possible to detect nucleic acids amplified by LAMP reaction sequence-specifically.

### (10) Multiamplification by LAMP method 1

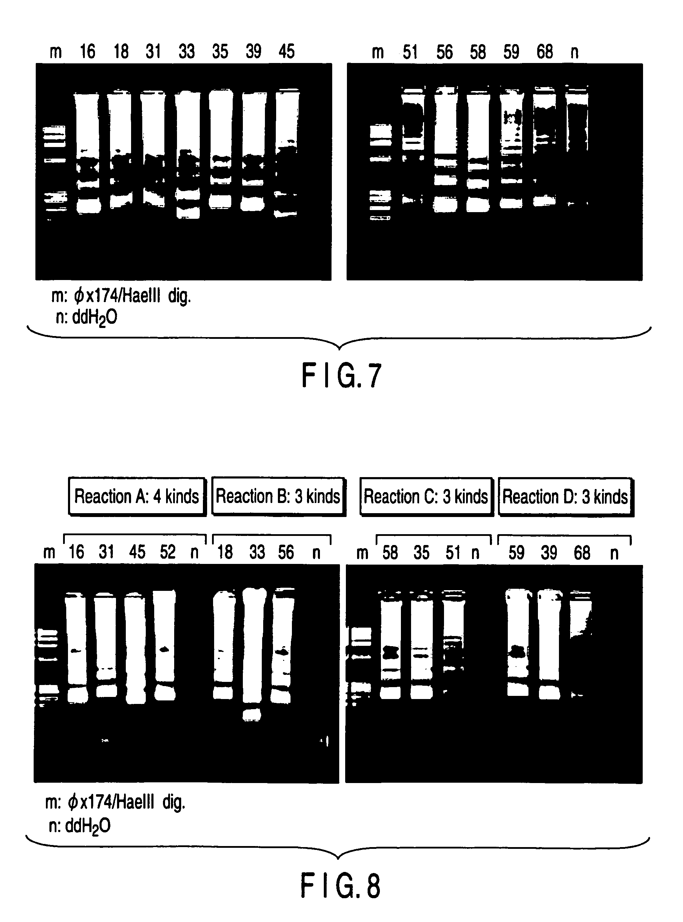

Amplification according to a kind of template was performed by using the primer shown in Table 9. As a result, a ladder-shaped band characteristic to LAMP amplification was observed, and genotype-specific amplification was confirmed (FIG. 7). Then, amplification according to a kind of template by using multiple primers, i.e., multiple mixed primer sets respectively in groups A to D, confirmed genotype-specific amplification (FIG. 8).

**Table 9**

| Group | Geno Type | SEQ ID No. |
|---|---|---|
| A | 16 | 116 |
| | | 117 |
| | | 210 |
| | | 211 |
| B | 18 | 120 |
| | | 121 |
| | | 212 |
| | | 213 |
| C | 58 | 125 |
| | | 128 |
| | | 230 |
| | | 231 |
| C | 35 | 136 |
| | | 137 |
| | | 232 |
| | | 233 |
| B | 56 | 142 |
| | | 143 |
| | | 234 |
| | | 235 |
| A | 31 | 147 |
| | | 148 |
| | | 214 |
| | | 217 |
| A | 45 | 152 |
| | | 158 |
| | | 218 |
| | | 220 |
| B | 33 | 165 |
| | | 167 |
| | | 222 |
| | | 225 |
| A | 52 | 171 |
| | | 174 |
| | | 226 |
| | | 228 |
| D | 59 | 179 |
| | | 180 |
| | | 236 |
| | | 237 |
| D | 39 | 187 |
| | | 188 |
| | | 240 |
| | | 242 |
| C | 51 | 194 |
| | | 195 |
| | | 244 |
| | | 245 |
| D | 68 | 202 |
| | | 203 |
| | | 247 |
| | | 253 |

### (11) Detection of LAMP multiamplification products 1

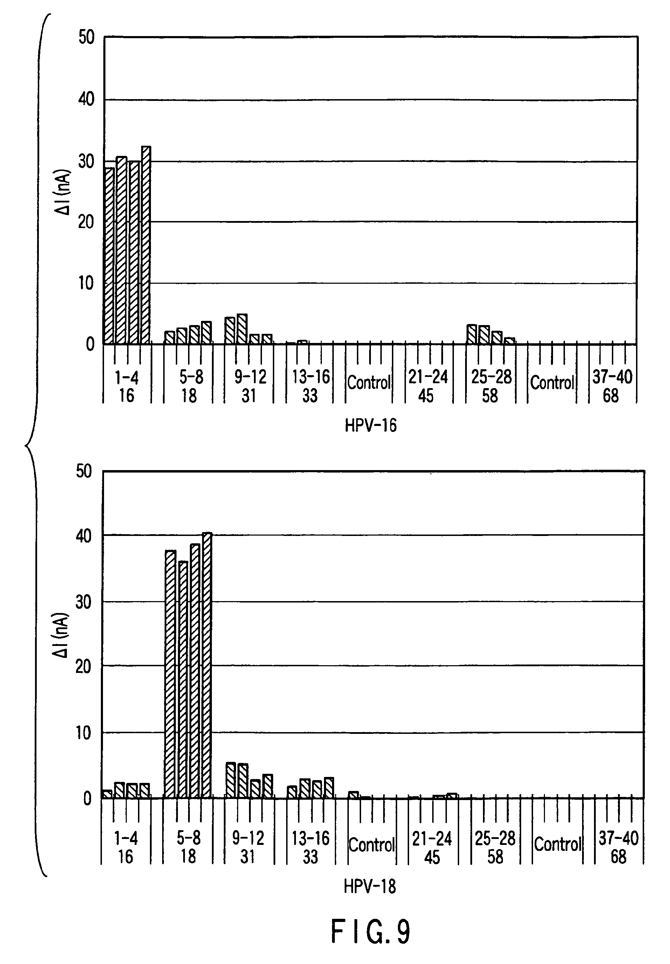

The amplification products shown in FIG. 8 were detected by using a current-detecting DNA chip carrying the probes of sequence numbers 605, 623, 635, 652, 683, 763, and 793 immobilized on the same chip. The probes are designed to react specifically with the nucleic acids of genotypes of 16, 18, 31, 33, 45, 58, and 68, respectively. Reaction of the sample amplified only by using the templates 16 and 18 resulted in increase in current only on the electrodes corresponding to the templates, indicating genotype-specific detection (FIG. 9).

### (12) Multiamplification by LAMP method 2

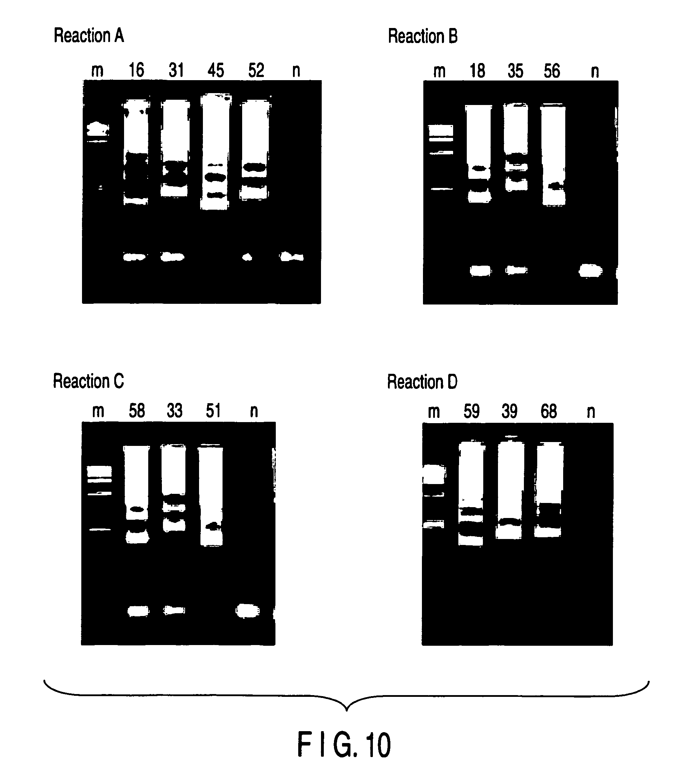

Primer sets of groups A to D shown in Table 10 were mixed respectively to give multiple primer sets. The amplification of a kind of template was performed by using the multiple primer sets. The amplification was performed at a template concentration of 10³ copies/reaction at 65 degrees for 2 hours. As a result, genotype-specific amplification was confirmed (FIG. 10).

**Table 10**

| Group | Geno Type | SEQ ID No. |
|---|---|---|
| A | 16 | 254 |
| | | 255 |
| | | 257 |
| | | 258 |
| B | 18 | 120 |
| | | 121 |
| | | 212 |
| | | 213 |
| C | 58 | 125 |
| | | 128 |
| | | 230 |
| | | 231 |
| B | 35 | 136 |
| | | 139 |
| | | 232 |
| | | 233 |
| B | 56 | 142 |
| | | 143 |
| | | 234 |
| | | 235 |
| A | 31 | 147 |
| | | 148 |
| | | 214 |
| | | 217 |
| A | 45 | 281 |
| | | 388 |
| | | 284 |
| | | 285 |
| C | 33 | 379 |
| | | 380 |
| | | 381 |
| | | 382 |
| A | 52 | 171 |
| | | 174 |
| | | 404 |
| | | 403 |
| D | 59 | 302 |
| | | 303 |
| | | 304 |
| | | 305 |
| D | 39 | 187 |
| | | 188 |
| | | 240 |
| | | 242 |
| C | 51 | 286 |
| | | 287 |
| | | 288 |
| | | 289 |
| D | 68 | 202 |
| | | 411 |
| | | 250 |
| | | 253 |

### (13) Detection of LAMP multiamplification products 2

The amplification products shown in FIG. 10 were detected by using a current-detecting DNA chip carrying the probes of SEQ ID No. 602, 623, 635, 647, 657, 681, 694, 750, 762, 774, and 793 immobilized on the same chip. The probes are designed to react specifically with the nucleic acids of genotypes of 16, 18, 31, 33, 35, 45, 51, 56, 58, 59, and 68, respectively. Reaction of the sample obtained by amplification by using each of the 11 kinds of templates resulted in increase in current only on the electrode corresponding to the template, indicating genotype specific detection of 11 kinds of genotypes.

### (14) Multiamplification by LAMP method 3

Primer sets of groups A to D shown in Table 11 were mixed respectively to give multiple primer sets. Specifically, tube A contains primer sets with primers for genotype 16, 35, and 59; tube B contains a primer set with primers for genotype 18, 39, and 56; tube C contains a primer set with primers for genotype 45, 51, 58, and 68; and tube D contains a primer set with primers for genotype 31, 33, and 52. Further, tube E contained primers of SEQ ID Nos. 801, 802, 803, and 804 prepared for amplification of human β-globin gene (Table 12). The amplification of a kind of template was performed by using the multiple primer sets respectively. A plasmid corresponding to each HPV type was used as the template, and the hybridization was performed at a concentration of 10³ copies/reaction at 63°C for 1.5 hours, which confirmed genotype-specific amplification.

**Table 11**

| Group | Geno Type | SEQ ID No. |
|---|---|---|
| A | 16 | 126 |
| | | 128 |
| | | 132 |
| | | 136 |
| | | 138 |
| | 35 | 277 |
| | | 281 |
| | | 278 |
| | | 276 |
| | | 280 |
| | 59 | 526 |
| | | 527 |
| | | 524 |
| | | 523 |
| | | 529 |
| B | 18 | 162 |
| | | 164 |
| | | 167 |
| | | 166 |
| | | 170 |
| | 39 | 319 |
| | | 320 |
| | | 327 |
| | | 324 |
| | | 328 |
| | 56 | 453 |
| | | 454 |
| | | 457 |
| | | 456 |
| | | 458 |
| C | 45 | 366 |
| | | 363 |
| | | 361 |
| | | 359 |
| | | 370 |
| | 51 | 395 |
| | | 388 |
| | | 399 |
| | | 391 |
| | | 402 |
| | 58 | 477 |
| | | 475 |
| | | 474 |
| | | 476 |
| | | 480 |
| | 53 | 548 |
| | | 546 |
| | | 549 |
| | | 547 |
| | | 572 |
| D | 31 | 210 |
| | | 208 |
| | | 203 |
| | | 205 |
| | | 214 |
| | 33 | 248 |
| | | 251 |
| | | 242 |
| | | 244 |
| | | 255 |
| | 52 | 420 |
| | | 422 |
| | | 425 |
| | | 429 |
| | | 431 |

**Table 12**

| Group | SEQ ID No. | Sequence (5' → 3') |
|---|---|---|
| | 795 | TGATGGTATGGGGCCAAGAG |
| | 796 | GAGGTCTAAGTGATGACAGCCGCTGAGGGTTTGAAGTCCAACTCC |
| | 797 | AATCTACTCCCAGGAGCAGGGCTAGTGAACACAGTTGTGTCAGAAG |
| | 798 | GAGTCAGATGCACCATGGTG |
| | 799 | TGAGGTCTAAGTGATGACAGCCGCTGAGGGTTTGAAGTCCAACTCC |
| β- globin | 800 | AATCTACTCCCAGGAGCAGGGAAGTGAACACAGTTGTGTCAGAAGC |
| | 801 | AGGGCTGAGGGTTTGAAGTC |
| | 802 | TGAGGTCTAAGTGATGACAGCCGCAACTCCTAAGCCAGTGCCAGA |
| | 803 | CTAGGGTTGGCCAATCTACTCCCAATAGATGGCTCTGCCCTGAC |
| | 804 | TGAACACAGTTGTGTCAGAAGC |

### (15) Detection of LAMP multiamplification products 3

The amplification products above were detected by using a current-detecting DNA chip carrying the probes of SEQ ID Nos. 600, 623, 630, 641, 654, 673, 676, 699, 725, 750, 752, 771, and 783 on a single chip. These probes are designed to react specifically with genotype 16, 18, 31, 33, 35, 39, 45, 51, 52, 56, 58, 59, and 68, respectively. The oligonucleotide of SEQ ID No. 813 as a probe for detecting β-globin gene was immobilized as the positive control of reaction, and the oligonucleotide of SEQ ID No. 814 was immobilized as the negative control on the same substrate (Table 13). Reaction of the sample obtained by amplification by using each of the 13 kinds of templates resulted in increase in current only on the electrode corresponding to the template, indicating genotype specific detection of the 13 kinds of genotypes.

**Table 13**

| Group | SEQ ID No. | Sequence (5'→ 3') |
|---|---|---|
| | 805 | ATAAAAGTCAGGGCAGAGCCATCTATTGCTTAC |
| | 806 | TCAGGGCAGAGCCATCTATTGCTTACATTT |
| | 807 | GTCAGGGCAGAGCCATCTATTGCTTACATTTGCTT |
| | 808 | AGGGCAGGAGCCAGGGCTGGGCATAAAAGTCAGGG |
| Positive control | 809 | AAAGTCAGGGCAGAGCCATCTATTGCTTACATTTG |
| | 810 | TGGGCATAAAAGTCAGGGCAGAGCCATC |
| | 811 | CAGGAGCAGGGAGGGCAGGAGCCAGGGCTGGGCAT |
| | 812 | CAGGGAGGGCAGGAGCCAGGGCTGGGCAT |
| | 813 | CAGGAGCAGGGAGGGCAGGAGCCAGGG |
| Negative control | 814 | GACTATAAACATGCTTTCCGTGGCA |

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.
It will be appreciated that the invention is also defined by the following clauses:
1. A nucleic acid primer for LAMP amplification for use in the detection of human papilloma virus and identification of its genotype, the nucleic acid primer being selected from the following (a)-(f);
   (a) a nucleic acid primer containing, on the same chain, a sequence complementary to a sequence selected from those in a first sequence group listed in Table 1 and a sequence selected from those in a second sequence group listed in Table 2;
   (b) a nucleic acid primer containing, on the same chain, a sequence complementary to a sequence selected from those in the first sequence group and a sequence selected from those in a third sequence group listed in Table 3;
   (c) a nucleic acid primer containing, on the same chain, a sequence complementary to a sequence selected from those in the second sequence group and a sequence selected from those in the third sequence group;
   (d) a nucleic acid primer containing a sequence that differs from a selected sequence of one of the nucleic acid primers (a), (b), and (c), by insertion, deletion, or substitution of one or more bases, and capable to hybridize with a nucleic acid chain having a sequence complementary to the selected sequence of one of the nucleic acid primers (a), (b), and (c);
   (e) a nucleic acid primer containing a sequence selected from those in a fourth sequence group listed in Table 4 or a sequence complementary thereto; and
   (f) a nucleic acid primer containing a sequence that differs from a selected sequence of one of the nucleic acid primers (e), by insertion, deletion, or substitution of one or more bases, and capable to hybridize with a nucleic acid chain having a sequence complementary to the selected sequence of one of the nucleic acid primers (e).
2. A LAMP-amplification kit for use in the detection of human papilloma virus and identification of its genotype, comprising the nucleic acid primer according to clause 1.
3. A method of detecting human papilloma virus and identifying its genotype, comprising:
   a step of amplifying the nucleic acid chains in a sample by using multiple primers including at least one primer selected from the nucleic acid primers according to clause 1 by LAMP reaction; and
   a step of detecting presence of amplified products after the amplification reaction and identifying their genotypes.
4. The method of detecting human papilloma virus and identifying its genotype, according to clause 3, wherein the step of detecting amplified products after the amplification reaction or identifying their genotypes further comprises:
   a step of hybridizing the amplification products with a virus- or genotype-specific nucleic acid chain; and,
   a step of detecting double-stranded chains formed by the hybridization to detect human papilloma virus or identifying its genotype.
5. The method of detecting human papilloma virus and identifying its genotype, according to clause 4,
   wherein the virus- or genotype-specific nucleic acid chain is immobilized on a support surface.
6. The method of detecting human papilloma virus and identifying its genotype, according to clause 4,
   wherein the virus- or gene-specific nucleic acid chain is;
   (g) a nucleic acid probe containing a sequence selected from those in a fifth sequence group listed in Table 5 or a sequence complementary thereto, or
   (h) a nucleic acid probe containing a sequence that differs from a selected sequence of one of the nucleic acid probe (g), by insertion, deletion, or substitution of one or more bases, and capable to hybridize with a nucleic acid chain having a sequence complementary to the selected sequence of one of the nucleic acid probe (g).
7. The method of detecting human papilloma virus and identifying its genotype, according to clause 4,
   wherein the support surface is made of an electrode material and the electrode is immobilized nucleic acid probes selected from the following (g) and (h);
   (g)a nucleic acid probe containing a sequence selected from those in a fifth sequence group listed in Table 5 or a sequence complementary thereto, or
   (h) a nucleic acid probe containing a sequence that differs from a selected sequence of one of the nucleic acid probe (g), by insertion, deletion, or substitution of one or more bases, and capable to hybridize with a nucleic acid chain having a sequence complementary to the selected sequence of one of the nucleic acid probe (g).
8. The method of detecting human papilloma virus and identifying its genotype, according to clause 7,
   wherein an electrochemically active nucleic acid chain-recognizing agent is used.
9. A nucleic acid chain-immobilized support for detection of human-papilloma-virus LAMP amplification products amplified by using the nucleic acid primer according to clause 1, comprising a support and a human papilloma virus- or genotype-specific nucleic acid chain immobilized on the support surface.
10. The nucleic acid chain-immobilized support according to clause 9, wherein the nucleic acid chain is;
   (g) a nucleic acid probe containing a sequence selected from those in a fifth sequence group listed in Table 5 or a sequence complementary thereto, or
   (h) a nucleic acid probe containing a sequence that differs from a selected sequence of one of the nucleic acid probe (g), by insertion, deletion, or substitution of one or more bases, and capable to hybridize with a nucleic acid chain having a sequence complementary to the selected sequence of one of the nucleic acid probe (g).
11. The nucleic acid-immobilized support according to clause 10, wherein the support is made of a permeable material or impermeable material.
12. The nucleic acid-immobilized support according to clause 11, wherein the permeable material is a porous material or a membrane.
13. The nucleic acid-immobilized support according to clause 11, wherein the impermeable material is glass or a resin.
14. The nucleic acid-immobilized support according to clause 10, wherein the surface of the support is made of an electrode material.
15. A detection kit for use in the detection of human papilloma virus and identification of its genotype, comprising the nucleic acid chain-immobilized support according to clause 10.
16. A nucleic acid chain-immobilized support for detection of the human-papilloma-virus LAMP amplification products, comprising a support and a human papilloma virus- or genotype-specific nucleic acid chain immobilized on the support surface, wherein the nucleic acid chain is;
   (g)a nucleic acid probe containing a sequence selected from those in a fifth sequence group listed in Table 5 or a sequence complementary thereto, or
   (h) a nucleic acid probe containing a sequence that differs from a selected sequence of one of the nucleic acid probe (g), by insertion, deletion, or substitution of one or more bases, and capable to hybridize with a nucleic acid chain having a sequence complementary to the selected sequence of one of the nucleic acid probe (g).
17. The nucleic acid-immobilized support according to clause 16, wherein the support is made of a permeable material or an impermeable material.
18. The nucleic acid-immobilized support according to clause 17, wherein the permeable material is a porous material or a membrane.
19. The nucleic acid-immobilized support according to clause 17, wherein the impermeable material is glass or a resin.
20. The nucleic acid-immobilized support according to clause 16, wherein the support surface is made of an electrode material.
21. A detection kit for use in the detection of human papilloma virus and identification of its genotype, comprising the nucleic acid-immobilized support according to clause 16.

## Claims

1. A set of nucleic acid primers for LAMP amplification for use in detection of genotypes of human papilloma virus, **characterized in that** the set is selected from the group consisting of:
Set 1 consisting of five nucleic acid primers consisting of a sequence of SEQ ID Nos. 136, 132, 126, 128 and 138 respectively, for detection of HPV genotype 16;
Set 2 consisting of five nucleic acid primers consisting of a sequence of SEQ ID Nos. 278, 276, 277, 281 and 280 respectively, for detection of HPV genotype 35;
Set 3 consisting of five nucleic acid primers consisting of a sequence of SEQ ID Nos. 527, 524, 526, 523 and 529 respectively, for detection of HPV genotype 59;
Set 4 consisting of five nucleic acid primers consisting of a sequence of SEQ ID Nos. 164, 167, 162, 166 and 170 respectively, for detection of HPV genotype 18;
Set 5 consisting of five nucleic acid primers consisting of a sequence of SEQ ID Nos. 320, 327, 319, 324 and 328 respectively, for detection of HPV genotype 39;
Set 6 consisting of five nucleic acid primers consisting of a sequence of SEQ ID Nos. 454, 457, 453, 456 and 458 respectively, for detection of HPV genotype 56;
Set 7 consisting of five nucleic acid primers consisting of a sequence of SEQ ID Nos. 363, 359, 366, 361 and 370 respectively, for detection of HPV genotype 45;
Set 8 consisting of five nucleic acid primers consisting of a sequence of SEQ ID Nos. 399, 391, 395, 388 and 402 respectively, for detection of HPV genotype 51;
Set 9 consisting of five nucleic acid primers consisting of a sequence of SEQ ID Nos. 474, 476, 477, 475 and 480 respectively, for detection of HPV genotype 58;
Set 10 consisting of five nucleic acid primers consisting of a sequence of SEQ ID Nos. 549, 547, 548, 546 and 572 respectively, for detection of HPV genotype 68;
Set 11 consisting of five nucleic acid primers consisting of a sequence of SEQ ID Nos. 208, 203, 210, 205 and 214 respectively, for detection of HPV genotype 31:
Set 12 consisting of five nucleic acid primers consisting of a sequence of SEQ ID Nos. 251, 244, 248, 242 and 255 respectively, for detection of HPV genotype 33; and
Set 13 consisting of five nucleic acid primers consisting of a sequence of SEQ ID Nos. 422, 425, 420, 429 and 431 respectively, for detection of HPV genotype 52.

2. A mixture of primer sets, according to claim 1, for use in LAMP multiamplification, **characterized in that** the mixture is selected from the group consisting of:
Mixture A comprising Set 1, 2 and 3;
Mixture B comprising Set 4, 5 and 6;
Mixture C comprising Set 7, 8, 9 and 10; and
Mixture D comprising Set 11, 12 and 13.

3. A detection kit for use in the detection genotypes of human papilloma virus, wherein said kit comprises at least one of Primer-Probe Set selected from:
Primer-Probe Set 1 consisting of Set 1 according to claim 1 and a nucleic acid probe consisting of a sequence of SEQ ID No. 600,
Primer-Probe Set 2 consisting of Set 2 according to claim 1 and a nucleic add probe consisting of a sequence of SEQ ID No. 654,
Primer-Probe Set 3 consisting of Set 3 according to claim 1 and a nucleic acid probe consisting of a sequence of SEQ ID No. 771,
Primer-Probe Set 4 consisting of Set 4 according to claim 1 and a nucleic acid probe consisting of a sequence of SEQ ID No. 623,
Primer-Probe Set 5 consisting of Set 5 according to claim 1 and a nucleic acid probe consisting of a sequence of SEQ ID No. 673,
Primer-Probe Set 6 consisting of Set 6 according to claim 1 and a nucleic acid probe consisting of a sequence of SEQ ID No. 750,
Primer-Probe Set 7 consisting of Set 7 according to claim 1 and a nucleic acid probe consisting of a sequence of SEQ ID No. 676,
Primer-Probe Set 8 consisting of Set 8 according to claim 1 and a nucleic acid probe consisting of a sequence of SEQ ID No. 699,
Primer-Probe Set 9 consisting of Set 9 according to claim 1 and a nucleic acid probe consisting of a sequence of SEQ ID No. 752,
Primer-Probe Set 10 consisting of Set 10 according to claim 1 and a nucleic acid probe consisting of a sequence of SEQ ID No. 783,
Primer-Probe Set 11 consisting of Set 11 according to claim 1 and a nucleic acid probe consisting of a sequence of SEQ ID No 630,
Primer-Probe Set 12 consisting of Set 12 according to claim 1 and a nucleic acid probe consisting of a sequence of SEQ ID No. 641, and
Primer-Probe Set 13 consisting of Set 13 according to claim 1 and a nucleic acid probe consisting of a sequence of SEQ ID No. 725.

4. The kit according to claim 3, wherein the nucleic acid probe is immobilized on a support surface.

5. The kit according to claim 4, wherein the support surface is an electrode.

6. A method of detecting genotypes of human papilloma virus, comprising:
a step of obtaining an amplification product by LAMP amplification of a nucleic acid chains in a sample by using nucleic acid primers;
a step of hybridizing the amplification product with a nucleic acid probe; and
a step of detecting double-stranded chains formed by the hybridization to detect human papilloma virus or identifying its genotype;
wherein the nucleic acid primers and the nucleic acid probe is selected from Primer-Probe Sets 1 - 13, as defined in claim 3.

7. The method according to claim 6, wherein the nucleic acid probe is immobilized on a support surface.

8. The method according to claim 7, wherein the support surface is made of an electrode material.

9. The method according to claim 8, wherein an electrochemically active nucleic acid chain-recognizing agent is used in the step of detecting double-stranded chains.

10. A detection kit, according to claim 3, comprising at least one Detection Set selected from Detection sets A, B, C, and D
wherein:
Detection Set A comprises at least one Primer-Probe Set selected from the group consisting of:
Primer-Probe Set 1, 2, and 3, according to claim 3;
Detection Set B comprises at least one Primer-Probe Set selected from the group consisting of:
Primer-Probe Set 4, 5, and 6, according to claim 3;
Detection Set C comprises at least one Primer-Probe Set selected from the group consisting of:
Primer-Probe Set 7, 8, 9, and 10, according to claim 3; and
Detection Set D comprises at least one Primer-Probe Set selected from the group consisting of:
Primer-Probe Set 11, 12, and 13, according to claim 3.

11. A detection kit for use in the detection of genotypes of human papilloma virus, comprising at least one mixture of primer sets, according to claim 2, and a DNA chip, wherein the DNA chip comprises nucleic acid probes consisting of a sequence of SEQ ID Nos. 600, 623, 630, 641, 654, 673, 676, 699, 725, 750, 752, 771, and 783 respectively, immobilized on a support surface.

## Patentansprüche

1. Set von Nukleinsäureprimern für die LAMP-Amplifikation zur Verwendung beim Nachweis von Genotypen des humanen Papilloma-Virus, **dadurch** charakterisiert, daß das Set ausgewählt wird aus der Gruppe bestehend aus:
Set 1 bestehend aus 5 Nukleinsäureprimern, bestehend aus einer Sequenz von SEQ ID Nos. 136, 132, 126, 128 und 138 zum Nachweis von HPV-Genotyp 16;
Set 2 bestehend aus 5 Nukleinsäureprimern, bestehend aus einer Sequenz von SEQ ID Nos. 278, 276, 277, 281 und 280 zum Nachweis von HPV-Genotyp 35;
Set 3 bestehend aus 5 Nukleinsäureprimern, bestehend aus einer Sequenz von SEQ ID Nos. 527, 524, 526, 523 und 529 zum Nachweis von HPV-Genotyp 59;
Set 4 bestehend aus 5 Nukleinsäureprimern, bestehend aus einer Sequenz von SEQ ID Nos. 164, 167, 162, 166 und 170 zum Nachweis von HPV-Genotyp 18;
Set 5 bestehend aus 5 Nukleinsäureprimern, bestehend aus einer Sequenz von SEQ ID Nos. 320, 327, 319, 324 und 328 zum Nachweis von HPV-Genotyp 39;
Set 6 bestehend aus 5 Nukleinsäureprimern, bestehend aus einer Sequenz von SEQ ID Nos. 454, 457, 453, 456 und 458 zum Nachweis von HPV-Genotyp 56;
Set 7 bestehend aus 5 Nukleinsäureprimern, bestehend aus einer Sequenz von SEQ ID Nos. 363, 359, 366, 361 und 370 zum Nachweis von HPV-Genotyp 45;
Set 8 bestehend aus 5 Nukleinsäureprimern, bestehend aus einer Sequenz von SEQ ID Nos. 399, 391, 395, 388 und 402 zum Nachweis von HPV-Genotyp 51;
Set 9 bestehend aus 5 Nukleinsäureprimern, bestehend aus einer Sequenz von SEQ ID Nos. 474, 476, 477, 475 und 480 zum Nachweis von HPV-Genotyp 58;
Set 10 bestehend aus 5 Nukleinsäureprimern, bestehend aus einer Sequenz von SEQ ID Nos. 549, 547, 548, 546 und 572 zum Nachweis von HPV-Genotyp 68;
Set 11 bestehend aus 5 Nukleinsäureprimern, bestehend aus einer Sequenz von SEQ ID Nos. 208, 203, 210, 205 und 214 zum Nachweis von HPV-Genotyp 31;
Set 12 bestehend aus 5 Nukleinsäureprimern, bestehend aus einer Sequenz von SEQ ID Nos. 251, 244, 248, 242 und 255 zum Nachweis von HPV-Genotyp 33;
Set 13 bestehend aus 5 Nukleinsäureprimern, bestehend aus einer Sequenz von SEQ ID Nos. 422, 425, 420, 429 und 431 zum Nachweis von HPV-Genotyp 52.

2. Mischung von Primer-Sets gemäß Anspruch 1 für die Verwendung bei der LAMP-Multiamplifikation, **dadurch** charakterisiert, daß die Mischung ausgewählt wird aus der Gruppe bestehend aus:
Mischung A, umfassend Set 1, 2 und 3;
Mischung B, umfassend Set 4, 5 und 6;
Mischung C, umfassend Set 7, 8, 9 und 10; und
Mischung D, umfassend Set 11, 12 und 13

3. Nachweis-Kit zur Verwendung beim Nachweis von Genotypen des humanen Papilloma-Virus, wobei das Kit mindestens ein Primer-Sondenset umfaßt, das ausgewählt wird aus:
Primer-Sondenset 1, bestehend aus Set 1 gemäß Anspruch 1 und einer Nukleinsäurensonde, bestehend aus einer Sequenz von SEQ ID No. 600,
Primer-Sondenset 2, bestehend aus Set 2 gemäß Anspruch 1 und einer Nukleinsäurensonde, bestehend aus einer Sequenz von SEQ ID No. 654,
Primer-Sondenset 3, bestehend aus Set 3 gemäß Anspruch 1 und einer Nukleinsäurensonde, bestehend aus einer Sequenz von SEQ ID No. 771,
Primer-Sondenset 4, bestehend aus Set 4 gemäß Anspruch 1 und einer Nukleinsäurensonde, bestehend aus einer Sequenz von SEQ ID No. 623,
Primer-Sondenset 5, bestehend aus Set 5 gemäß Anspruch 1 und einer Nukleinsäurensonde, bestehend aus einer Sequenz von SEQ ID No. 673,
Primer-Sondenset 6, bestehend aus Set 6 gemäß Anspruch 1 und einer Nukleinsäurensonde, bestehend aus einer Sequenz von SEQ ID No. 750,
Primer-Sondenset 7, bestehend aus Set 7 gemäß Anspruch 1 und einer Nukleinsäurensonde, bestehend aus einer Sequenz von SEQ ID No. 676,
Primer-Sondenset 8, bestehend aus Set 8 gemäß Anspruch 1 und einer Nukleinsäurensonde, bestehend aus einer Sequenz von SEQ ID No. 699,
Primer-Sondenset 9, bestehend aus Set 9 gemäß Anspruch 1 und einer Nukleinsäurensonde, bestehend aus einer Sequenz von SEQ ID No. 752,
Primer-Sondenset 10, bestehend aus Set 10 gemäß Anspruch 1 und einer Nukleinsäurensonde, bestehend aus einer Sequenz von SEQ ID No. 783,
Primer-Sondenset 11, bestehend aus Set 11 gemäß Anspruch 1 und einer Nukleinsäurensonde, bestehend aus einer Sequenz von SEQ ID No. 630,
Primer-Sondenset 12, bestehend aus Set 12 gemäß Anspruch 1 und einer Nukleinsäurensonde, bestehend aus einer Sequenz von SEQ ID No. 641, und
Primer-Sondenset 13, bestehend aus Set 13 gemäß Anspruch 1 und einer Nukleinsäurensonde, bestehend aus einer Sequenz von SEQ ID No. 725.

4. Kit gemäß Anspruch 3, wobei die Nukleinsäuresonde auf einer Trägeroberfläche immobilisiert wird.

5. Kit gemäß Anspruch 4, wobei der Träger eine Elektrode ist.

6. Verfahren zum Nachweis von Genotypen des humanen Papilloma-Virus, umfassend:
einen Schritt des Erhaltens eines Amplifikationsprodukts durch LAMP-Amplifikation von Nukleinsäureketten in einer Probe unter Verwendung von Nukleinsäureprimern;
einen Schritt des Hybridisierens des Amplifikationsprodukts mit einer Nukleinsäuresonde; und
einen Schritt des Nachweises doppelsträngiger Ketten, die durch die Hybridisierung gebildet wurden, um humanes Papilloma-Virus nachzuweisen oder dessen Genotyp zu identifizieren;
wobei die Nukleinsäureprimer und die Nukleinsäuresonde ausgewählt werden aus den Primer-Sondensets 1-13, wie in Anspruch 3 definiert.

7. Verfahren gemäß Anspruch 6, wobei die Nukleinsäuresonde auf einer Trägeroberfläche immobilisiert wird.

8. Verfahren gemäß Anspruch 7, wobei die Trägeroberfläche aus einem Elektrodenmaterial hergestellt ist.

9. Verfahren gemäß Anspruch 8, wobei ein elektrochemisch aktives Nukleinsäureketten-erkennendes Mittel beim Schritt des Nachweisens doppelsträngiger Ketten verwendet wird.

10. Nachweis-Kit gemäß Anspruch 3, umfassend mindestens ein Nachweis-Set ausgewählt aus den Nachweis-Sets A, B, C und D,
wobei:
Nachweis-Set A mindestens ein Primer-Sondenset umfasst, ausgewählt aus der Gruppe bestehend aus:
Primer-Sondenset 1, 2 und 3 gemäß Anspruch 3;
Nachweis-Set B mindestens ein Primer-Sondenset umfasst, ausgewählt aus der Gruppe bestehend aus:
Primer-Sondenset 4, 5 und 6 gemäß Anspruch 3;
Nachweis-Set C mindestens ein Primer-Sondenset umfasst, ausgewählt aus der Gruppe bestehend aus:
Primer-Sondenset 7, 8, 9 und 10 gemäß Anspruch 3; und
Nachweis-Set D mindestens ein Primer-Sondenset umfasst, ausgewählt aus der Gruppe bestehend aus:
Primer-Sondenset 11, 12 und 13 gemäß Anspruch 3.

11. Nachweis-Kit zur Verwendung beim Nachweis von Genotypen des humanen Papilloma-Virus, umfassend mindestens eine Mischung von Primer-Sets gemäß Anspruch 2 sowie einen DNA-Chip, wobei der DNA-Chip Nukleinsäuresonden, bestehend aus einer Sequenz von SEQ ID Nos. 600, 623, 630, 641, 654, 673, 676, 699, 725, 750, 752, 771 und 783, immobilisiert auf einer Trägeroberfläche, umfaßt.

## Revendications

1. Ensemble d'amorces d'acide nucléique pour l'amplification LAMP destiné à être utilisé dans la détection de génotypes du papillomavirus humain, **caractérisé en ce que** l'ensemble est choisi dans le groupe consistant en :
Ensemble 1 consistant en cinq amorces d'acide nucléique consistant en une séquence de SEQ ID N°. 136, 132, 126, 128 et 138 respectivement, pour la détection du génotype de PVH 16 ;
Ensemble 2 consistant en cinq amorces d'acide nucléique consistant en une séquence de SEQ ID N°. 278, 276, 277, 281 et 280 respectivement, pour la détection du génotype de PVH 35 ;
Ensemble 3 consistant en cinq amorces d'acide nucléique consistant en une séquence de SEQ ID N°. 527, 524, 526, 523 et 529 respectivement, pour la détection du génotype de PVH 59 ;
Ensemble 4 consistant en cinq amorces d'acide nucléique consistant en une séquence de SEQ ID N°. 164, 167, 162, 156 et 170 respectivement, pour la détection du génotype de PVH 18 ;
Ensemble 5 consistant en cinq amorces d'acide nucléique consistant en une séquence de SEQ ID N°. 320, 327, 319, 324 et 328 respectivement, pour la détection du génotype de PVH 39 ;
Ensemble 6 consistant en cinq amorces d'acide nucléique consistant en une séquence de SEQ ID N°. 454, 457, 453, 458 et 458 respectivement, pour la détection du génotype de PVH 56 ;
Ensemble 7 consistant en cinq amorces d'acide nucléique consistant en une séquence de SEQ ID N°. 363, 359, 366, 361 et 370 respectivement, pour la détection du génotype de PVH 45 ;
Ensemble 8 consistant en cinq amorces d'acide nucléique consistant en une séquence de SEQ ID N°. 399, 391, 395, 388 et 402 respectivement, pour la détection du génotype de PVH 51 ;
Ensemble 9 consistant en cinq amorces d'acide nucléique consistant en une séquence de SEQ ID N°. 474, 476, 477, 475 et 480 respectivement, pour la détection du génotype de PVH 58;
Ensemble 10 consistant en cinq amorces d'acide nucléique consistant en une séquence de SEQ ID N°. 549, 547, 548, 546 et 572 respectivement, pour la détection du génotype de PVH 68 ;
Ensemble 11 consistant en cinq amorces d'acide nucléique consistant en une séquence de SEQ ID N°. 208, 203, 210, 205 et 214 respectivement, pour la détection du génotype de PVH 31 ;
Ensemble 12 consistant en cinq amorces d'acide nucléique consistant en une séquence de SEQ ID N°. 251, 244, 248, 242 et 255 respectivement, pour la détection du génotype de PVH 33 ; et
Ensemble 13 consistant en cinq amorces d'acide nucléique consistant en une séquence de SEQ ID N°. 422, 425, 420, 429 et 431 respectivement, pour la détection du génotype de PVH 52.

2. Mélange d'ensembles d'amorces, selon la revendication 1, destiné à être utilisé dans la multiamplification LAMP, **caractérisé en ce que** le mélange est choisi dans le groupe consistant en:
Mélange A comprenant Ensemble 1, 2 et 3 ;
Mélange B comprenant Ensemble 4, 5 et 6 ;
Mélange C comprenant Ensemble 7, 8, 9 et 10 ; et
Mélange D comprenant Ensemble 11, 12 et 13.

3. Kit de détection destiné à être utilisé dans la détection de génotypes du papillomavirus humain, où ledit kit comprend au moins l'un des Ensembles Amorce-Sonde choisis parmi:
Ensemble Amorce-Sonde 1 consistant en Ensemble 1 selon la revendication 1 et une sonde d'acide nucléique consistant en une séquence de SEQ ID No. 600,
Ensemble Amorce-Sonde 2 consistant en Ensemble 2 selon la revendication 1 et une sonde d'acide nucléique consistant en une séquence de SEQ ID No. 654,
Ensemble Amorce-Sonde 3 consistant en Ensemble 3 selon la revendication 1 et une sonde d'acide nucléique consistant en une séquence de SEQ ID No. 771,
Ensemble Amorce-Sonde 4 consistant en Ensemble 4 selon la revendication 1 et une sonde d'acide nucléique consistant en une séquence de SEQ ID No. 623,
Ensemble Amorce-Sonde 5 consistant en Ensemble 5 selon la revendication 1 et une sonde d'acide nucléique consistant en une séquence de SEQ ID No. 673,
Ensemble Amorce-Sonde 6 consistant en Ensemble 6 selon la revendication 1 et une sonde d'acide nucléique consistant en une séquence de SEQ ID No. 750,
Ensemble Amorce-Sonde 7 consistant en Ensemble 7 selon la revendication 1 et une sonde d'acide nucléique consistant en une séquence de SEQ ID No. 676,
Ensemble Amorce-Sonde 8 consistant en Ensemble 8 selon la revendication 1 et une sonde d'acide nucléique consistant en une séquence de SEQ ID No. 699,
Ensemble Amorce-Sonde 9 consistant en Ensemble 9 selon la revendication 1 et une sonde d'acide nucléique consistant en une séquence de SEQ ID No. 752,
Ensemble Amorce-Sonde 10 consistant en Ensemble 10 selon la revendication 1 et une sonde d'acide nucléique consistant en une séquence de SEQ ID No. 783,
Ensemble Amorce-Sonde 11 consistant en Ensemble 11 selon la revendication 1 et une sonde d'acide nucléique consistant en une séquence de SEQ ID No. 630,
Ensemble Amorce-Sonde 12 consistant en Ensemble 12 selon la revendication 1 et une sonde d'acide nucléique consistant en une séquence de SEQ ID No. 641, et
Ensemble Amorce-Sonde 13 consistant en Ensemble 13 selon la revendication 1 et une sonde d'acide nucléique consistant en une séquence de SEQ ID No. 725.

4. Kit selon la revendication 3, où la sonde d'acide nucléique est immobilisée sur une surface de support.

5. Kit selon la revendication 4, où la surface de support est une électrode.

6. Procédé de détection de génotypes du papillomavirus humain, comprenant :
une étape d'obtention d'un produit d'amplification par amplification LAMP d'une chaîne d'acide nucléique dans un échantillon au moyen d'amorces d'acide nucléique ;
une étape d'hybridation du produit d'amplification avec une sonde d'acide nucléique ; et
une étape de détection de chaînes double brin formées par l'hybridation pour détecter le papillomavirus humain ou identifier son génotype ;
où les amorces d'acide nucléique et la sonde d'acide nucléique sont choisies dans les Ensembles Amorce-Sonde 1 - 13, selon la revendication 3.

7. Procédé selon la revendication 6, où la sonde d'acide nucléique est immobilisée sur une surface de support.

8. Procédé selon la revendication 7, où la surface de support est faite d'une matière d'électrode.

9. Procédé selon la revendication 8, où un agent reconnaissant les chaînes d'acide nucléique électrochimiquement actif est utilisé dans l'étape de détection de chaînes double brin.

10. Kit de détection selon la revendication 3, comprenant au moins un Ensemble de Détection choisi parmi les ensembles de Détection A,B,C et D
où :
l'Ensemble de Détection A comprend au moins un Ensemble Amorce-Sonde choisi dans le groupe consistant en :
Ensembles Amorce-Sonde 1, 2 et 3, selon la revendication 3 ;
l'Ensemble de Détection B comprend au moins un Ensemble Amorce-Sonde choisi dans le groupe consistant en :
Ensembles Amorce-Sonde 4, 5 et 6, selon la revendication 3 ;
l'Ensemble de Détection C comprend au moins un Ensemble Amorce-Sonde choisi dans le groupe consistant en :
Ensembles Amorce-Sonde 7, 8, 9 et 10, selon la revendication 3 ; et l'Ensemble de Détection D comprend au moins un Ensemble Amorce-Sonde choisi dans le groupe consistant en :
Ensembles Amorce-Sonde 11, 12 et 13, selon la revendication 3.

11. Kit de détection destiné à être utilisé dans la détection de génotypes du papillomavirus humain, comprenant au moins un mélange d'ensembles d'amorces, selon la revendication 2, et une puce à ADN, où la puce à ADN comprend des sondes d'acide nucléique consistant en une séquence de SEQ ID N°. 600, 623, 630, 641, 654, 673, 676, 699, 725, 750, 752, 771 et 783 respectivement, immobilisées sur une surface de support.


### SEQUENCE LISTING

<110> KABUSHIKI KAISHA TOSHIBA
<120> Method of detecting human papilloma virus by using nucleic acid amplification method and nucleic acid chain-immobilized carrier
<130> PN506005EPA
<140> Divisional Application derived from EP 06834753.3 (PCT/JP2006/325010)
<141> 2006-12-08
<150> JP 2005-354826
   <151> 2005-12-08
<150>. JP 2006-187871
   <151> 2006-07-07
<150> PCT/JP2006/323261
   <151> 2006-11-15
<160> 814
<170> Patent In version 3.1
   <210> 1
   <211> 25
   <212> DNA
   <213> Human papillomavirus
<400> 1
   gaatatgatt tacagtttat ttttc 25
<210> 2
   <211> 23
   <212> DNA
   <213> Human papillomavirus
<400> 2
   tttgttactg ttgttgatac tac 23
<210> 3
   <211> 20
   <212> DNA
   <213> Human papillomavirus
<400> 3
   gcacagggcc acaataatgg 20
<210> 4
   <211> 30
   <212> DNA
   <213> Human papillomavirus
<400> 4
   gtcattatgtgctgccatatctacttcaga 30
<210> 5
   <211> 20
   <212> DNA
   <213> Human papillomavirus
<400> 5
   gatcagtttcctttaggacg 19
<210> 6
   <211> 20
   <212> DNA
   <213> Human papillomavirus
<400> 6
   aaatttttactacaagcagg 20
<210> 7
   <211> 30
   <212> DNA
   <213> Human papillomavirus
<400> 7
   tctgaagtag atatggcagc acataatgac 30
<210> 8
   <211> 30
   <212> DNA
   <213> Human papillomavirus
<400> 8
   tgcccaggta caggagactg tgtagaagca 30
<210> 9
   <211> 30
   <212> DNA
   <213> Human papillomavirus
<400> 9
   agtggatgca gatgctgcag ataatgtact 30
<210> 10
   <211> 30
   <212> DNA
   <213> Human papillomavirus
<400> 10
   gtatcactgt ttgcaattgc agcacaaaca 30
<210> 11
   <211> 20
   <212> DNA
   <213> E.coli
<220>
<223> probe
<400> 11
   ctggacgaag actgacgctc 20
<210> 12
   <211> 48
   <212> DNA
   <213> Human papillomavirus
<400> 12
   gaaaaataaa ctgtaaatca tattctttgt tactgtggta gatactac 48
<210> 13
   <211> 20
   <212> DNA
   <213> Human papillomavirus
<400> 13
   gcacagggcc acaataatgg 20
<210> 14
   <211> 44
   <212> DNA
   <213> Human papillomavirus
<400> 14
   tttttgggaa gtaaatttaa aggacgtcct aaaggaaact gatc 44

<210> 15
   <211> 20
   <212> DNA
   <213> Human papillomavirus
<400> 15
   cctgcttgta gtaaaaattt 20
<210> 16
   <211> 20
   <212> DNA
   <213> Human papillomavirus
<400> 16
   tgatttacag tttatttttc 20
<210> 17
   <211> 25
   <212> DNA
   <213> Human papillomavirus
<400> 17
   gaatatgatt tacagtttat ttttc 25
<210> 18
   <211> 28
   <212> DNA
   <213> Human papillomavirus
<400> 18
   gaagaatatg atttacagtt tatttttc 28
<210> 19
   <211> 28
   <212> DNA
   <213> Human papillomavirus
<400> 19
   gaggaatatg atttacagtt tatttttc 28
<210> 20
   <211> 25
   <212> DNA
   <213> Human papillomavirus
<400> 20
   gagtatgatt tacaatttat ttttc 25
<210> 21
   <211> 25
   <212> DNA
   <213> Human papillomavirus
<400> 21
   gagtttgatt tacagtttat ttttc 25
<210> 22
   <211> 25
   <212> DNA
   <213> Human papillomavirus
<400> 22
   gaatttgatt tacaatttat ttttc 25
<210> 23
   <211> 25
   <212> DNA
   <213> Human papillomavirus
<400> 23
   gaatatgatt tacagtttat ttttc 25
<210> 24
   <211> 25
   <212> DNA
   <213> Human papillomavirus
<400> 24
   gaatatgatt tgcagtttat ttttc 25
<210> 25
   <211> 25
   <212> DNA
   <213> Human papillomavirus
<400> 25
   gaatatgaat tacagtttgt gtttc 25
<210> 26
   <211> 25
   <212> DNA
   <213> Human papillomavirus
<400> 26
   gaatttgatt tacaatttat atttc 25
<210> 27
   <211> 25
   <212> DNA
   <213> Human papillomavirus
<400> 27
   gaatatgata tacagtttat atttc 25
<210> 28
   <211> 25
   <212> DNA
   <213> Human papillomavirus
<400> 28
   gaatatgatc tacagtttgt ttttc 25
<210> 29
   <211> 25
   <212> DNA
   <213> Human papillomavirus
<400> 29
   gagtatgacc tgcagtttgt gtttc 25
<210> 30
   <211> 25
   <212> DNA
   <213> Human papillomavirus
<400> 30
   gaatatgatt tacagtttat ttttc 25
<210> 31
   <211> 25
   <212> DNA
   <213> Human papillomavirus
<400> 31
   gagtatgatt tacaatttat atttc 25

<210> 32
   <211> 25
   <212> DNA
   <213> Human papillomavirus
<400> 32
   gagtttgatt tgcagtttat ttttc 25
<210> 33
   <211> 25
   <212> DNA
   <213> Human papillomavirus
<400> 33
   gaatatgatg tgcaatttat atttc 25
<210> 34
   <211> 25
   <212> DNA
   <213> Human papillomavirus
<400> 34
   gaatatgatt tacagtttat ttttc 25
<210> 35
   <211> 25
   <212> DNA
   <213> Human papillomavirus
<400> 35
   gagtatgaat tgcaatttat ttttc 25
<210> 36
   <211> 25
   <212> DNA
   <213> Human papillomavirus
<400> 36
   gaatttgatt tacaatttat ttttc 25
<210> 37
   <211> 25
   <212> DNA
   <213> Human papillomavirus
<400> 37
   gaatatgaat tacaatttgt gtttc 25
<210> 38
   <211> 25
   <212> DNA
   <213> Human papillomavirus
<400> 38
   gaatatgaat tacaatttgt ttttc 25
<210> 39
   <211> 25
   <212> DNA
   <213> Human papillomavirus
<400> 39
   gaatatgact tacagtttgt ttttc 25
<210> 40
   <211> 25
   <212> DNA
   <213> Human papillomavirus
<400> 40
   gagtttgatt tgcaatttat ttttc 25
<210> 41
   <211> 25
   <212> DNA
   <213> Human papillomavirus
<400> 41
   gaatatgaac tacagtttgt gtttc 25
<210> 42
   <211> 25
   <212> DNA
   <213> Human papillomavirus
<400> 42
   gaatatgatt tgcaatttat atttc 25
<210> 43
   <211> 25
   <212> DNA
   <213> Human papillomavirus
<400> 43
   gaaahataaa ytgyaadtca taytc 25
<210> 44
   <211> 20
   <212> DNA
   <213> Human papillomavirus
<400> 44
   tttgttactg tggtagatac 20
<210> 45
   <211> 23
   <212> DNA
   <213> Human papillomavirus
<400> 45
   tttgttactg tggtagatac tac 23
<210> 46
   <211> 23
   <212> DNA
   <213> Human papillomavirus
<400> 46
   tttgttactg tggtagatac cac 23
<210> 47
   <211> 23
   <212> DNA
   <213> Human papillomavirus
<400> 47
   tttgttactg tggtagatac cac 23
<210> 48
   <211> 23
   <212> DNA
   <213> Human papillomavirus
<400> 48
   tttgttactg tagttgatac tac 23

<210> 49
   <211> 23
   <212> DNA
   <213> Human papillomavirus
<400> 49
   tttgttactg ttgttgatac tac 23
<210> 50
   <211> 23
   <212> DNA
   <213> Human papillomavirus
<400> 50
   tttgttactg tggtagatac cac 23
<210> 51
   <211> 23
   <212> DNA
   <213> Human papillomavirus
<400> 51
   tttgttactg ttgtggacac cac 23
<210> 52
   <211> 23
   <212> DNA
   <213> Human papillomavirus
<400> 52
   tttgttactg tggtagatac cac 23
<210> 53
   <211> 23
   <212> DNA
   <213> Human papillomavirus
<400> 53
   tttctaactg ttgtggatac tac 23
<210> 54
   <211> 23
   <212> DNA
   <213> Human papillomavirus
<400> 54
   tttgttactg tggtagatac cac 23
<210> 55
   <211> 23
   <212> DNA
   <213> Human papillomavirus
<400> 55
   tttttaactg ttgtagatac tac 23
<210> 56
   <211> 23
   <212> DNA
   <213> Human papillomavirus
<400> 56
   tttgttactg tagttgatac aac 23
<210> 57
   <211> 23
   <212> DNA
   <213> Human papillomavirus
<400> 57
   tttcttactg ttgtggacac tac 23
<210> 58
   <211> 23
   <212> DNA
   <213> Human papillomavirus
<400> 58
   tttgttacag ttgtagacac cac 23
<210> 59
   <211> 23
   <212> DNA
   <213> Human papillomavirus
<400> 59
   tttttaactg tggttgatac tac 23
<210> 60
   <211> 23
   <212> DNA
   <213> Human papillomavirus
<400> 60
   tttgttactg tagtggacac tac 23
<210> 61
   <211> 23
   <212> DNA
   <213> Human papillomavirus
<400> 61
   tttattacct gtgttgatac tac 23
<210> 62
   <211> 23
   <212> DNA
   <213> Human papillomavirus
<400> 62
   tttgtcacag ttgtggatac cac 23
<210> 63
   <211> 23
   <212> DNA
   <213> Human papillomavirus
<400> 63
   tttgtaactg ttgtggatac cac 23
<210> 64
   <211> 23
   <212> DNA
   <213> Human papillomavirus
<400> 64
   tttgttactg tagtagatac tac 23
<210> 65
   <211> 23
   <212> DNA
   <213> Human papillomavirus

<400> 65
   tttgttaccg tggttgatac cac 23
<210> 66
   <211> 23
   <212> DNA
   <213> Human papillomavirus
<400> 66
   tttgtaaccg ttgtggatac cac 23
<210> 67
   <211> 23
   <212> DNA
   <213> Human papillomavirus
<400> 67
   tttgttactg ttgtggatac tac 23
<210> 68
   <211> 23
   <212> DNA
   <213> Human papillomavirus
<400> 68
   tttcttactg ttgtggatac cac 23
<210> 69
   <211> 23
   <212> DNA
   <213> Human papillomavirus
<400> 69
   tttkttachg tkgtdgatac yac 23
<210> 70
   <211> 20
   <212> DNA
   <213> Human papillomavirus
<400> 70
   gcacagggcc acaataatgg 20
<210> 71
   <211> 20
   <212> DNA
   <213> Human papillomavirus
<400> 71
   gcacagggac ataacaatgg 20
<210> 72
   <211> 20
   <212> DNA
   <213> Human papillomavirus
<400> 72
   gcgcagggcc acaataatgg 20
<210> 73
   <211> 20
   <212> DNA
   <213> Human papillomavirus
<400> 73
   gcacagggac ataataatgg 20
<210> 74
   <211> 20
   <212> DNA
   <213> Human papillomavirus
<400> 74
   gcccagggcc acaacaatgg 20
<210> 75
   <211> 20
   <212> DNA
   <213> Human papillomavirus
<400> 75
   gctcagggtt taaacaatgg 20
<210> 76
   <211> 20
   <212> DNA
   <213> Human papillomavirus
<400> 76
   gctcagggtt taaacaatgg 20
<210> 77
   <211> 20
   <212> DNA
   <213> Human papillomavirus
<400> 77
   gcccagggac ataacaatgg 20
<210> 78
   <211> 20
   <212> DNA
   <213> Human papillomavirus
<400> 78
   gctcagggac ataacaatgg 20
<210> 79
   <211> 20
   <212> DNA
   <213> Human papillomavirus
<400> 79
   gcccagggac acaataatgg 20
<210> 80
   <211> 20
   <212> DNA
   <213> Human papillomavirus
<400> 80
   gcacagggtc ataacaatgg 20
<210> 81
   <211> 20
   <212> DNA
   <213> Human papillomavirus
<400> 81
   gcacagggtc ataataatgg 20
<210> 82
   <211> 20
   <212> DNA
   <213> Human papillomavirus
<400> 82
   gcacagggac acaataatgg 20
<210> 83
   <211> 20
   <212> DNA
   <213> Human papillomavirus
<400> 83
   gctcagggac acaataatgg 20
<210> 84
   <211> 20
   <212> DNA
   <213> Human papillomavirus
<400> 84
   gcacaaggtc ataataatgg 20
<210> 85
   <211> 20
   <212> DNA
   <213> Human papillomavirus
<400> 85
   gcccagggac aaaacaatgg 20
<210> 86
   <211> 20
   <212> DNA
   <213> Human papillomavirus
<400> 86
   gcacaaggcc ataataatgg 20
<210> 87
   <211> 20
   <212> DNA
   <213> Human papillomavirus
<400> 87
   gcccagggcc acaacaatgg 20

<210> 88
   <211> 20
   <212> DNA
   <213> Human papillomavirus
<400> 88
   gcccagggcc ataacaatgg 20
<210> 89
   <211> 20
   <212> DNA
   <213> Human papillomavirus
<400> 89
   gcacaaggac acaataatgg 20
<210> 90
   <211> 14
   <212> DNA
   <213> Human papillomavirus
<400> 90
   ggacataata atgg 14
<210> 91
   <211> 20
   <212> DNA
   <213> Human papillomavirus
<400> 91
   gcgcagggcc acaataatgg 20
<210> 92
   <211> 20
   <212> DNA
   <213> Human papillomavirus
<400> 92
   gcccagggcc ataacaatgg 20
<210> 93
   <211> 20
   <212> DNA
   <2₁3> Human papillomavirus
<400> 93
   gcgcagggtc acaataatgg 20
<210> 94
   <211> 20
   <212> DNA
   <213> Human papillomavirus
<400> 94
   gcgcagggcc acaataatgg 20
<210> 95
   <211> 20
   <212> DNA
   <213> Human papillomavirus
<400> 95
   gcccagggac ataataatgg 20
<210> 96
   <211> 20
   <212> DNA
   <213> Human papillomavirus
<400> 96
   gcccagggtc aaaacaatgg 20
<210> 97
   <211> 20
   <212> DNA
   <213> Human papillomavirus
<400> 97
   gcgcagggcc acaataatgg 20
<210> 98
   <211> 20
   <212> DNA
   <213> Human papillomavirus
<400> 98
   gcccaaggcc ataataatgg 20
<210> 99
   <211> 20
   <212> DNA
   <213> Human papillomavirus
<400> 99
   gcacaaggtc ataacaatgg 20
<210> 100
   <211> 20
   <212> DNA
   <213> Human papillomavirus
<400> 100
   gctcagggtt taaacaatgg 20
<210> 101
   <211> 20
   <212> DNA
   <213> Human papillomavirus
<400> 101
   gcccagggcc acaacaatgg 20
<210> 102
   <211> 20
   <212> DNA
   <213> Human papillomavirus
<400> 102
   gcacagggtc ataataatgg 20
<210> 103
   <211> 20
   <212> DNA
   <213> Human papillomavirus
<400> 103
   gcacagggac ataacaatgg 20
<210> 104
   <211> 20
   <212> DNA
   <213> Human papillomavirus
<400> 104
   gcacagggtc ataataatgg 20
<210> 105
   <211> 20
   <212> DNA
   <213> Human papillomavirus
<400> 105
   gcccagggac ataacaatgg 20
<210> 106
   <211> 20
   <212> DNA
   <213> Human papillomavirus
<400> 106
   gcacagggac acaacaatgg 20
<210> 107
   <211> 20
   <212> DNA
   <213> Human papillomavirus
<400> 107
   gcacagggac ataacaatgg 20
<210> 108
   <211> 20
   <212> DNA
   <213> Human papillomavirus
<400> 108
   gcccagggaa ctaataatgg 20
<210> 109
   <211> 20
   <212> DNA
   <213> Human papillomavirus
<400> 109
   gcccagggtc ataataatgg 20
<210> 110
   <211> 20
   <212> DNA
   <213> Human papillomavirus

<400> 110
   gcacagggtc ataataatgg 20
<210> 111
   <211> 20
   <212> DNA
   <213> Human papillomavirus
<400> 111
   gcgcaaggcc acaataatgg 20
<210> 112
   <211> 20
   <212> DNA
   <213> Human papillomavirus
<400> 112
   gcacagggac ataataatgg 20
<210> 113
   <211> 20
   <212> DNA
   <213> Human papillomavirus
<400> 113
   gcccagggac ataataatgg 20
<210> 114
   <211> 20
   <212> DNA
   <213> Human papillomavirus
<400> 114
   gcgcggggtc ataacaatgg 20
<210> 115
   <211> 20
   <212> DNA
   <213> Human papillomavirus
<400> 115
   gcmcagggwc ataayaatgg 20
<210> 116
   <211> 21
   <212> DNA
   <213> Human papillomavirus
<400> 116
   gcgtgtagta tcaacaacag t 21
<210> 117
   <211> 43
   <212> DNA
   <213> Human papillomavirus
<400> 117
   ccttattggt tacaacgagc acaacaaata gttggttacc cca 43
<210> 118
   <211> 21
   <212> DNA
   <213> Human papillomavirus
<400> 118
   ggtgaaaatg taccagacga t 21
<210> 119
   <211> 44
   <212> DNA
   <213> Human papillomavirus
<400> 119
   agaggtaacc atagaaccac tagggtctac tgcaaattta gcca 44
<210> 120
   <211> 19
   <212> DNA
   <213> Human papillomavirus
<400> 120
   agtagatatg gcagcacat 19
<210> 121
   <211> 41
   <212> DNA
   <213> Human papillomavirus
<400> 121
   acagggccac aataatggca tgacatattt gtactgcgtg t 41
<210> 122
   <211> 20
   <212> DNA
   <213> Human papillomavirus
<400> 122
   cattaaaggc tctgggtcta 20
<210> 123
   <211> 44
   <212> DNA
   <213> Human papillomavirus
<400> 123
   gcatcagagg taaccataga accactgcaa atttagccag ttca 44
<210> 124
   <211> 22
   <212> DNA
   <213> Human papillomavirus
<400> 124
   ttccagtcct ccaaaatagt gg 22
<210> 125
   <211> 48
   <212> DNA
   <213> Human papillomavirus
<400> 125
   atactacacg cagtacaaat atgtctgtca taacgtctgc agttaagg 48
<210> 126
   <211> 25
   <212> DNA
   <213> Human papillomavirus
<400> 126
   caaattattt tcctacacct agtgg 25
<210> 127
   <211> 44
   <212> DNA
   <213> Human papillomavirus
<400> 127
   gttggttacc ccaacaaatg cctctatggt tacctctgat gccc 44
<210> 128
   <211> 22
   <212> DNA
   <213> Human papillomavirus
<400> 128
   gtcataacgt ctgcagttaa gg 22
<210> 129
   <211> 24
   <212> DNA
   <213> Human papillomavirus
<400> 129
   gtcgtaggta ctccttaaag ttag 24
<210> 130
   <211> 46
   <212> DNA
   <213> Human papillomavirus
<400> 130
   atactacacg cagtacaaat atgtctcccc atgtcgtagg tactcc 46
<210> 131
   <211> 43
   <212> DNA
   <213> Human papillomavirus
<400> 131
   ctacacgcag tacaaatatg tctccccatg tcgtaggtac tcc 43
<210> 132
   <211> 40
   <212> DNA
   <213> Human papillomavirus
<400> 132
   cacgcagtac aaatatgtca ccccatgtcg taggtactcc 40

<210> 133
   <211> 46
   <212> DNA
   <213> Human papillomavirus
<400> 133
   ctacacgcag tacaaatatg tcgtagtttc tgaagtagat atggca 46
<210> 134
   <211> 46
   <212> DNA
   <213> Human papillomavirus
<400> 134
   ctacacgcag tacaaatatg tctatgtagt ttctgaagta gatatg 46
<210> 135
   <211> 41
   <212> DNA
   <213> Human papillomavirus
<400> 135
   gtggccctgt gctcgttgtt ctatggttac ctctgatgcc c 41
<210> 136
   <211> 39
   <212> DNA
   <213> Human papillomavirus
<400> 136
   gtggccctgt gctcgttgtc tatggttacc tctgatgcc 39
<210> 137
   <211> 25
   <212> DNA
   <213> Human papillomavirus
<400> 137
   gtgctgccat atctacttca gaaac 25
<210> 138
   <211> 27
   <212> DNA
   <213> Human papillomavirus
<400> 138
   gctgccatat ctacttcaga aactaca 27
<210> 139
   <211> 25
   <212> DNA
   <213> Human papillomavirus
<400> 139
   aaccaataag gtttattgaa tattt 25
<210> 140
   <211> 25
   <212> DNA
   <213> Human papillomavirus
<400> 140
   ccaataaggt ttattgaata tttgg 25
<210> 141
   <211> 45
   <212> DNA
   <213> Human papillomavirus
<400> 141
   ttatgcagca aatgcaggtg tggcccctat aggtggtttg caacc 45
<210> 142
   <211> 20
   <212> DNA
   <213> Human papillomavirus
<400> 142
   ccctataggt ggtttgcaac 20
<210> 143
   <211> 20
   <212> DNA
   <213> Human papillomavirus
<400> 143
   gtacatgggg atcctttgcc 20

<210> 144
   <211> 52
   <212> DNA
   <213> Human papillomavirus
<400> 144
   acagaaaatg ctagtgctta tgcagccaat tgtgtttgtt tgtaatccat ag 52
<210> 145
   <211> 44
   <212> DNA
   <213> Human papillomavirus
<400> 145
   cacagaaaat gctagtgctt attgtgtttg tttgtaatcc atag 44
<210> 146
   <211> 45
   <212> DNA
   <213> Human papillomavirus
<400> 146
   ataaaggatg gccactaatg cccgtgtagg tgttgaggta ggtcg 45
<210> 147
   <211> 25
   <212> DNA
   <213> Human papillomavirus
<400> 147
   cctgacacct cattttataa tccag 25
<210> 148
   <211> 20
   <212> DNA
   <213> Human papillomavirus
<400> 148
   atccagatac acagcggctg 20
<210> 149
   <211> 38
   <212> DNA
   <213> Human papillomavirus
<400> 149
   ccacacctaa tggctgacca cacacagcgg ctggtttg 38
<210> 150
   <211> 40
   <212> DNA
   <213> Human papillomavirus
<400> 150
   gccactaatg cccacaccta atgacacagc ggctggtttg 40
<210> 151
   <211> 19
   <212> DNA
   <213> Human papillomavirus
<400> 151
   ccaacagtac cagccctat 19
<210> 152
   <211> 41
   <212> DNA
   <213> Human papillomavirus
<400> 152
   tggtgtcaga accatatggc gacaaacatt tgttcccttc g 41
<210> 153
   <211> 22
   <212> DNA
   <213> Human papillomavirus
<400> 153
   cacagttatt caggatggtg at 22
<210> 154
   <211> 43
   <212> DNA
   <213> Human papillomavirus
<400> 154
   ggaacttcac ttttgttagc ctgttggttc atactggctt tgg 43
<210> 155
   <211> 20
   <212> DNA
   <213> Human papillomavirus
<400> 155
   ccctataggt ggtttgcaac 20
<210> 156
   <211> 52
   <212> DNA
   <213> Human papillomavirus
<400> 156
   acagaaaatg ctagtgctta tgcagccaat tgtgtttgtt tgtaatccat ag 52
<210> 157
   <211> 25
   <212> DNA
   <213> Human papillomavirus
<400> 157
   cctgacacct cattttataa tccag 25
<210> 158
   <211> 38
   <212> DNA
   <213> Human papillomavirus
<400> 158
   ccacacctaa tggctgacca cacacagcgg ctggtttg 38
<210> 159
   <211> 40
   <212> DNA
   <213> Human papillomavirus
<400> 159
   gccactaatg cccacaccta atgacacagc ggctggtttg 40
<210> 160
   <211> 25
   <212> DNA
   <213> Human papillomavirus
<400> 160
   aagttccaat cctctaaaat actgc 25
<210> 161
   <211> 48
   <212> DNA
   <213> Human papillomavirus
<400> 161
   accactcgca gtaccaattt aactgaatat aggacataac atctgcag 48
<210> 162
   <211> 21
   <212> DNA
   <213> Human papillomavirus
<400> 162
   tgtattctcc ctctccaagt g 21
<210> 163
   <211> 47
   <212> DNA
   <213> Human papillomavirus
<400> 163
   taattgatta tgccagcaaa caccctctat tgttacctct gactccc 47
<210> 164
   <211> 43
   <212> DNA
   <213> Human papillomavirus
<400> 164
   gccagcaaac accattgtta ctctattgtt acctctgact ccc 43
<210> 165
   <211> 45
   <212> DNA
   <213> Human papillomavirus
<400> 165
   gccagcaaac accattgtta tgctctattg ttacctctga ctccc 45
<210> 166
   <211> 24
   <212> DNA
   <213> Human papillomavirus

<400> 166
   gaatatagga cataacatct gcag 24
<210> 167
   <211> 46
   <212> DNA
   <213> Human papillomavirus
<400> 167
   accactcgca gtaccaattt aaccctcaac atgtctgcta tactgc 46
<210> 168
   <211> 45
   <212> DNA
   <213> Human papillomavirus
<400> 168
   ccactcgcag taccaattta accctcaaca tgtctgctat actgc 45
<210> 169
   <211> 43
   <212> DNA
   <213> Human papillomavirus
<400> 169
   ccactcgcag taccaattta acctcaacat gtctgctata ctg 43
<210> 170
   <211> 20
   <212> DNA
   <213> Human papillomavirus
<400> 170
   accctgtgcc ttatgtaacc 20
<210> 171
   <211> 48
   <212> DNA
   <213> Human papillomavirus
<400> 171
   gatgacactg aaagttccca tgcgcccaaa atacataact gtgtctgc 48
<210> 172
   <211> 47
   <212> DNA
   <213> Human papillomavirus
<400> 172
   gatgacactg aaagttccca tgccccaaaa tacataactg tgtctgc 47
<210> 173
   <211> 19
   <212> DNA
   <213> Human papillomavirus
<400> 173
   agtgttcccc aatagcagg 19
<210> 174
   <211> 20
   <212> DNA
   <213> Human papillomavirus
<400> 174
   cagtgttccc caatagcagg 20
<210> 175
   <211> 49
   <212> DNA
   <213> Human papillomavirus
<400> 175
   gacactgaaa gttcccatgc cgctgtgtct gcttataatc tacagacac 49
<210> 176
   <211> 44
   <212> DNA
   <213> Human papillomavirus
<400> 176
   taaaatggat gcccactaag gcctgctgga gtggaaattg gccg 44
<210> 177
   <211> 21
   <212> DNA
   <213> Human papillomavirus
<400> 177
   cctgaaacac aacgtttagt g 21
<210> 178
   <211> 20
   <212> DNA
   <213> Human papillomavirus
<400> 178
   cacaacgttt agtgtgggcc 20
<210> 179
   <211> 27
   <212> DNA
   <213> Human papillomavirus
<400> 179
   acctgatact agtatttata atcctga 27
<210> 180
   <211> 38
   <212> DNA
   <213> Human papillomavirus
<400> 180
   gatgcccact aaggccaaca cgcctgtgct ggagtgga 38
<210> 181
   <211> 43
   <212> DNA
   <213> Human papillomavirus
<400> 181
   cactaaggcc aacacctaaa ggcaacacaa cgtttagtgt ggg 43
<210> 182
   <211> 19
   <212> DNA
   <213> Human papillomavirus
<400> 182
   agtgttcccc aatagcagg 19
<210> 183
   <211> 49
   <212> DNA
   <213> Human papillomavirus
<400> 183
   gacactgaaa gttcccatgc cgctgtgtct gcttataatc tacagacac 49
<210> 184
   <211> 21
   <212> DNA
   <213> Human papillomavirus
<400> 184
   cctgaaacac aacgtttagt g 21
<210> 185
   <211> 27
   <212> DNA
   <213> Human papillomavirus
<400> 185
   acctgatact agtatttata atcctga 27
<210> 186
   <211> 38
   <212> DNA
   <213> Human papillomavirus
<400> 186
   gatgcccact aaggccaaca cgcctgtgct ggagtgga 38
<210> 187
   <211> 43
   <212> DNA
   <213> Human papillomavirus
<400> 187
   cactaaggcc aacacctaaa ggcaacacaa cgtttagtgt ggg 43
<210> 188
   <211> 25
   <212> DNA
   <213> Human papillomavirus
<400> 188
   ccaatcttcc aaaatagcag gattc 25

<210> 189
   <211> 51
   <212> DNA
   <213> Human papillomavirus
<400> 189
   accacacgta gtaccaatat gtcctgtgaa tatatgtcat tatgtctgca g 51
<210> 190
   <211> 21
   <212> DNA
   <213> Human papillomavirus
<400> 190
   catactttcc tacacctagc g 21
<210> 191
   <211> 46
   <212> DNA
   <213> Human papillomavirus
<400> 191
   aactgattgc cccaacaaat accctccatg gttacttcag atgcac 46
<210> 192
   <211> 45
   <212> DNA
   <213> Human papillomavirus
<400> 192
   ccattattgt gtccctgagc acctccatgg ttacttcaga tgcac 45
<210> 193
   <211> 41
   <212> DNA
   <213> Human papillomavirus
<400> 193
   ctgattgccc caacaaatac ctccatggtt acttcagatg c 41
<210> 194
   <211> 41
   <212> DNA
   <213> Human papillomavirus
<400> 194
   ctgattgccc caacaaatac ccatggttac ttcagatgca c 41
<210> 195
   <211> 26
   <212> DNA
   <213> Human papillomavirus
<400> 195
   gtgaatatat gtcattatgt ctgcag 26
<210> 196
   <211> 47
   <212> DNA
   <213> Human papillomavirus
<400> 196
   accacacgta gtaccaatat gtcttcctca ccatgtctta aatactc 47
<210> 197
   <211> 48
   <212> DNA
   <213> Human papillomavirus
<400> 197
   accacacgta gtaccaatat gtcattcctc accatgtctt aaatactc 48
<210> 198
   <211> 48
   <212> DNA
   <213> Human papillomavirus
<400> 198
   cacacgtagt accaatatgt ctgattcctc accatgtctt aaatactc 48
<210> 199
   <211> 44
   <212> DNA
   <213> Human papillomavirus
<400> 199
   ccacacgtag taccaatatg tccctcacca tgtcttaaat actc 44
<210> 200
   <211> 43
   <212> DNA
   <213> Human papillomavirus
<400> 200
   ccacacgtag taccaatatg tcctcaccat gtcttaaata ctc 43
<210> 201
   <211> 20
   <212> DNA
   <213> Human papillomavirus
<400> 201
   cacgttgcat ccaatatggt 20
<210> 202
   <211> 19
   <212> DNA
   <213> Human papillomavirus
<400> 202
   cacgttgcat ccaatatgg 19
<210> 203
   <211> 51
   <212> DNA
   <213> Human papillomavirus
<400> 203
   cactgaaaac tctaatagat atgccggtgc aaccaagtaa acacagttgt g 51
<210> 204
   <211> 19
   <212> DNA
   <213> Human papillomavirus
<400> 204
   ccaataggtg gtttgcaac 19
<210> 205
   <211> 20
   <212> DNA
   <213> Human papillomavirus
<400> 205
   cctttacccc aatgctctcc 20
<210> 206
   <211> 52
   <212> DNA
   <213> Human papillomavirus
<400> 206
   cactgaaaac tctaatagat atgccggagt tgtgtttgtt tataatccat tg 52
<210> 207
   <211> 45
   <212> DNA
   <213> Human papillomavirus
<400> 207
   ctgaaaactc taatagatat gcctgtgttt gtttataatc cattg 45
<210> 208
   <211> 46
   <212> DNA
   <213> Human papillomavirus
<400> 208
   ggatgaccac taatacctac accctgtgtt ggtttagagg taggtc 46
<210> 209
   <211> 26
   <212> DNA
   <213> Human papillomavirus
<400> 209
   tcctgataca tctttttata atcctg 26
<210> 210
   <211> 21
   <212> DNA
   <213> Human papillomavirus
<400> 210
   aactcaacgc ttagtttggg c 21

<210> 211
   <211> 49
   <212> DNA
   <213> Human papillomavirus
<400> 211
   ccactaatac ctacacctaa tggctgcaaa ctcaacgctt agtttgggc 49
<210> 212
   <211> 41
   <212> DNA
   <213> Human papillomavirus
<400> 212
   cactaatacc tacacctaat ggcctcaacg cttagtttgg g 41
<210> 213
   <211> 23
   <212> DNA
   <213> Human papillomavirus
<400> 213
   gacctacctc taaaccaaca cag 23
<210> 214
   <211> 16
   <212> DNA
   <213> Human papillomavirus
<400> 214
   taatggctgc ccgcga 16
<210> 215
   <211> 19
   <212> DNA
   <213> Human papillomavirus
<400> 215
   ccaataggtg gtttgcaac 19
<210> 216
   <211> 52
   <212> DNA
   <213> Human papillomavirus
<400> 216
   cactgaaaac tctaatagat atgccggagt tgtgtttgtt tataatccat tg 52
<210> 217
   <211> 26
   <212> DNA
   <213> Human papillomavirus
<400> 217
   tcctgataca tctttttata atcctg 26
<210> 218
   <211> 49
   <212> DNA
   <213> Human papillomavirus
<400> 218
   ccactaatac ctacacctaa tggctgcaaa ctcaacgctt agtttgggc 49
<210> 219
   <211> 22
   <212> DNA
   <213> Human papillomavirus
<400> 219
   ggaggtgtta aaccaaattg cc 22
<210> 220
   <211> 48
   <212> DNA
   <213> Human papillomavirus
<400> 220
   accactcgca gtactaatat gactatgtca taacttctgc agttaagg 48
<210> 221
   <211> 47
   <212> DNA
   <213> Human papillomavirus
<400> 221
   accactcgca gtactaatat gaccttctgc agttaaggta actttgc 47
<210> 222
   <211> 22
   <212> DNA
   <213> Human papillomavirus
<400> 222
   gctttttttc ccactcctag tg 22
<210> 223
   <211> 46
   <212> DNA
   <213> Human papillomavirus
<400> 223
   cctgattgcc ccaacaaata ccgatcaatg gttacttccg aatctc 46
<210> 224
   <211> 44
   <212> DNA
   <213> Human papillomavirus
<400> 224
   cctgattgcc ccaacaaata ccagccatat tggctacaac gtgc 44
<210> 225
   <211> 24
   <212> DNA
   <213> Human papillomavirus
<400> 225
   atgtcataac ttctgcagtt aagg 24
<210> 226
   <211> 47
   <212> DNA
   <213> Human papillomavirus
<400> 226
   accactcgca gtactaatat gacttcttca acatgtctta tatattc 47
<210> 227
   <211> 47
   <212> DNA
   <213> Human papillomavirus
<400> 227
   ataccactcg cagtactaat atgttcttca acatgtctta tatattc 47
<210> 228
   <211> 49
   <212> DNA
   <213> Human papillomavirus
<400> 228
   accactcgca gtactaatat gacattcttc aacatgtctt atatattct 49
<210> 229
   <211> 49
   <212> DNA
   <213> Human papillomavirus
<400> 229
   ataccactcg cagtactaat atgattcttc aacatgtctt atatattct 49
<210> 230
   <211> 38
   <212> DNA
   <213> Human papillomavirus
<400> 230
   cctggacaac cgggtgctgt tggaggctta catccaag 38
<210> 231
   <211> 44
   <212> DNA
   <213> Human papillomavirus
<400> 231
   tatcctggac aaccgggtgc tcctgttgga ggcttacatc caag 44
<210> 232
   <211> 22
   <212> DNA
   <213> Human papillomavirus
<400> 232
   ggaggcttac atccaagtaa ac 22
<210> 233
   <211> 22
   <212> DNA
   <213> Human papillomavirus

<400> 233
   gtacaagcaa cacctttacc cc 22
<210> 234
   <211> 50
   <212> DNA
   <213> Human papillomavirus
<400> 234
   gacactgaaa ccggtaacaa gtatccactg tgtttgttta taatccatgg 50
<210> 235
   <211> 46
   <212> DNA
   <213> Human papillomavirus
<400> 235
   ggatgaccac ttatgccaac gccacaacga ttagtatggg catgtg 46
<210> 236
   <211> 24
   <212> DNA
   <213> Human papillomavirus
<400> 236
   cctgacacct ccttttataa ccct 24
<210> 237
   <211> 23
   <212> DNA
   <213> Human papillomavirus
<400> 237
   cctgacacct ccttttataa ccc 23
<210> 238
   <211> 45
   <212> DNA
   <213> Human papillomavirus
<400> 238
   cacttatgcc aacgcctaat gggatacaca acgattagta tgggc 45
<210> 239
   <211> 43
   <212> DNA
   <213> Human papillomavirus
<400> 239
   ggatgaccac ttatgccaac gcacaacgat tagtatgggc atg 43
<210> 240
   <211> 23
   <212> DNA
   <213> Human papillomavirus
<400> 240
   gctgccctct acctatttca agg 23
<210> 241
   <211> 20
   <212> DNA
   <213> Human papillomavirus
<400> 241
   cacctttacc ccaatgttcc 20
<210> 242
   <211> 22
   <212> DNA
   <213> Human papillomavirus
<400> 242
   gtcatattag tactgcgagt gg 22
<210> 243
   <211> 40
   <212> DNA
   <213> Human papillomavirus
<400> 243
   ccgggtgctg ataataggga cctgttggag gcttacatcc 40
<210> 244
   <211> 38
   <212> DNA
   <213> Human papillomavirus
<400> 244
   ccatattggc tacaacgtgc tacctgattg ccccaaca 38
<210> 245
   <211> 40
   <212> DNA
   <213> Human papillomavirus
<400> 245
   ccatattggc tacaacgtgc accaaatacc tgattgcccc 40
<210> 246
   <211> 22
   <212> DNA
   <213> Human papillomavirus
<400> 246
   gtgcacaagg tcataataat gg 22
<210> 247
   <211> 19
   <212> DNA
   <213> Human papillomavirus
<400> 247
   agtatgggca tgtgtaggc 19
<210> 248
   <211> 20
   <212> DNA
   <213> Human papillomavirus
<400> 248
   agggctggta cattaggaga 20
<210> 249
   <211> 19
   <212> DNA
   <213> Human papillomavirus
<400> 249
   tgttcccgat gacctgtac 19
<210> 250
   <211> 39
   <212> DNA
   <213> Human papillomavirus
<400> 250
   cttgttaccg gtttcagtgt cagcagccat taggcgttg 39
<210> 251
   <211> 38
   <212> DNA
   <213> Human papillomavirus
<400> 251
   gcactgcttt gaatagaggc actgttcccg atgacctg 38
<210> 252
   <211> 45
   <212> DNA
   <213> Human papillomavirus
<400> 252
   gtaaccattg atccactagg agtgaaaggt tcaggaacta ctgcc 45
<210> 253
   <211> 26
   <212> DNA
   <213> Human papillomavirus
<400> 253
   gtagttcctg aacctttaat gtacag 26
<210> 254
   <211> 21
   <212> DNA
   <213> Human papillomavirus
<400> 254
   gcactgcttt gaatagaggc a 21
<210> 255
   <211> 27
   <212> DNA
   <213> Human papillomavirus
<400> 255
   cagtagttcc tgaaccttta atgtaca 27

<210> 256
   <211> 22
   <212> DNA
   <213> Human papillomavirus
<400> 256
   ggaggcttac atccaagtaa ac 22
<210> 257
   <211> 50
   <212> DNA
   <213> Human papillomavirus
<400> 257
   gacactgaaa ccggtaacaa gtatccactg tgtttgttta taatccatgg 50
<210> 258
   <211> 24
   <212> DNA
   <213> Human papillomavirus
<400> 258
   cctgacacct ccttttataa ccct 24
<210> 259
   <211> 45
   <212> DNA
   <213> Human papillomavirus
<400> 259
   cacttatgcc aacgcctaat gggatacaca acgattagta tgggc 45
<210> 260
   <211> 20
   <212> DNA
   <213> Human papillomavirus
<400> 260
   caaatcctgt gtctaccatg 20
<210> 261
   <211> 42
   <212> DNA
   <213> Human papillomavirus
<400> 261
   ggcacacctt gtaatgctaa ctccccgtct tgtagtacag tg 42
<210> 262
   <211> 22
   <212> DNA
   <213> Human papillomavirus
<400> 262
   atgttggtaa ctctggtaac tc 22
<210> 263
   <211> 44
   <212> DNA
   <213> Human papillomavirus
<400> 263
   ggaggcctac aacctattaa acaggtacag ataacaggga atgc 44
<210> 264
   <211> 25
   <212> DNA
   <213> Human papillomavirus
<400> 264
   aattgtgttt gtttataatc catag 25
<210> 265
   <211> 18
   <212> DNA
   <213> Human papillomavirus
<400> 265
   tgcaccaaat cctgtgtc 18
<210> 266
   <211> 39
   <212> DNA
   <213> Human papillomavirus
<400> 266
   gctaaccagg taaaagcagg ataccatgtc cccgtcttg 39
<210> 267
   <211> 22
   <212> DNA
   <213> Human papillomavirus
<400> 267
   aactctggta actctggtac ag 22
<210> 268
   <211> 44
   <212> DNA
   <213> Human papillomavirus
<400> 268
   ggaggcctac aacctattaa acaacaggga atgcatttct atgg 44
<210> 269
   <211> 26
   <212> DNA
   <213> Human papillomavirus
<400> 269
   caattgtgtt tgtttataat ccatag 26
<210> 270
   <211> 20
   <212> DNA
   <213> Human papillomavirus
<400> 270
   gatatttgca aatggaactg 20
<210> 271
   <211> 41
   <212> DNA
   <213> Human papillomavirus
<400> 271
   gacggggaca tggtagacac atatatctag gggaacatca c 41
<210> 272
   <211> 21
   <212> DNA
   <213> Human papillomavirus
<400> 272
   gtgtttaata ggttgtaggc c 21
<210> 273
   <211> 42
   <212> DNA
   <213> Human papillomavirus
<400> 273
   ctcctgcttt tacctggtta gctcctatag gtgaacattg gg 42
<210> 274
   <211> 20
   <212> DNA
   <213> Human papillomavirus
<400> 274
   attacaaggt gtgccttttc 20
<210> 275
   <211> 21
   <212> DNA
   <213> Human papillomavirus
<400> 275
   ggatatttgc aaatggaact g 21
<210> 276
   <211> 42
   <212> DNA
   <213> Human papillomavirus
<400> 276
   gggacatggt agacacagga catatatcta ggggaacatc ac 42
<210> 277
   <211> 19
   <212> DNA
   <213> Human papillomavirus
<400> 277
   cctataggtg aacattggg 19
<210> 278
   <211> 43
   <212> DNA
   <213> Human papillomavirus

<400> 278
   gtttagtaac tccaaaggag gacaaaggca caccttgtaa tgc 43
<210> 279
   <211> 20
   <212> DNA
   <213> Human papillomavirus
<400> 279
   attctcctgc ttttacctgg 20
<210> 280
   <211> 22
   <212> DNA
   <213> Human papillomavirus
<400> 280
   cattctcctg cttttacctg gt 22
<210> 281
   <211> 22
   <212> DNA
   <213> Human papillomavirus
<400> 281
   atcctctaaa atggacgggt tc 22
<210> 282
   <211> 50
   <212> DNA
   <213> Human papillomavirus
<400> 282
   caacccgtag tacaaatatg tctgatatgt cataacatct gctgttagtg 50
<210> 283
   <211> 25
   <212> DNA
   <213> Human papillomavirus
<400> 283
   ctagttattt tcctactcct agtgg 25
<210> 284
   <211> 47
   <212> DNA
   <213> Human papillomavirus
<400> 284
   caattggtta ctccaacaaa taccctctat ggtaacctcc gatgcac 47
<210> 285
   <211> 46
   <212> DNA
   <213> Human papillomavirus
<400> 285
   ccattattat ggccttgtgc acgctctatg gtaacctccg atgcac 46
<210> 286
   <211> 45
   <212> DNA
   <213> Human papillomavirus
<400> 286
   atggccttgt gcacgttgca acctctatgg taacctccga tgcac 45
<210> 287
   <211> 38
   <212> DNA
   <213> Human papillomavirus
<400> 287
   ggccttgtgc acgttgccta tggtaacctc cgatgcac 38
<210> 288
   <211> 38
   <212> DNA
   <213> Human papillomavirus
<400> 288
   gccttgtgca cgttgcacta tggtaacctc cgatgcac 38
<210> 289
   <211> 24
   <212> DNA
   <213> Human papillomavirus
<400> 289
   atgtcataac atctgctgtt agtg 24
<210> 290
   <211> 24
   <212> DNA
   <213> Human papillomavirus
<400> 290
   atgtcataac atctgctgtt agtg 24
<210> 291
   <211> 49
   <212> DNA
   <213> Human papillomavirus
<400> 291
   caacccgtag tacaaatatg tctgttcttc accatgcctt aaatattcc 49
<210> 292
   <211> 41
   <212> DNA
   <213> Human papillomavirus
<400> 292
   cccgtagtac aaatatgtct gcaccatgcc ttaaatattc c 41
<210> 293
   <211> 43
   <212> DNA
   <213> Human papillomavirus
<400> 293
   cccgtagtac aaatatgtct gttcaccatg ccttaaatat tcc 43
<210> 294
   <211> 44
   <212> DNA
   <213> Human papillomavirus
<400> 294
   cccgtagtac aaatatgtct gcttcaccat gccttaaata ttcc 44
<210> 295
   <211> 24
   <212> DNA
   <213> Human papillomavirus
<400> 295
   ccaatatggt ttattaaata tttg 24
<210> 296
   <211> 25
   <212> DNA
   <213> Human papillomavirus
<400> 296
   caatatggtt tattaaatat ttgtg 25
<210> 297
   <211> 26
   <212> DNA
   <213> Human papillomavirus
<400> 297
   caatatggtt tattaaatat ttgtgc 26
<210> 298
   <211> 47
   <212> DNA
   <213> Human papillomavirus
<400> 298
   gttggtaact ctggtaactc tggggaggcc tacaacctat taaacac 47
<210> 299
   <211> 46
   <212> DNA
   <213> Human papillomavirus
<400> 299
   gttggtaact ctggtaactc tgggaggcct acaacctatt aaacac 46
<210> 300
   <211> 22
   <212> DNA
   <213> Human papillomavirus
<400> 300
   cccaatgttc acctatagga gg 22

<210> 301
   <211> 21
   <212> DNA
   <213> Human papillomavirus
<400> 301
   gccttttccc caatgttcac c 21
<210> 302
   <211> 52
   <212> DNA
   <213> Human papillomavirus
<400> 302
   tggtaactct ggtaactctg gtacagccta caacctatta aacacaattg tg 52
<210> 303
   <211> 45
   <212> DNA
   <213> Human papillomavirus
<400> 303
   ggatgaccac taatacctac tccgtttggt ttgggcctgt acagg 45
<210> 304
   <211> 44
   <212> DNA
   <213> Human papillomavirus
<400> 304
   ggatgaccac taatacctac tccgtttggt ttgggcctgt acag 44
<210> 305
   <211> 23
   <212> DNA
   <213> Human papillomavirus
<400> 305
   ccagacacat cattttatga tcc 23
<210> 306
   <211> 20
   <212> DNA
   <213> Human papillomavirus
<400> 306
   ttatgatccc tgcctccagc 20
<210> 307
   <211> 43
   <212> DNA
   <213> Human papillomavirus
<400> 307
   gaccactaat acctactcct aatggcctgc ctccagcgtt tgg 43
<210> 308
   <211> 18
   <212> DNA
   <213> Human papillomavirus
<400> 308
   agcattacaa ggtgtgcc 18
<210> 309
   <211> 43
   <212> DNA
   <213> Human papillomavirus
<400> 309
   cagataacag ggaatgcatt tcaatgttca cctataggag gcc 43
<210> 310
   <211> 18
   <212> DNA
   <213> Human papillomavirus
<400> 310
   aagtaggtcg tggtcagc 18
<210> 311
   <211> 46
   <212> DNA
   <213> Human papillomavirus
<400> 311
   ccagagttac cagagttacc aacggagtag gtattagtgg tcatcc 46
<210> 312
   <211> 24
   <212> DNA
   <213> Human papillomavirus
<400> 312
   gattttcagt atcatccaat ttat 24
<210> 313
   <211> 22
   <212> DNA
   <213> Human papillomavirus
<400> 313
   cccaatgttc acctatagga gg 22
<210> 314
   <211> 52
   <212> DNA
   <213> Human papillomavirus
<400> 314
   tggtaactct ggtaactctg gtacagccta caacctatta aacacaattg tg 52
<210> 315
   <211> 23
   <212> DNA
   <213> Human papillomavirus
   <400> 315
   ccagacacat cattttatga tcc 23
<210> 316
   <211> 43
   <212> DNA
   <213> Human papillomavirus
<400> 316
   gaccactaat acctactcct aatggcctgc ctccagcgtt tgg 43
<210> 317
   <211> 25
   <212> DNA
   <213> Human papillomavirus
<400> 317
   ccaattgtcc aatatagagg aattc 25
<210> 318
   <211> 49
   <212> DNA
   <213> Human papillomavirus
<400> 318
   actacccgta gtaccaactt tacgacataa catcagttgt taatgtgac 49
<210> 319
   <211> 21
   <212> DNA
   <213> Human papillomavirus
<400> 319
   gttctgtata ctgcccctct c 21
<210> 320
   <211> 46
   <212> DNA
   <213> Human papillomavirus
<400> 320
   aacatatacc attgttgtgg cccttccatg gtaacctctg attccc 46
<210> 321
   <211> 41
   <212> DNA
   <213> Human papillomavirus
   <400> 321
   gccttatgta gccaataagg cccagcggtt ccatggtaac c 41
<210> 322
   <211> 42
   <212> DNA
   <213> Human papillomavirus
<400> 322
   gccttatgta gccaataagg cgcggttcca tggtaacctc tg 42

<210> 323
   <211> 41
   <212> DNA
   <213> Human papillomavirus
<400> 323
   gccaacatat accattgttg tcatggtaac ctctgattcc c 41
<210> 324
   <211> 26
   <212> DNA
   <213> Human papillomavirus
<400> 324
   gacataacat cagttgttaa tgtgac 26
<210> 325
   <211> 44
   <212> DNA
   <213> Human papillomavirus
<400> 325
   actacccgta gtaccaactt tactccacgt gcctggtata ttcc 44
<210> 326
   <211> 45
   <212> DNA
   <213> Human papillomavirus
<400> 326
   actacccgta gtaccaactt tactccacgt gcctggtata ttcct 45
<210> 327
   <211> 42
   <212> DNA
   <213> Human papillomavirus
<400> 327
   ctacccgtag taccaacttt acccacgtgc ctggtatatt cc 42
<210> 328
   <211> 20
   <212> DNA
   <213> Human papillomavirus
<400> 328
   ccttatgtag ccaataaggc 20
<210> 329
   <211> 22
   <212> DNA
   <213> Human papillomavirus
<400> 329
   ggccttatgt agccaataag gc 22
<210> 330
   <211> 45
   <212> DNA
   <213> Human papillomavirus
<400> 330
   cagtagggat aatgtgtctg tggatgcctt tcccttaccc cagtg 45
<210> 331
   <211> 17
   <212> DNA
   <213> Human papillomavirus
<400> 331
   aatggcggga acacagc 17
<210> 332
   <211> 24
   <212> DNA
   <213> Human papillomavirus
<400> 332
   ccgtagatac attattgggc ttgc 24
<210> 333
   <211> 52
   <212> DNA
   <213> Human papillomavirus
<400> 333
   ctgaaaactc accattttca tcaacccaca actgtgtctg tttataatcc ac 52
<210> 334
   <211> 48
   <212> DNA
   <213> Human papillomavirus
<400> 334
   ggtgagtttt cagtatcatc ctgtcccatt gggtgttggt attagtgg 48
<210> 335
   <211> 24
   <212> DNA
   <213> Human papillomavirus
<400> 335
   ccagatgcat ccttatataa tcca 24
<210> 336
   <211> 22
   <212> DNA
   <213> Human papillomavirus
   <400> 336
   gtttagtatg ggcttgtgta gg 22
<210> 337
   <211> 47
   <212> DNA
   <213> Human papillomavirus
<400> 337
   atgggtgtcc actaatacca acacgtttag tatgggcttg tgtaggg 47
<210> 338
   <211> 17
   <212> DNA
   <213> Human papillomavirus
<400> 338
   aatggcggga acacagc 17
<210> 339
   <211> 52
   <212> DNA
   <213> Human papillomavirus
<400> 339
   ctgaaaactc accattttca tcaacccaca actgtgtctg tttataatcc ac 52
<210> 340
   <211> 24
   <212> DNA
   <213> Human papillomavirus
<400> 340
   ccagatgcat ccttatataa tcca 24
<210> 341
   <211> 47
   <212> DNA
   <213> Human papillomavirus
<400> 341
   atgggtgtcc actaatacca acacgtttag tatgggcttg tgtaggg 47
<210> 342
   <211> 22
   <212> DNA
   <213> Human papillomavirus
<400> 342
   tggtggaggg acaccaaaat tc 22
<210> 343
   <211> 27
   <212> DNA
   <213> Human papillomavirus
<400> 343
   ttccaatttt ctaatatact actattc 27
<210> 344
   <211> 50
   <212> DNA
   <213> Human papillomavirus
<400> 344
   cactacccgc agtactaatt taacatatga cataacctct gcagttaaag 50
<210> 345
   <211> 48
   <212> DNA
   <213> Human papillomavirus

<400> 345
   cactacccgc agtactaatt taacggatat atgacataac ctctgcag 48
<210> 346
   <211> 48
   <212> DNA
   <213> Human papillomavirus
<400> 346
   cactacccgc agtactaatt taaccctctg cagttaaagt aatagtgc 48
<210> 347
   <211> 50
   <212> DNA
   <213> Human papillomavirus
<400> 347
   cactacccgc agtactaatt taacctatgg atatatgaca taacctctgc 50
<210> 348
   <211> 19
   <212> DNA
   <213> Human papillomavirus
<400> 348
   tgtattcccc ttctcccag 19
<210> 349
   <211> 19
   <212> DNA
   <213> Human papillomavirus
<400> 349
   gtgaaacccc tggcagttg 19
<210> 350
   <211> 48
   <212> DNA
   <213> Human papillomavirus
<400> 350
   aaataccatt gttatggccc tggggctcta ttattacttc tgattctc 48
<210> 351
   <211> 43
   <212> DNA
   <213> Human papillomavirus
<400> 351
   ataccattgt tatggccctg ggtgtgtatt ccccttctcc cag 43
<210> 352
   <211> 43
   <212> DNA
   <213> Human papillomavirus
<400> 352
   ataccattgt tatggccctg ggtgtattcc ccttctccca gtg 43
<210> 353
   <211> 47
   <212> DNA
   <213> Human papillomavirus
<400> 353
   ataccattgt tatggccctg ggggctctat tattacttct gattctc 47
<210> 354
   <211> 47
   <212> DNA
   <213> Human papillomavirus
<400> 354
   caactgatta tgccaacaaa taccagccat attggttaca taaggcc 47
<210> 355
   <211> 25
   <212> DNA
   <213> Human papillomavirus
<400> 355
   tatgacataa cctctgcagt taaag 25
<210> 356
   <211> 47
   <212> DNA
   <213> Human papillomavirus
<400> 356
   cactacccgc agtactaatt taaccctcca catgtctact atactgc 47
<210> 357
   <211> 49
   <212> DNA
   <213> Human papillomavirus
<400> 357
   cactacccgc agtactaatt taacactata ctgcttaaac ttagtaggg 49
<210> 358
   <211> 48
   <212> DNA
   <213> Human papillomavirus
<400> 358
   ggatgataca gaaagtgctc atgccctaaa atacacagct gtgtttgc 48
<210> 359
   <211> 41
   <212> DNA
   <213> Human papillomavirus
<400> 359
   ggatgataca gaaagtgctc aaaatacaca gctgtgtttg c 41
<210> 360
   <211> 22
   <212> DNA
   <213> Human papillomavirus
<400> 360
   caccaatagc aggtacacaa cc 22
<210> 361
   <211> 21
   <212> DNA
   <213> Human papillomavirus
<400> 361
   gtgctcacca atagcaggta c 21
<210> 362
   <211> 52
   <212> DNA
   <213> Human papillomavirus
<400> 362
   ggatgataca gaaagtgctc atgcagctgt ttgcttataa tcaactgaca ca 52
<210> 363
   <211> 45
   <212> DNA
   <213> Human papillomavirus
<400> 363
   taaaatggat ggccacttag gccggtatgg aaattggtcg tgggc 45
<210> 364
   <211> 42
   <212> DNA
   <213> Human papillomavirus
<400> 364
   ccacttaggc caatacctaa gtgtaggtat ggaaattggt cg 42
<210> 365
   <211> 21
   <212> DNA
   <213> Human papillomavirus
<400> 365
   cctgaaacac aacgtttggt t 21
<210> 366
   <211> 23
   <212> DNA
   <213> Human papillomavirus
<400> 366
   gaaacacaac gtttggtttg ggc 23
<210> 367
   <211> 46
   <212> DNA
   <213> Human papillomavirus
<400> 367
   gccacttagg ccaataccta aaggcatgtg taggtatgga aattgg 46

<210> 368
   <211> 44
   <212> DNA
   <213> Human papillomavirus
<400> 368
   gccacttagg ccaataccta aagtgggcat gtgtaggtat ggaa 44
<210> 369
   <211> 15
   <212> DNA
   <213> Human papillomavirus
<400> 369
   aggctgccca cgacc 15
<210> 370
   <211> 18
   <212> DNA
   <213> Human papillomavirus
<400> 370
   caatacctaa aggctgcc 18
<210> 371
   <211> 22
   <212> DNA
   <213> Human papillomavirus
<400> 371
   caccaatagc aggtacacaa cc 22
<210> 372
   <211> 52
   <212> DNA
   <213> Human papillomavirus
<400> 372
   ggatgataca gaaagtgctc atgcagctgt ttgcttataa tcaactgaca ca 52
<210> 373
   <211> 21
   <212> DNA
   <213> Human papillomavirus
<400> 373
   cctgaaacac aacgtttggt t 21
<210> 374
   <211> 46
   <212> DNA
   <213> Human papillomavirus
<400> 374
   gccacttagg ccaataccta aaggcatgtg taggtatgga aattgg 46
<210> 375
   <211> 44
   <212> DNA
   <213> Human papillomavirus
<400> 375
   gccacttagg ccaataccta aagtgggcat gtgtaggtat ggaa 44
<210> 376
   <211> 22
   <212> DNA
   <213> Human papillomavirus
   <400> 376
   ctgttcaaga atggtaggat cc 22
<210> 377
   <211> 22
   <212> DNA
   <213> Human papillomavirus
   <400> 377
   tccactgttc aagaatggta gg 22
<210> 378
   <211> 48
   <212> DNA
   <213> Human papillomavirus
<400> 378
   taactattag cactgccact gcataagcca ttacctctgt agttaaag 48
<210> 379
   <211> 47
   <212> DNA
   <213> Human papillomavirus
<400> 379
   taactattag cactgccact gcgtgtgtaa ataagccatt acctctg 47
<210> 380
   <211> 22
   <212> DNA
   <213> Human papillomavirus
<400> 380
   atatactctg ctactcccag tg 22
<210> 381
   <211> 20
   <212> DNA
   <213> Human papillomavirus
<400> 381
   ggccgtgacc ctatagaaag 20
<210> 382
   <211> 46
   <212> DNA
   <213> Human papillomavirus
<400> 382
   gctgattgtt ccagcaaatg ccggtctatg ataacatctg attctc 46
<210> 383
   <211> 44
   <212> DNA
   <213> Human papillomavirus
<400> 383
   attattgtga ccctgcgcac ggatactctg ctactcccag tggg 44
<210> 384
   <211> 25
   <212> DNA
   <213> Human papillomavirus
<400> 384
   taagccatta cctctgtagt taaag 25
<210> 385
   <211> 45
   <212> DNA
   <213> Human papillomavirus
   <400> 385
   taactattag cactgccact gccttcccca tgcctaatat attgc 45
<210> 386
   <211> 48
   <212> DNA
   <213> Human papillomavirus
<400> 386
   atactaccag aagtacaaat ttaaccttcc ccatgcctaa tatattgc 48
<210> 387
   <211> 47
   <212> DNA
   <213> Human papillomavirus
<400> 387
   gcacaacaag atgttagaga taacacccaa taggtggagc acagcct 47
<210> 388
   <211> 21
   <212> DNA
   <213> Human papillomavirus
<400> 388
   ttgcatgtag tgccaatacc c 21
<210> 389
   <211> 20
   <212> DNA
   <213> Human papillomavirus
<400> 389
   gcatgtagtg ccaatacccc 20
<210> 390
   <211> 46
   <212> DNA
   <213> Human papillomavirus

<400> 390
   tgcacaacaa gatgttagag ataacacaat aggtggagca cagcct 46
<210> 391
   <211> 42
   <212> DNA
   <213> Human papillomavirus
<400> 391
   gcacaacaag atgttagaga taacaatagg tggagcacag cc 42
<210> 392
   <211> 40
   <212> DNA
   <213> Human papillomavirus
<400> 392
   gcacaacaag atgttagaga taaataggtg gagcacagcc 40
<210> 393
   <211> 40
   <212> DNA
   <213> Human papillomavirus
<400> 393
   gcacaacaag atgttagaga tcaataggtg gagcacagcc 40
<210> 394
   <211> 46
   <212> DNA
   <213> Human papillomavirus
<400> 394
   tgctatgcgt gaattttctg tgtcttggtg ttggccttag tggtca 46
<210> 395
   <211> 19
   <212> DNA
   <213> Human papillomavirus
<400> 395
   gttgaggtgg gcagaggac 19
<210> 396
   <211> 21
   <212> DNA
   <213> Human papillomavirus
<400> 396
   ccagacacag ataggttggt g 21
<210> 397
   <211> 44
   <212> DNA
   <213> Human papillomavirus
<400> 397
   ctatgcgtga attttctgtg tcatcccttg gtgttggcct tagt 44
<210> 398
   <211> 39
   <212> DNA
   <213> Human papillomavirus
<400> 398
   ctatgcgtga attttctgtg cccttggtgt tggccttag 39
<210> 399
   <211> 40
   <212> DNA
   <213> Human papillomavirus
<400> 399
   gctatgcgtg aattttctgt gcccttggtg ttggccttag 40
<210> 400
   <211> 25
   <212> DNA
   <213> Human papillomavirus
<400> 400
   catcatattt attaaataag ggatg 25
<210> 401
   <211> 22
   <212> DNA
   <213> Human papillomavirus
<400> 401
   tttattaaat aagggatgac ca 22
<210> 402
   <211> 27
   <212> DNA
   <213> Human papillomavirus
<400> 402
   catatttatt aaataaggga tgaccac 27
<210> 403
   <211> 21
   <212> DNA
   <213> Human papillomavirus
<400> 403
   ttgcatgtag tgccaatacc c 21
<210> 404
   <211> 46
   <212> DNA
   <213> Human papillomavirus
<400> 404
   tgcacaacaa gatgttagag ataacacaat aggtggagca cagcct 46
<210> 405
   <211> 19
   <212> DNA
   <213> Human papillomavirus
<400> 405
   gttgaggtgg gcagaggac 19
<210> 406
   <211> 44
   <212> DNA
   <213> Human papillomavirus
<400> 406
   ctatgcgtga attttctgtg tcatcccttg gtgttggcct tagt 44
<210> 407
   <211> 20
   <212> DNA
   <213> Human papillomavirus
<400> 407
   cctcagcaca taaagtcatg 20
<210> 408
   <211> 41
   <212> DNA
   <213> Human papillomavirus
<400> 408
   gtactggtta caacgtgcgc agtggtatcc acaactgtga c 41
<210> 409
   <211> 18
   <212> DNA
   <213> Human papillomavirus
<400> 409
   gggtctaact ctggcaat 18
<210> 410
   <211> 42
   <212> DNA
   <213> Human papillomavirus
<400> 410
   ggattctgag gttaccatag aaccactgcc actgtacaaa gc 42
<210> 411
   <211> 20
   <212> DNA
   <213> Human papillomavirus
<400> 411
   cctcagcaca taaagtcatg 20
<210> 412
   <211> 38
   <212> DNA
   <213> Human papillomavirus
<400> 412
   cagggccaca ataatggcat acgagtggta tccacaac 38

<210> 413
   <211> 18
   <212> DNA
   <213> Human papillomavirus
<400> 413
   gggtctaact ctggcaat 18
<210> 414
   <211> 42
   <212> DNA
   <213> Human papillomavirus
<400> 414
   ggattctgag gttaccatag aaccactgcc actgtacaaa gc 42
<210> 415
   <211> 22
   <212> DNA
   <213> Human papillomavirus
<400> 415
   gtcctctaaa atagtggcat cc 22
<210> 416
   <211> 19
   <212> DNA
   <213> Human papillomavirus
<400> 416
   ggggtaaggc caaattgcc 19
<210> 417
   <211> 48
   <212> DNA
   <213> Human papillomavirus
<400> 417
   ccactcgtag cactaacatg acgtatgtca taacatcagc tgttaatg 48
<210> 418
   <211> 45
   <212> DNA
   <213> Human papillomavirus
<400> 418
   ccactcgtag cactaacatg accctctaaa atagtggcat ccatc 45
<210> 419
   <211> 23
   <212> DNA
   <213> Human papillomavirus
<400> 419
   gctttttttc ctactcctag tgg 23
<210> 420
   <211> 20
   <212> DNA
   <213> Human papillomavirus
<400> 420
   gccactgtac aaagcagtgc 20
<210> 421
   <211> 45
   <212> DNA
   <213> Human papillomavirus
<400> 421
   actgattgcc ccaacatatg ccttctatgg taacctcaga atccc 45
<210> 422
   <211> 44
   <212> DNA
   <213> Human papillomavirus
<400> 422
   attattgtgg ccctgcgcac gttctatggt aacctcagaa tccc 44
<210> 423
   <211> 38
   <212> DNA
   <213> Human papillomavirus
<400> 423
   ccctgcgcac gttgtaacta tggtaacctc agaatccc 38
<210> 424
   <211> 42
   <212> DNA
   <213> Human papillomavirus
<400> 424
   ccaacatatg ccattattgt gctatggtaa cctcagaatc cc 42
<210> 425
   <211> 45
   <212> DNA
   <213> Human papillomavirus
<400> 425
   accactcgta gcactaacat gactcgccat gacgaaggta ttcct 45
<210> 426
   <211> 39
   <212> DNA
   <213> Human papillomavirus
<400> 426
   ccactcgtag cactaacatg gccatgacga aggtattcc 39
<210> 427
   <211> 41
   <212> DNA
   <213> Human papillomavirus
<400> 427
   ccactcgtag cactaacatg acgccatgac gaaggtattc c 41
<210> 428
   <211> 26
   <212> DNA
   <213> Human papillomavirus
<400> 428
   gtatgtcata acatcagctg ttaatg 26
<210> 429
   <211> 25
   <212> DNA
   <213> Human papillomavirus
<400> 429
   tgaatgtatg tcataacatc agctg 25
<210> 430
   <211> 43
   <212> DNA
   <213> Human papillomavirus
<400> 430
   ccactcgtag cactaacatg actcgccatg acgaaggtat tcc 43
<210> 431
   <211> 24
   <212> DNA
   <213> Human papillomavirus
<400> 431
   gctgaggtta aaaaggaaag caca 24
<210> 432
   <211> 26
   <212> DNA
   <213> Human papillomavirus
<400> 432
   gactttatgt gctgaggtta aaaagg 26
<210> 433
   <211> 28
   <212> DNA
   <213> Human papillomavirus
<400> 433
   ctttatgtgc tgaggttaaa aaggaaag 28
<210> 434
   <211> 23
   <212> DNA
   <213> Human papillomavirus
<400> 434
   ccagtacggt ttattaaata att 23
<210> 435
   <211> 20
   <212> DNA
   <213> Human papillomavirus

<400> 435
   gcgcacgttg taaccagtac 20
<210> 436
   <211> 27
   <212> DNA
   <213> Human papillomavirus
<400> 436
   gttgtaacca gtacggttta ttaaata 27
<210> 437
   <211> 27
   <212> DNA
   <213> Human papillomavirus
<400> 437
   cgttgtaacc agtacggttt attaaat 27
<210> 438
   <211> 22
   <212> DNA
   <213> Human papillomavirus
<400> 438
   cacctatagg aggtttgcat cc 22
<210> 439
   <211> 53
   <212> DNA
   <213> Human papillomavirus
<400> 439
   tgatactgaa accagtaaca aatatgctaa ctgagtctgc ttataatcca tag 53
<210> 440
   <211> 25
   <212> DNA
   <213> Human papillomavirus
<400> 440
   ccagatacat ctttttataa cccag 25
<210> 441
   <211> 43
   <212> DNA
   <213> Human papillomavirus
<400> 441
   aaaggatgcc cactaatacc cacaaaaccc aaaggttggt gtg 43
<210> 442
   <211> 20
   <212> DNA
   <213> Human papillomavirus
<400> 442
   cctcagcaca taaagtcatg 20
<210> 443
   <211> 40
   <212> DNA
   <213> Human papillomavirus
<400> 443
   cagggccaca ataatggcat agtggtatcc acaactgtga 40
<210> 444
   <211> 18
   <212> DNA
   <213> Human papillomavirus
<400> 444
   gccactgtac aaagcagt 18
<210> 445
   <211> 42
   <212> DNA
   <213> Human papillomavirus
<400> 445
   cacgttgtaa ccagtacggt ttttcctact cctagtggtt ct 42
<210> 446
   <211> 22
   <212> DNA
   <213> Human papillomavirus
<400> 446
   cacctatagg aggtttgcat cc 22
<210> 447
   <211> 53
   <212> DNA
   <213> Human papillomavirus
<400> 447
   tgatactgaa accagtaaca aatatgctaa ctgagtctgc ttataatcca tag 53
<210> 448
   <211> 25
   <212> DNA
   <213> Human papillomavirus
<400> 448
   ccagatacat ctttttataa cccag 25
<210> 449
   <211> 43
   <212> DNA
   <213> Human papillomavirus
<400> 449
   aaaggatgcc cactaatacc cacaaaaccc aaaggttggt gtg 43
<210> 450
   <211> 22
   <212> DNA
   <213> Human papillomavirus
<400> 450
   gtcctccagt aggttagcat tc 22
<210> 451
   <211> 49
   <212> DNA
   <213> Human papillomavirus
<400> 451
   agtactaaca tgactattag tactgctgcc ataacctctg cagacaaag 49
<210> 452
   <211> 48
   <212> DNA
   <213> Human papillomavirus
<400> 452
   atactactag aagtactaac atgactgcca taacctctgc agacaaag 48
<210> 453
   <211> 22
   <212> DNA
   <213> Human papillomavirus
<400> 453
   gtatatgttg ctacgcctag tg 22
<210> 454
   <211> 46
   <212> DNA
   <213> Human papillomavirus
<400> 454
   aattgattac cccagcaaat gccgtctatg attacgtctg aggcac 46
<210> 455
   <211> 41
   <212> DNA
   <213> Human papillomavirus
<400> 455
   ccccagcaaa tgccattatt ctatgattac gtctgaggca c 41
<210> 456
   <211> 22
   <212> DNA
   <213> Human papillomavirus
<400> 456
   gccataacct ctgcagacaa ag 22
<210> 457
   <211> 47
   <212> DNA
   <213> Human papillomavirus
<400> 457
   atactactag aagtactaac atgaccctcc acatgtctaa ggtactg 47

<210> 458
   <211> 20
   <212> DNA
   <213> Human papillomavirus
<400> 458
   gcacgttgca accaataagg 20
<210> 459
   <211> 46
   <212> DNA
   <213> Human papillomavirus
<400> 459
   ggctggatga tactgaaagt tcccacaact gtgtttgctt gccatc 46
<210> 460
   <211> 19
   <212> DNA
   <213> Human papillomavirus
<400> 460
   tccaatgttc acccatagc 19
<210> 461
   <211> 20
   <212> DNA
   <213> Human papillomavirus
<400> 461
   ccaatgttca cccatagcgg 20
<210> 462
   <211> 47
   <212> DNA
   <213> Human papillomavirus
<400> 462
   ggatgatact gaaagttcca atttagccac aactgtgttt gcttgcc 47
<210> 463
   <211> 45
   <212> DNA
   <213> Human papillomavirus
<400> 463
   aaacaatgga tggccactta gccatgtgta ggtttggagg taggc 45
<210> 464
   <211> 21
   <212> DNA
   <213> Human papillomavirus
<400> 464
   ttataatccg gaccaggaac g 21
<210> 465
   <211> 20
   <212> DNA
   <213> Human papillomavirus
<400> 465
   ataatccgga ccaggaacgg 20
<210> 466
   <211> 44
   <212> DNA
   <213> Human papillomavirus
<400> 466
   acaatggatg gccacttagc cgcatgtgta ggtttggagg tagg 44
<210> 467
   <211> 47
   <212> DNA
   <213> Human papillomavirus
<400> 467
   ggatgatact gaaagttcca atttagccac aactgtgttt gcttgcc 47
<210> 468
   <211> 21
   <212> DNA
   <213> Human papillomavirus
<400> 468
   ttataatccg gaccaggaac g 21
<210> 469
   <211> 44
   <212> DNA
   <213> Human papillomavirus
<400> 469
   acaatggatg gccacttagc cgcatgtgta ggtttggagg tagg 44
<210> 470
   <211> 19
   <212> DNA
   <213> Human papillomavirus
<400> 470
   tccaatgttc acccatagc 19
<210> 471
   <211> 20
   <212> DNA
   <213> Human papillomavirus
<400> 471
   caaagtccat gcatccaaac 20
<210> 472
   <211> 41
   <212> DNA
   <213> Human papillomavirus
<400> 472
   gcctgtaaca ataatgcagc tgcaccatgt caccatcctc a 41
<210> 473
   <211> 18
   <212> DNA
   <213> Human papillomavirus
<400> 473
   aacagatatc ccgcacag 18
<210> 474
   <211> 42
   <212> DNA
   <213> Human papillomavirus
<400> 474
   gtgggaggtt tacagccaat taggtctgat aacagggaat gc 42
<210> 475
   <211> 20
   <212> DNA
   <213> Human papillomavirus
<400> 475
   ccattgttat gaccttgtgc 20
<210> 476
   <211> 41
   <212> DNA
   <213> Human papillomavirus
<400> 476
   tccaactcct agtggctcta tagcgctgta gccaataagg c 41
<210> 477
   <211> 19
   <212> DNA
   <213> Human papillomavirus
<400> 477
   gacgtgagca gatgtttgt 19
<210> 478
   <211> 42
   <212> DNA
   <213> Human papillomavirus
<400> 478
   ggataactgc agtattaccg gacctagggc tggaaaactt gg 42
<210> 479
   <211> 32
   <212> DNA
   <213> Human papillomavirus
<400> 479
   gttacctcag aatcacaatt atttaataag cc 32
<210> 480
   <211> 28
   <212> DNA
   <213> Human papillomavirus

<400> 480
   cctcagaatc acaattattt aataagcc 28
<210> 481
   <211> 24
   <212> DNA
   <213> Human papillomavirus
<400> 481
   cagtcctcca aaatattgga atcc 24
<210> 482
   <211> 20
   <212> DNA
   <213> Human papillomavirus
<400> 482
   aaccaaattg ccagtcctcc 20
<210> 483
   <211> 50
   <212> DNA
   <213> Human papillomavirus
<400> 483
   taccactcgt agcactaata tgacatatgt cattatctct gcagttagtg 50
<210> 484
   <211> 48
   <212> DNA
   <213> Human papillomavirus
<400> 484
   accactcgta gcactaatat gactatgtca ttatctctgc agttagtg 48
<210> 485
   <211> 49
   <212> DNA
   <213> Human papillomavirus
<400> 485
   accactcgta gcactaatat gacatatgtc attatctctg cagttagtg 49
<210> 486
   <211> 49
   <212> DNA
   <213> Human papillomavirus
<400> 486
   accactcgta gcactaatat gacgcagtta gtgtaatttt gcaaagctg 49
<210> 487
   <211> 50
   <212> DNA
   <213> Human papillomavirus
<400> 487
   taccactcgt agcactaata tgacgcagtt agtgtaattt tgcaaagctg 50
<210> 488
   <211> 25
   <212> DNA
   <213> Human papillomavirus
<400> 488
   gtagtgcatt ttttccaact cctag 25
<210> 489
   <211> 22
   <212> DNA
   <213> Human papillomavirus
<400> 489
   ctgcagttat ccaaagtagt gc 22
<210> 490
   <211> 45
   <212> DNA
   <213> Human papillomavirus
<400> 490
   ctgattgccc cagcaaatgc cgctctatag ttacctcaga atcac 45
<210> 491
   <211> 42
   <212> DNA
   <213> Human papillomavirus
<400> 491
   ctgattgccc cagcaaatgc cttggctaca gcgtgcacaa gg 42
<210> 492
   <211> 45
   <212> DNA
   <213> Human papillomavirus
<400> 492
   ctgattgccc cagcaaatgc cggctctata gttacctcag aatca 45
<210> 493
   <211> 24
   <212> DNA
   <213> Human papillomavirus
<400> 493
   atgtcattat ctctgcagtt agtg 24
<210> 494
   <211> 48
   <212> DNA
   <213> Human papillomavirus
<400> 494
   accactcgta gcactaatat gacttcttca acatgacgta catattcc 48
<210> 495
   <211> 20
   <212> DNA
   <213> Human papillomavirus
<400> 495
   ctcaccagtg ggaggtttac 20
<210> 496
   <211> 48
   <212> DNA
   <213> Human papillomavirus
<400> 496
   ctgaaaccag taacagatat cccggccaat taaacataat tgtgtttg 48
<210> 497
   <211> 44
   <212> DNA
   <213> Human papillomavirus
<400> 497
   tgaaaccagt aacagatatc ccgtccatag ataagcattc cctg 44
<210> 498
   <211> 44
   <212> DNA
   <213> Human papillomavirus
<400> 498
   tgaaaccagt aacagatatc ccgaatccat agataagcat tccc 44
<210> 499
   <211> 22
   <212> DNA
   <213> Human papillomavirus
<400> 499
   taaccctgat acacaacgtt tg 22
<210> 500
   <211> 43
   <212> DNA
   <213> Human papillomavirus
<400> 500
   aggatgacca cttacgccaa cactctgggc atgtgtaggc ctt 43
<210> 501
   <211> 20
   <212> DNA
   <213> Human papillomavirus
<400> 501
   caatggctgt cccctaccta 20
<210> 502
   <211> 22
   <212> DNA
   <213> Human papillomavirus
<400> 502
   atggctgtcc cctacctatt tc 22

<210> 503
   <211> 19
   <212> DNA
   <213> Human papillomavirus
<400> 503
   cctttacccc aatgctcac 19
<210> 504
   <211> 39
   <212> DNA
   <213> Human papillomavirus
<400> 504
   agccagggtc tgataacagg gggaggttta cagccaatt 39
<210> 505
   <211> 18
   <212> DNA
   <213> Human papillomavirus
<400> 505
   aataggtagg ggacagcc 18
<210> 506
   <211> 42
   <212> DNA
   <213> Human papillomavirus
<400> 506
   gcgggatatc tgttactggt ttcagcgtaa gtggtcatcc tt 42
<210> 507
   <211> 20
   <212> DNA
   <213> Human papillomavirus
<400> 507
   ctcaccagtg ggaggtttac 20
<210> 508
   <211> 48
   <212> DNA
   <213> Human papillomavirus
<400> 508
   ctgaaaccag taacagatat cccggccaat taaacataat tgtgtttg 48
<210> 509
   <211> 22
   <212> DNA
   <213> Human papillomavirus
<400> 509
   taaccctgat acacaacgtt tg 22
<210> 510
   <211> 43
   <212> DNA
   <213> Human papillomavirus
<400> 510
   aggatgacca cttacgccaa cactctgggc atgtgtaggc ctt 43
<210> 511
   <211> 25
   <212> DNA
   <213> Human papillomavirus
<400> 511
   ccaatcctcc aaaatagtgg tattc 25
<210> 512
   <211> 48
   <212> DNA
   <213> Human papillomavirus
<400> 512
   ctactcgcag caccaatctt tcgtatgaca ttacctctgt agttaatg 48
<210> 513
   <211> 46
   <212> DNA
   <213> Human papillomavirus
<400> 513
   ctactcgcag caccaatctt tcgaatgtat gacattacct ctgtag 46
<210> 514
   <211> 47
   <212> DNA
   <213> Human papillomavirus
<400> 514
   ctactcgcag caccaatctt tctgaatgta tgacattacc tctgtag 47
<210> 515
   <211> 49
   <212> DNA
   <213> Human papillomavirus
<400> 515
   acaattgatt gtgccaacat ataccggtct gtggttactt ctgattcac 49
<210> 516
   <211> 21
   <212> DNA
   <213> Human papillomavirus
<400> 516
   tatattcccc ttccccaagt g 21
<210> 517
   <211> 48
   <212> DNA
   <213> Human papillomavirus
<400> 517
   caattgattg tgccaacata taccggtctg tggttacttc tgattcac 48
<210> 518
   <211> 46
   <212> DNA
   <213> Human papillomavirus
<400> 518
   ttaaaccctg agccttgtgc agggtctgtg gttacttctg attcac 46
<210> 519
   <211> 26
   <212> DNA
   <213> Human papillomavirus
<400> 519
   gtatgacatt acctctgtag ttaatg 26
<210> 520
<211> 24
<212> DNA
   <213> Human papillomavirus
<400> 520
   gaatgtatga cattacctct gtag 24
<210> 521
   <211> 25
   <212> DNA
   <213> Human papillomavirus
<400> 521
   tgaatgtatg acattacctc tgtag 25
<210> 522
   <211> 44
   <212> DNA
   <213> Human papillomavirus
<400> 522
   ctactcgcag caccaatctt tccctccaca tgtctggcat attc 44
<210> 523
   <211> 24
   <212> DNA
   <213> Human papillomavirus
<400> 523
   gcaggtacac agccaataat acac 24
<210> 524
   <211> 50
   <212> DNA
   <213> Human papillomavirus
<400> 524
   gatgacactg aaaactctca tgtagcgctg agtttgttta taatccacag 50
<210> 525
   <211> 45
   <212> DNA
   <213> Human papillomavirus

<400> 525
   gatgacactg aaaactctca tgctgagttt gtttataatc cacag 45
<210> 526
   <211> 27
   <212> DNA
   <213> Human papillomavirus
<400> 526
   ccagataaca cagtatatga tcctaac 27
<210> 527
   <211> 43
   <212> DNA
   <213> Human papillomavirus
<400> 527
   cactgagtcc tacccctaaa ggttgtctca acgcttggtc tgg 43
<210> 528
   <211> 40
   <212> DNA
   <213> Human papillomavirus
<400> 528
   cactgagtcc tacccctaaa ggtctcaacg cttggtctgg 40
<210> 529
   <211> 25
   <212> DNA
   <213> Human papillomavirus
<400> 529
   gctgttgata ccaaagatac acgtg 25
<210> 530
   <211> 27
   <212> DNA
   <213> Human papillomavirus
<400> 530
   ctgttgatac caaagataca cgtgata 27
<210> 531
   <211> 25
   <212> DNA
   <213> Human papillomavirus
<400> 531
   gataccaaag atacacgtga taatg 25
<210> 532
   <211> 26
   <212> DNA
   <213> Human papillomavirus
<400> 532
   gcatctgctg ttgataccaa agatac 26
<210> 533
   <211> 21
   <212> DNA
   <213> Human papillomavirus
<400> 533
   gatttcaaca cctacacagg c 21
<210> 534
   <211> 24
   <212> DNA
   <213> Human papillomavirus
<400> 534
   gcaggtacac agccaataat acac 24
<210> 535
   <211> 50
   <212> DNA
   <213> Human papillomavirus
<400> 535
   gatgacactg aaaactctca tgtagcgctg agtttgttta taatccacag 50
<210> 536
   <211> 27
   <212> DNA
   <213> Human papillomavirus
<400> 536
   ccagataaca cagtatatga tcctaac 27
<210> 537
   <211> 43
   <212> DNA
   <213> Human papillomavirus
<400> 537
   cactgagtcc tacccctaaa ggttgtctca acgcttggtc tgg 43
<210> 538
   <211> 18
   <212> DNA
   <213> Human papillomavirus
<400> 538
   gtgccttgtg cagccaat 18
<210> 539
   <211> 50
   <212> DNA
   <213> Human papillomavirus
<400> 539
   gtagttatgt atatgccccc tcgccgcttg ttaaataact gggagtctga 50
<210> 540
   <211> 24
   <212> DNA
   <213> Human papillomavirus
<400> 540
   acgtagggaa cagttatttg ctag 24
<210> 541
   <211> 44
   <212> DNA
   <213> Human papillomavirus
<400> 541
   tgtcacgtat gtcagtgccc ttaatggaat agagggggca tggt 44
<210> 542
   <211> 18
   <212> DNA
   <213> Human papillomavirus
<400> 542
   aatggcagga acacagcc 18
<210> 543
   <211> 50
   <212> DNA
   <213> Human papillomavirus
<400> 543
   tgaaaattcc ccgttttcct ccaacgcgtt tgtttatagt ccactgaaac 50
<210> 544
   <211> 21
   <212> DNA
   <213> Human papillomavirus
<400> 544
   tgatacgcag cgattggtat g 21
<210> 545
   <211> 39
   <212> DNA
   <213> Human papillomavirus
<400> 545
   tgcccactaa ggccaacacc ggcctgtgtt ggtgttgaa 39
<210> 546
   <211> 25
   <212> DNA
   <213> Human papillomavirus
<400> 546
   gttaaataac tgggagtctg aggat 25
<210> 547
   <211> 44
   <212> DNA
   <213> Human papillomavirus
<400> 547
   taagggcact gacatacgtg acagccatag acccactagg cgag 44

<210> 548
   <211> 24
   <212> DNA
   <213> Human papillomavirus
<400> 548
   ctgcagatgt atatggagac agta 24
<210> 549
   <211> 42
   <212> DNA
   <213> Human papillomavirus
<400> 549
   agtgtcccct accatgcccc acgtagggaa cagttatttg ct 42
<210> 550
   <211> 18
   <212> DNA
   <213> Human papillomavirus
<400> 550
   tgtgtccctg tgccttgt 18
<210> 551
   <211> 44
   <212> DNA
   <213> Human papillomavirus
<400> 551
   gtagttatgt atatgccccc tcgccgcagc caatagggct tgtt 44
<210> 552
   <211> 54
   <212> DNA
   <213> Human papillomavirus
<400> 552
   cgcagtacca attttacttt gtctactact acctcaacat gcctaatata ttcc 54
<210> 553
   <211> 23
   <212> DNA
   <213> Human papillomavirus
<400> 553
   actgacatac gtgacagtcc tag 23
<210> 554
   <211> 48
   <212> DNA
   <213> Human papillomavirus
<400> 554
   gattatgcca acaaatacca ttgttgttcc cagttattta acaagccc 48
<210> 555
   <211> 47
   <212> DNA
   <213> Human papillomavirus
<400> 555
   cagtaagaaa taattgatta tgccaacaaa caagccctat tggctgc 47
<210> 556
   <211> 49
   <212> DNA
   <213> Human papillomavirus
<400> 556
   ccactcgcag taccaatttt actttgcctc aacatgccta atatattcc 49
<210> 557
   <211> 48
   <212> DNA
   <213> Human papillomavirus
<400> 557
   ataccactcg cagtaccaat tttaccctca acatgcctaa tatattcc 48
<210> 558
   <211> 24
   <212> DNA
   <213> Human papillomavirus
<400> 558
   tcagtggaca atgttatagt acac 24
<210> 559
   <211> 49
   <212> DNA
   <213> Human papillomavirus
<400> 559
   ccactcgcag tacaaatttt actttgcctc aacatgccta atatattcc 49
<210> 560
   <211> 48
   <212> DNA
   <213> Human papillomavirus
<400> 560
   ataccactcg cagtacaaat tttaccctca acatgcctaa tatattcc 48
<210> 561
   <211> 26
   <212> DNA
   <213> Human papillomavirus
<400> 561
   catcagtgga taatgttata gtacac 26
<210> 562
   <211> 48
   <212> DNA
   <213> Human papillomavirus
<400> 562
   gcgcagtact aattttacat tgtcccctca acatgcctaa catattcc 48
<210> 563
   <211> 48
   <212> DNA
   <213> Human papillomavirus
<400> 563
   atacaacgcg cagtactaat tttaccctca acatgcctaa catattcc 48
<210> 564
   <211> 20
   <212> DNA
   <213> Human papillomavirus
<400> 564
   gatttacctt tggcccagtg 20
<210> 565
   <211> 49
   <212> DNA
   <213> Human papillomavirus
<400> 565
   tagatgatac tgaaaattcc ccgttgccta taatacatag ttgcgtttg 49
<210> 566
   <211> 26
   <212> DNA
   <213> Human papillomavirus
<400> 566
   cctgagtcta cattatataa ccctga 26
<210> 567
   <211> 42
   <212> DNA
   <213> Human papillomavirus
<400> 567
   cccactaagg ccaacaccta atgtacgcag cgattggtat gg 42
<210> 568
   <211> 25
   <212> DNA
   <213> Human papillomavirus
<400> 568
   cagctgattc agtagtagta gacaa 25
<210> 569
   <211> 43
   <212> DNA
   <213> Human papillomavirus
<400> 569
   ggcacaggga cacaacaatg gtaaattggt actgcgagtg gta 43 <210> 570
   <211> 22
   <212> DNA
   <213> Human papillomavirus

<400> 570
   tgacatacgt gacagtccta gt 22
<210> 571
   <211> 44
   <212> DNA
   <213> Human papillomavirus
<400> 571
   ttgtgcagcc aatagggctt gttaagtata tgccccctcg ccta 44
<210> 572
   <211> 23
   <212> DNA
   <213> Human papillomavirus
<400> 572
   ctctattcca aaaatgccta gca 23
<210> 573
   <211> 26
   <212> DNA
   <213> Human papillomavirus
<400> 573
   cctagtagtt atgtatatgc cccctc 26
<210> 574
   <211> 27
   <212> DNA
   <213> Human papillomavirus
<400> 574
   tacaattcag taggtatagt gtcccct 27
<210> 575
   <211> 25
   <212> DNA
   <213> Human papillomavirus
<400> 575
   gtgggtctat ggtatcctca gactc 25
<210> 576
   <211> 52
   <212> DNA
   <213> Human papillomavirus
<400> 576
   ccactcgcag taccaatttt actttgcatc agtggacaat gttatagtac ac 52
<210> 577
   <211> 50
   <212> DNA
   <213> Human papillomavirus
<400> 577
   taccactcgc agtaccaatt ttaccatcag tggacaatgt tatagtacac 50
<210> 578
   <211> 43
   <212> DNA
   <213> Human papillomavirus
<400> 578
   ccattgttgt gtccctgtgc ctctatggta tcctcagact ccc 43
<210> 579
   <211> 46
   <212> DNA
   <213> Human papillomavirus
<400> 579
   caacaaatac cattgttgtg tccctctatg gtatcctcag actccc 46
<210> 580
   <211> 45
   <212> DNA
   <2₁3> Human papillomavirus .
<400> 580
   cctccaacaa aaatcctaag gacagccaat ggcaggaaca cagcc 45
<210> 581
   <211> 45
   <212> DNA
   <213> Human papillomavirus
<400> 581
   ctccaacaaa aatcctaagg acagccaatg gcaggaacac agcct 45
<210> 582
   <211> 21
   <212> DNA
   <213> Human papillomavirus
<400> 582
   gatttacctt tggcccagtg c 21
<210> 583
   <211> 46
   <212> DNA
   <213> Human papillomavirus
<400> 583
   tataatggat gcccactaag gccgcctgtg ttggtgttga aatagg 46
<210> 584
   <211> 20
   <212> DNA
   <213> Human papillomavirus
<400> 584
   accctgatac gcagcgattg 20
<210> 585
   <211> 20
   <212> DNA
   <213> Human papillomavirus
<400> 585
   gttatgtata tgccccctcg 20
<210> 586
   <211> 52
   <212> DNA
   <213> Human papillomavirus
<400> 586
   caacgcgcag tactaatttt acattgcatc agtggataat gttatagtac ac 52
<210> 587
   <211> 50
   <212> DNA
   <213> Human papillomavirus
<400> 587
   tacaacgcgc agtactaatt ttaccatcag tggataatgt tatagtacac 50
<210> 588
   <211> 20
   <212> DNA
   <213> Human papillomavirus
<400> 588
   gttatgtgta tgccccctcg 20
<210> 589
   <211> 46
   <212> DNA
   <213> Human papillomavirus
<400> 589
   caacaaatac cattgttgtg tccctctatg gtgtcctctg actccc 46
<210> 590
   <211> 25
   <212> DNA
   <213> Human papillomavirus
<400> 590
   ccaatcatcc aaaatagcag gattc 25
<210> 591
   <211> 49
   <212> DNA
   <213> Human papillomavirus
<400> 591
   tagatgatac tgaaaattcc ccgttgccta taatacatag ttgcgtttg 49
<210> 592
   <211> 26
   <212> DNA
   <213> Human papillomavirus
<400> 592
   cctgagtcta cattatataa ccctga 26

<210> 593
   <211> 42
   <212> DNA
   <213> Human papillomavirus
<400> 593
   cccactaagg ccaacaccta atgtacgcag cgattggtat gg 42
<210> 594
   <211> 20
   <212> DNA
   <213> Human papillomavirus
<400> 594
   gatttacctt tggcccagtg 20
<210> 595
   <211> 33
   <212> DNA
   <213> Human papillomavirus
<400> 595
   attatgtgct gccatatcta cttcagaaac tac 33
<210> 596
   <211> 32
   <212> DNA
   <213> Human papillomavirus
<400> 596
   aaccaataag gtttattgaa tatttgggca tc 32
<210> 597
   <211> 34
   <212> DNA
   <213> Human papillomavirus
<400> 597
   accaataagg tttattgaat atttgggcat caga 34
<210> 598
   <211> 31
   <212> DNA
   <213> Human papillomavirus
<400> 598
   caataaggtt tattgaatat ttgggcatca g 31
<210> 599
   <211> 31
   <212> DNA
   <213> Human papillomavirus
<400> 599
   ataaggttta ttgaatattt gggcatcaga g 31
<210> 600
   <211> 30
   <212> DNA
   <213> Human papillomavirus
<400> 600
   aaggtttatt gaatatttgg gcatcagagg 30
<210> 601
   <211> 30
   <212> DNA
   <213> Human papillomavirus
<400> 601
   aaatgcaggt gtggataata gagaatgtat 30
<210> 602
   <211> 29
   <212> DNA
   <213> Human papillomavirus
<400> 602
   aaatgcaggt gtggataata gagaatgta 29
<210> 603
   <211> 30
   <212> DNA
   <213> Human papillomavirus
<400> 603
   attatgtgct gccatatcta cttcagaaac 30
<210> 604
   <211> 30
   <212> DNA
   <213> Human papillomavirus
<400> 604
   gtgctgccat atctacttca gaaactacat 30
<210> 605
   <211> 34
   <212> DNA
   <213> Human papillomavirus
<400> 605
   tatgtgctgc catatctact tcagaaacta cata 34
<210> 606
   <211> 35
   <212> DNA
   <213> Human papillomavirus
<400> 606
   acttcagaaa ctacatataa aaatactaac tttaa 35
<210> 607
   <211> 30
   <212> DNA
   <213> Human papillomavirus
<400> 607
   ttatgtgctg ccatatctac ttcagaaact 30
<210> 608
   <211> 34
   <212> DNA
   <213> Human papillomavirus
<400> 608
   ctacttcaga aactacatat aaaaatacta actt 34
<210> 609
   <211> 36
   <212> DNA
   <213> Human papillomavirus
<400> 609
   tcagaaacta catataaaaa tactaacttt aaggag 36
<210> 610
   <211> 37
   <212> DNA
   <213> Human papillomavirus
<400> 610
   aactacatat aaaaatacta actttaagga gtaccta 37
<210> 611
   <211> 38
   <212> DNA
   <213> Human papillomavirus
<400> 611
   atgtgctgcc atatctactt cagaaactac atataaaa 38
<210> 612
   <211> 37
   <212> DNA
   <213> Human papillomavirus
<400> 612
   tgccatatct acttcagaaa ctacatataa aaatact 37
<210> 613
   <211> 31
   <212> DNA
   <213> Human papillomavirus
<400> 613
   tctacacagt ctcctgtacc tgggcaatat g 31
<210> 614
   <211> 31
   <212> DNA
   <213> Human papillomavirus
<400> 614
   aatatgtgct tctacacagt ctcctgtacc t 31

<210> 615
   <211> 31
   <212> DNA
   <213> Human papillomavirus
<400> 615
   ctcctgtacc tgggcaatat gatgctacca a 31
<210> 616
   <211> 30
   <212> DNA
   <213> Human papillomavirus
<400> 616
   cacgtctaat gtttctgagg acgttaggga 30
<210> 617
   <211> 27
   <212> DNA
   <213> Human papillomavirus
<400> 617
   gtctaatgtt tctgaggacg ttaggga 27
<210> 618
   <211> 24
   <212> DNA
   <213> Human papillomavirus
<400> 618
   taatgtttct gaggacgtta ggga 24
<210> 619
   <211> 31
   <212> DNA
   <213> Human papillomavirus
<400> 619
   taatgtttct gaggacgtta gggacaatgt g 31
<210> 620
   <211> 29
   <212> DNA
   <213> Human papillomavirus
<400> 620
   taatgtttct gaggacgtta gggacaatg 29
<210> 621
   <211> 30
   <212> DNA
   <213> Human papillomavirus
<400> 621
   aatgtttctg aggacgttag ggacaatgtg 30
<210> 622
   <211> 30
   <212> DNA
   <213> Human papillomavirus
<400> 622
   aatatgtgct tctacacagt ctcctgtacc 30
<210> 623
   <211> 30
   <212> DNA
   <213> Human papillomavirus
<400> 623
   tgcttctaca cagtctcctg tacctgggca 30
<210> 624
   <211> 30
   <212> DNA
   <213> Human papillomavirus
<400> 624
   tgcttctaca cagtctcctg tacctgggca 30
<210> 625
   <211> 30
   <212> DNA
   <213> Human papillomavirus
<400> 625
   acctgggcaa tatgatgcta ccaaatttaa 30
<210> 626
   <211> 35
   <212> DNA
   <213> Human papillomavirus
<400> 626
   cctgtacctg ggcaatatga tgctaccaaa tttaa 35
<210> 627
   <211> 38
   <212> DNA
   <213> Human papillomavirus
<400> 627
   tggtcctggc actgataata gggaatgtat atcaatgg 38
<210> 628
   <211> 39
   <212> DNA
   <213> Human papillomavirus
<400> 628
   ataataggga atgtatatca atggattata aacaaacac 39
<210> 629
   <211> 39
   <212> DNA
   <213> Human papillomavirus
<400> 629
   ggcactgata atagggaatg tatatcaatg gattataaa 39
<210> 630
   <211> 30
   <212> DNA
   <213> Human papillomavirus
<400> 630
   tggtcctggc actgataata gggaatgtat 30
<210> 631
   <211> 32
   <212> DNA
   <213> Human papillomavirus
<400> 631
   ataataggga atgtatatca atggattata aa 32
<210> 632
   <211> 33
   <212> DNA
   <213> Human papillomavirus
<400> 632
   ataataggga atgtatatca atggattata aac 33
<210> 633
   <211> 37
   <212> DNA
   <213> Human papillomavirus
<400> 633
   ataataggga atgtatatca atggattata aacaaac 37
<210> 634
   <211> 30
   <212> DNA
   <213> Human papillomavirus
<400> 634
   tgctgcaatt gcaaacagtg atactacatt 30
<210> 635
   <211> 35
   <212> DNA
   <213> Human papillomavirus
<400> 635
   aacagtgata ctacatttaa aagtagtaat tttaa 35
<210> 636
   <211> 35
   <212> DNA
   <213> Human papillomavirus
<400> 636
   ttatccatgg attataaaca aacacagtta tgttt 35
<210> 637
   <211> 37
   <212> DNA
   <213> Human papillomavirus

<400> 637
   ttatccatgg attataaaca aacacagtta tgtttac 37
<210> 638
   <211> 42
   <212> DNA
   <213> Human papillomavirus
<400> 638
   ttatccatgg attataaaca aacacagtta tgtttacttg ga 42
<210> 639
   <211> 30
   <212> DNA
   <213> Human papillomavirus
<400> 639
   acaaggtcat aataatggta tttgttgggg 30
<210> 640
   <211> 30
   <212> DNA
   <213> Human papillomavirus
<400> 640
   gtagttcctg aacctttaat gtacaggtca 30
<210> 641
   <211> 30
   <212> DNA
   <213> Human papillomavirus
<400> 641
   caaggtcata ataatggtat ttgttggggc 30
<210> 642
   <211> 35
   <212> DNA
   <213> Human papillomavirus
<400> 642
   atgtttatcc atggattata aacaaacaca gttat 35
<210> 643
   <211> 30
   <212> DNA
   <213> Human papillomavirus
<400> 643
   ttatccatgg attataaaca aacacagtta 30
<210> 644
   <211> 33
   <212> DNA
   <213> Human papillomavirus
<400> 644
   ttatccatgg attataaaca aacacagtta tgt 33
<210> 645
   <211> 30
   <212> DNA
   <213> Human papillomavirus
<400> 645
   tggacaaccg ggtgctgata atagggaatg 30
<210> 646
   <211> 23
   <212> DNA
   <213> Human papillomavirus
<400> 646
   tggacaaccg ggtgctgata ata 23
<210> 647
   <211> 36
   <212> DNA
   <213> Human papillomavirus
<400> 647
   gataataggg aatgtttatc catggattat aaacaa 36
<210> 648
   <211> 34
   <212> DNA
   <213> Human papillomavirus
<400> 648
   agggaatgtt tatccatgga ttataaacaa acac 34
<210> 649
   <211> 30
   <212> DNA
   <213> Human papillomavirus
<400> 649
   agggaatgtt tatccatgga ttataaacaa 30
<210> 650
   <211> 33
   <212> DNA
   <213> Human papillomavirus
<400> 650
   aatgtttatc catggattat aaacaaacac agt 33
<210> 651
   <211> 30
   <212> DNA
   <213> Human papillomavirus
<400> 651
   actttatgca cacaagtaac tagtgacagt 30
<210> 652
   <211> 35
   <212> DNA
   <213> Human papillomavirus
<400> 652
   actagtgaca gtacatataa aaatgaaaat tttaa 35
<210> 653
   <211> 31
   <212> DNA
   <213> Human papillomavirus
<400> 653
   atggatttta ctacattaca agctaataaa a 31
<210> 654
   <211> 34
   <212> DNA
   <213> Human papillomavirus
<400> 654
   tttggtgcaa tggattttac tacattacaa gcta 34
<210> 655
   <211> 33
   <212> DNA
   <213> Human papillomavirus
<400> 655
   gtgcaatgga ttttactaca ttacaagcta ata 33
<210> 656
   <211> 29
   <212> DNA
   <213> Human papillomavirus
<400> 656
   ataacaggga atgcatttct atggattat 29
<210> 657
   <211> 30
   <212> DNA
   <213> Human papillomavirus
<400> 657
   ttctgctgtg tcttctagtg acagtacata 30
<210> 658
   <211> 35
   <212> DNA
   <213> Human papillomavirus
<400> 658
   tctagtgaca gtacatataa aaatgacaat tttaa 35
<210> 659
   <211> 36
   <212> DNA
   <213> Human papillomavirus
<400> 659
   tctatagagt cttccatacc ttctacatat gatcct 36

<210> 660
   <211> 38
   <212> DNA
   <213> Human papillomavirus
<400> 660
   tgggccttat gtagccaata aggcttatta aataactg 38
<210> 661
   <211> 35
   <212> DNA
   <213> Human papillomavirus
<400> 661
   gccaataagg cttattaaat aactgggaat cagag 35
<210> 662
   <211> 30
   <212> DNA
   <213> Human papillomavirus
<400> 662
   caataaggct tattaaataa ctgggaatca 30
<210> 663
   <211> 31
   <212> DNA
   <213> Human papillomavirus
<400> 663
   aataaggctt attaaataac tgggaatcag a 31
<210> 664
   <211> 32
   <212> DNA
   <213> Human papillomavirus
<400> 664
   tcttccatac cttctacata tgatccttct aa 32
<210> 665
   <211> 40
   <212> DNA
   <213> Human papillomavirus
<400> 665
   tccatacctt ctacatatga tccttctaag tttaaggaat 40
<210> 666
   <211> 36
   <212> DNA
   <213> Human papillomavirus
<400> 666
   ttatctacct ctatagagtc ttccatacct tctaca 36
<210> 667
   <211> 33
   <212> DNA
   <213> Human papillomavirus
<400> 667
   ataccttcta catatgatcc ttctaagttt aag 33
<210> 668
   <211> 37
   <212> DNA
   <213> Human papillomavirus
<400> 668
   ttctacatat gatccttcta agtttaagga atatacc 37
<210> 669
   <211> 34
   <212> DNA
   <213> Human papillomavirus
<400> 669
   tatgtagcca ataaggctta ttaaataact ggga 34
<210> 670
   <211> 30
   <212> DNA
   <213> Human papillomavirus
<400> 670
   accaataagg acagtaggga taatgtgtct 30
<210> 671
   <211> 28
   <212> DNA
   <213> Human papillomavirus
<400> 671
   accaataagg acagtaggga taatgtgt 28
<210> 672
   <211> 27
   <212> DNA
   <213> Human papillomavirus
<400> 672
   accaataagg acagtaggga taatgtg 27
<210> 673
   <211> 30
   <212> DNA
   <213> Human papillomavirus
<400> 673
   cctctataga gtcttccata ccttctacat 30
<210> 674
   <211> 35
   <212> DNA
   <213> Human papillomavirus
<400> 674
   tccatacctt ctacatatga tccttctaag tttaa 35
<210> 675
   <211> 30
   <212> DNA
   <213> Human papillomavirus
<400> 675
   gttattacgc aggatgttag ggataatgtg 30
<210> 676
   <211> 31
   <212> DNA
   <213> Human papillomavirus
<400> 676
   agctacagct gttattacgc aggatgttag g 31
<210> 677
   <211> 33
   <212> DNA
   <213> Human papillomavirus
<400> 677
   acgcaggatg ttagggataa tgtgtcagtt gat 33
<210> 678
   <211> 40
   <212> DNA
   <213> Human papillomavirus
<400> 678
   ctacagctgt tattacgcag gatgttaggg ataatgtgtc 40
<210> 679
   <211> 30
   <212> DNA
   <213> Human papillomavirus
<400> 679
   acagctgtta ttacgcagga tgttagggat 30
<210> 680
   <211> 30
   <212> DNA
   <213> Human papillomavirus
<400> 680
   tgttattacg caggatgtta gggataatgt 30
<210> 681
   <211> 30
   <212> DNA
   <213> Human papillomavirus
<400> 681
   ttacgcagga tgttagggat aatgtgtcag 30
<210> 682
   <211> 30
   <212> DNA
   <213> Human papillomavirus

<400> 682
   tacacaaaat cctgtgccaa gtacatatga 30
<210> 683
   <211> 35
   <212> DNA
   <213> Human papillomavirus
<400> 683
   cctgtgccaa gtacatatga ccctactaag tttaa 35
<210> 684
   <211> 38
   <212> DNA
   <213> Human papillomavirus
<400> 684
   gacaacaaac agactcagtt atgtataata ggctgtgc 38
<210> 685
   <211> 32
   <212> DNA
   <213> Human papillomavirus
<400> 685
   tcatcatatt tattaaataa gggatgacca ct 32
<210> 686
   <211> 34
   <212> DNA
   <213> Human papillomavirus
<400> 686
   acatctgttg acaacaaaca gactcagtta tgta 34
<210> 687
   <211> 34
   <212> DNA
   <213> Human papillomavirus
<400> 687
   atcatattta ttaaataagg gatgaccact aagg 34
<210> 688
   <211> 33
   <212> DNA
   <213> Human papillomavirus
<400> 688
   tgttgacaac aaacagactc agttatgtat aat 33
<210> 689
   <211> 33
   <212> DNA
   <213> Human papillomavirus
<400> 689
   tatttattaa ataagggatg accactaagg cca 33
<210> 690
   <211> 36
   <212> DNA
   <213> Human papillomavirus
<400> 690
   ttgacaacaa acagactcag ttatgtataa taggct 36
<210> 691
   <211> 33
   <212> DNA
   <213> Human papillomavirus
<400> 691
   gcggtttccc caacatttac tccaagtaac ttt 33
<210> 692
   <211> 31
   <212> DNA
   <213> Human papillomavirus
<400> 692
   tccccaacat ttactccaag taactttaag c 31
<210> 693
   <211> 31
   <212> DNA
   <213> Human papillomavirus
<400> 693
   aaacagactc agttatgtat aataggctgt g 31
<210> 694
   <211> 30
   <212> DNA
   <213> Human papillomavirus
<400> 694
   cagactcagt tatgtataat aggctgtgct 30
<210> 695
   <211> 38
   <212> DNA
   <213> Human papillomavirus
<400> 695
   tctgttgaca acaaacagac tcagttatgt ataatagg 38
<210> 696
   <211> 35
   <212> DNA
   <213> Human papillomavirus
<400> 696
   tctgttgaca acaaacagac tcagttatgt ataat 35
<210> 697
   <211> 35
   <212> DNA
   <213> Human papillomavirus
<400> 697
   gttgacaaca aacagactca gttatgtata atagg 35
<210> 698
   <211> 36
   <212> DNA
   <213> Human papillomavirus
<400> 698
   tgttgacaac aaacagactc agttatgtat aatagg 36
<210> 699
   <211> 40
   <212> DNA
   <213> Human papillomavirus
<400> 699
   tctgttgaca acaaacagac tcagttatgt ataataggct 40
<210> 700
   <211> 30
   <212> DNA
   <213> Human papillomavirus
<400> 700
   gctgcggttt ccccaacatt tactccaagt 30
<210> 701
   <211> 30
   <212> DNA
   <213> Human papillomavirus
<400> 701
   gcggtttccc caacatttac tccaagtaac 30
<210> 702
   <211> 35
   <212> DNA
   <213> Human papillomavirus
<400> 702
   gcggtttccc caacatttac tccaagtaac tttaa 35
<210> 703
   <211> 38
   <212> DNA
   <213> Human papillomavirus
<400> 703
   taatggcata tgttggggca atcagttgtt tgtcacag 38
<210> 704
   <211> 30
   <212> DNA
   <213> Human papillomavirus
<400> 704
   actaggagta ggaaaaaaag cactgctttg 30

<210> 705
   <211> 39
   <212> DNA
   <213> Human papillomavirus
<400> 705
   gggccacaat aatggcatat gttggggcaa tcagttgtt 39
<210> 706
   <211> 32
   <212> DNA
   <213> Human papillomavirus
<400> 706
   tatgttgggg caatcagttg tttgtcacag tt 32
<210> 707
   <211> 30
   <212> DNA
   <213> Human papillomavirus
<400> 707
   taggagtagg aaaaaaagca ctgctttgta 30
<210> 708
   <211> 31
   <212> DNA
   <213> Human papillomavirus
<400> 708
   cacaataatg gcatatgttg gggcaatcag t 31
<210> 709
   <211> 37
   <212> DNA
   <213> Human papillomavirus
<400> 709
   acaataatgg catatgttgg ggcaatcagt tgtttgt 37
<210> 710
   <211> 30
   <212> DNA
   <213> Human papillomavirus
<400> 710
   tacggtttat taaataattg ggattctgag 30
<210> 711
   <211> 40
   <212> DNA
   <213> Human papillomavirus
<400> 711
   gaggttaaaa aggaaagcac atataaaaat gaaaatttta 40
<210> 712
   <211> 31
   <212> DNA
   <213> Human papillomavirus
<400> 712
   tttatgtgct gaggttaaaa aggaaagcac a 31
<210> 713
   <211> 33
   <212> DNA
   <213> Human papillomavirus
<400> 713
   ttgtaaccag tacggtttat taaataattg gga 33
<210> 714
   <211> 33
   <212> DNA
   <213> Human papillomavirus
<400> 714
   gttaaaaagg aaagcacata taaaaatgaa aat 33
<210> 715
   <211> 40
   <212> DNA
   <213> Human papillomavirus
<400> 715
   taaaaaggaa agcacatata aaaatgaaaa ttttaaggaa 40
<210> 716
   <211> 40
   <212> DNA
   <213> Human papillomavirus
<400> 716
   ggaaagcaca tataaaaatg aaaattttaa ggaatacctt 40
<210> 717
   <211> 38
   <212> DNA
   <213> Human papillomavirus
<400> 717
   tgtaaccagt acggtttatt aaataattgg gattctga 38
<210> 718
   <211> 40
   <212> DNA
   <213> Human papillomavirus
<400> 718
   tgtgctgagg ttaaaaagga aagcacatat aaaaatgaaa 40
<210> 719
   <211> 30
   <212> DNA
   <213> Human papillomavirus
<400> 719
   ccagtacggt ttattaaata attgggattc 30
<210> 720
   <211> 31
   <212> DNA
   <213> Human papillomavirus
<400> 720
   ctgaggttaa aaaggaaagc acatataaaa a 31
<210> 721
   <211> 33
   <212> DNA
   <213> Human papillomavirus
<400> 721
   ttaaaaagga aagcacatat aaaaatgaaa aat 33
<210> 722
   <211> 32
   <212> DNA
   <213> Human papillomavirus
<400> 722
   ctgaggttaa aaaggaaagc acatataaaa at 32
<210> 723
   <211> 31
   <212> DNA
   <213> Human papillomavirus
<400> 723
   tgctgaggtt aaaaaggaaa gcacatataa a 31
<210> 724
   <211> 38
   <212> DNA
   <213> Human papillomavirus
<400> 724
   aaaaaggaaa gcacatataa aaatgaaaat tttaagga 38
<210> 725
   <211> 39
   <212> DNA
   <213> Human papillomavirus
<400> 725
   tgctgaggtt aaaaaggaaa gcacatataa aaatgaaaa 39
<210> 726
   <211> 34
   <212> DNA
   <213> Human papillomavirus
<400> 726
   tgctgaggtt aaaaaggaaa gcacatataa aaat 34

<210> 727
   <211> 45
   <212> DNA
   <213> Human papillomavirus
<400> 727
   tttatgtgct gaggttaaaa aggaaagcac atataaaaat gaaaa 45
<210> 728
   <211> 40
   <212> DNA
   <213> Human papillomavirus
<400> 728
   tttatgtgct gaggttaaaa aggaaagcac atataaaaat 40
<210> 729
   <211> 35
   <212> DNA
   <213> Human papillomavirus
<400> 729
   tttatgtgct gaggttaaaa aggaaagcac atata 35
<210> 730
   <211> 30
   <212> DNA
   <213> Human papillomavirus
<400> 730
   ggtaaacctg gtatagataa tagggaatgt 30
<210> 731
   <211> 31
   <212> DNA
   <213> Human papillomavirus
<400> 731
   tggtaaacct ggtatagata atagggaatg t 31
<210> 732
   <211> 30
   <212> DNA
   <213> Human papillomavirus
<400> 732
   tttatgtgct gaggttaaaa aggaaagcac 30
<210> 733
   <211> 40
   <212> DNA
   <213> Human papillomavirus
<400> 733
   ccttgggcac gttgcaacca ataaggttta ttaaataact 40
<210> 734
   <211> 40
   <212> DNA
   <213> Human papillomavirus
<400> 734
   ttagtactgc tacagaacag ttaagtaaat atgatgcacg 40
<210> 735
   <211> 40
   <212> DNA
   <213> Human papillomavirus
<400> 735
   ggcacgttgc aaccaataag gtttattaaa taactgtgcc 40
<210> 736
   <211> 38
   <212> DNA
   <213> Human papillomavirus
<400> 736
   cgttgcaacc aataaggttt attaaataac tgtgcctc 38
<210> 737
   <211> 38
   <212> DNA
   <213> Human papillomavirus
<400> 737
   gctacagaac agttaagtaa atatgatgca cgaaaaat 38
<210> 738
   <211> 38
   <212> DNA
   <213> Human papillomavirus
<400> 738
   ttattatggc cttgggcacg ttgcaaccaa taaggttt 38
<210> 739
   <211> 33
   <212> DNA
   <213> Human papillomavirus
<400> 739
   acagaacagt taagtaaata tgatgcacga aaa 33
<210> 740
   <211> 35
   <212> DNA
   <213> Human papillomavirus
<400> 740
   accaataagg tttattaaat aactgtgcct cagac 35
<210> 741
   <211> 40
   <212> DNA
   <213> Human papillomavirus
<400> 741
   agaacagtta agtaaatatg atgcacgaaa aattaatcag 40
<210> 742
   <211> 40
   <212> DNA
   <213> Human papillomavirus
<400> 742
   taacatgact attagtactg ctacagaaca gttaagtaaa 40
<210> 743
   <211> 37
   <212> DNA
   <213> Human papillomavirus
<400> 743
   gtaaatatga tgcacgaaaa attaatcagt accttag 37
<210> 744
   <211> 40
   <212> DNA
   <213> Human papillomavirus
<400> 744
   tgactattag tactgctaca gaacagttaa gtaaatatga 40
<210> 745
   <211> 31
   <212> DNA
   <213> Human papillomavirus
<400> 745
   gaacagttaa gtaaatatga tgcacgaaaa a 31
<210> 746
   <211> 36
   <212> DNA
   <213> Human papillomavirus
<400> 746
   tactgctaca gaacagttaa gtaaatatga tgcacg 36
<210> 747
   <211> 30
   <212> DNA
   <213> Human papillomavirus
<400> 747
   aataataatg ttatagaaga tagtagggac 30
<210> 748
   <211> 30
   <212> DNA
   <213> Human papillomavirus
<400> 748
   atagtaggga caatatatca gttgatggca 30
<210> 749
   <211> 30
   <212> DNA
   <213> Human papillomavirus

<400> 749
   tattagtact gctacagaac agttaagtaa 30
<210> 750
   <211> 30
   <212> DNA
   <213> Human papillomavirus
<400> 750
   acagaacagt taagtaaata tgatgcacga 30
<210> 751
   <211> 35
   <212> DNA
   <213> Human papillomavirus
<400> 751
   gaacagttaa gtaaatatga tgcacgaaaa attaa 35
<210> 752
   <211> 30
   <212> DNA
   <213> Human papillomavirus
<400> 752
   aggtcatccg ggacagcctc gccaagtttt 30
<210> 753
   <211> 32
   <212> DNA
   <213> Human papillomavirus
<400> 753
   ttacctcaga atcacaatta tttaataagc ct 32
<210> 754
   <211> 32
   <212> DNA
   <213> Human papillomavirus
<400> 754
   ctttaatata aaggtcatcc gggacagcct cg 32
<210> 755
   <211> 30
   <212> DNA
   <213> Human papillomavirus
<400> 755
   cagaatcaca attatttaat aagccttatt 30
<210> 756
   <211> 30
   <212> DNA
   <213> Human papillomavirus
<400> 756
   ccaatggctg tcccctacct atttcaaggc 30
<210> 757
   <211> 31
   <212> DNA
   <213> Human papillomavirus
<400> 757
   cacagccagg gtctgataac agggaatgct t 31
<210> 758
   <211> 30
   <212> DNA
   <213> Human papillomavirus
<400> 758
   tggctgtccc ctacctattt caaggcctac 30
<210> 759
   <211> 30
   <212> DNA
   <213> Human papillomavirus
<400> 759
   gggtctgata acagggaatg cttatctatg 30
<210> 760
   <211> 30
   <212> DNA
   <213> Human papillomavirus
<400> 760
   cacagccagg gtctgataac agggaatgct 30
<210> 761
   <211> 30
   <212> DNA
   <213> Human papillomavirus
<400> 761
   cagggaatgc ttatctatgg attataaaca 30
<210> 762
   <211> 30
   <212> DNA
   <213> Human papillomavirus
<400> 762
   cactgaagta actaaggaag gtacatataa 30
<210> 763
   <211> 30
   <212> DNA
   <213> Human papillomavirus
<400> 763
   attatgcact gaagtaacta aggaaggtac 30
<210> 764
   <211> 35
   <212> DNA
   <213> Human papillomavirus
<400> 764
   actaaggaag gtacatataa aaatgataat tttaa 35
<210> 765
   <211> 32
   <212> DNA
   <213> Human papillomavirus
<400> 765
   tgataccaaa gatacacgtg ataatgtatc tg 32
<210> 766
   <211> 30
   <212> DNA
   <213> Human papillomavirus
<400> 766
   atctgctgtt gataccaaag atacacgtga 30
<210> 767
   <211> 30
   <212> DNA
   <213> Human papillomavirus
<400> 767
   gccccgaccg atttcaacac ctacacaggc 30
<210> 768
   <211> 39
   <212> DNA
   <213> Human papillomavirus
<400> 768
   gccccgaccg atttcaacac ctacacaggc ccagaccaa 39
<210> 769
   <211> 37
   <212> DNA
   <213> Human papillomavirus
<400> 769
   accaaagata cacgtgataa tgtatctgtg gattata 37
<210> 770
   <211> 34
   <212> DNA
   <213> Human papillomavirus
<400> 770
   cccgaccgat ttcaacacct acacaggccc agac 34
<210> 771
   <211> 30
   <212> DNA
   <213> Human papillomavirus
<400> 771
   ctgttgatac caaagataca cgtgataatg 30

<210> 772
   <211> 38
   <212> DNA
   <213> Human papillomavirus
<400> 772
   gttgatacca aagatacacg tgataatgta tctgtgga 38
<210> 773
   <211> 30
   <212> DNA
   <213> Human papillomavirus
<400> 773
   cgatttcaac acctacacag gcccagacca 30
<210> 774
   <211> 30
   <212> DNA
   <213> Human papillomavirus
<400> 774
   atctgctgtt gataccaaag atacacgtga 30
<210> 775
   <211> 28
   <212> DNA
   <213> Human papillomavirus
<400> 775
   ctgctgttga taccaaagat acacgtga 28
<210> 776
   <211> 30
   <212> DNA
   <213> Human papillomavirus
<400> 776
   tctgctgttg ataccaaaga tacacgtgat 30
<210> 777
   <211> 35
   <212> DNA
   <213> Human papillomavirus
<400> 777
   atctgctgtt gataccaaag atacacgtga taatg 35
<210> 778
   <211> 37
   <212> DNA
   <213> Human papillomavirus
<400> 778
   gttgatacca aagatacacg tgataatgta tctgtgg 37
<210> 779
   <211> 40
   <212> DNA
   <213> Human papillomavirus
<400> 779
   tctgctgttg ataccaaaga tacacgtgat aatgtatctg 40
<210> 780
   <211> 30
   <212> DNA
   <213> Human papillomavirus
<400> 780
   tctattccta atgtatacac acctaccagt 30
<210> 781
   <211> 35
   <212> DNA
   <213> Human papillomavirus
<400> 781
   tctattccta atgtatacac acctaccagt tttaa 35
<210> 782
   <211> 31
   <212> DNA
   <213> Human papillomavirus
<400> 782
   tcctagtagt tatgtatatg ccccctcgcc t 31
<210> 783
   <211> 30
   <212> DNA
   <213> Human papillomavirus
<400> 783
   agtagttatg tatatgcccc ctcgcctagt 30
<210> 784
   <211> 30
   <212> DNA
   <213> Human papillomavirus
<400> 784
   agtagttatg tgtatgcccc ctcgcctagc 30
<210> 785
   <211> 31
   <212> DNA
   <213> Human papillomavirus
<400> 785
   tgaatcagct gtaccaaata tttatgatcc t 31
<210> 786
   <211> 31
   <212> DNA
   <213> Human papillomavirus
<400> 786
   agactctact gtaccagctg tgtatgattc t 31
<210> 787
   <211> 30
   <212> DNA
   <213> Human papillomavirus
<400> 787
   ttcctccaac aaaaatccta aggacagtag 30
<210> 788
   <211> 29
   <212> DNA
   <213> Human papillomavirus
<400> 788
   ttcctccaac aaaaatccta aggacagta 29
<210> 789
   <211> 30
   <212> DNA
   <213> Human papillomavirus
<400> 789
   tcctaaggac agtagggaat atgtttcagt 30
<210> 790
   <211> 29
   <212> DNA
   <213> Human papillomavirus
<400> 790
   tcagtggact ataaacaaac gcaactatg 29
<210> 791
   <211> 30
   <212> DNA
   <213> Human papillomavirus
<400> 791
   tccactacta cagactctac tgtaccagct 30
<210> 792
   <211> 35
   <212> DNA
   <213> Human papillomavirus
<400> 792
   gctgtaccaa atatttatga tcctaataaa tttaa 35
<210> 793
   <211> 33
   <212> DNA
   <213> Human papillomavirus
<400> 793
   tttgtctact actactgaat cagctgtacc aaa 33
<210> 794
   <211> 35
   <212> DNA
   <213> Human papillomavirus

<400> 794
   actgtaccag ctgtgtatga ttctaataaa tttaa 35
<210> 795
   <211> 20
   <212> DNA
   <213> Homo sapiens
<400> 795
   tgatggtatg gggccaagag 20
<210> 796
   <211> 45
   <212> DNA
   <213> Homo sapiens
<400> 796
   gaggtctaag tgatgacagc cgctgagggt ttgaagtcca actcc 45
<210> 797
   <211> 46
   <212> DNA
   <213> Homo sapiens
<400> 797
   aatctactcc caggagcagg gctagtgaac acagttgtgt cagaag 46
<210> 798
   <211> 20
   <212> DNA
   <213> Homo sapiens
<400> 798
   gagtcagatg caccatggtg 20
<210> 799
   <211> 46
   <212> DNA
   <213> Homo sapiens
<400> 799
   tgaggtctaa gtgatgacag ccgctgaggg tttgaagtcc aactcc 46
<210> 800
   <211> 46
   <212> DNA
   <213> Homo sapiens
<400> 800
   aatctactcc caggagcagg gaagtgaaca cagttgtgtc agaagc 46
<210> 801
   <211> 20
   <212> DNA
   <213> Homo sapiens
<400> 801
   agggctgagg gtttgaagtc 20
<210> 802
   <211> 45
   <212> DNA
   <213> Homo sapiens
<400> 802
   tgaggtctaa gtgatgacag ccgcaactcc taagccagtg ccaga 45
<210> 803
   <211> 44
   <212> DNA
   <213> Homo sapiens
<400> 803
   ctagggttgg ccaatctact cccaatagat ggctctgccc tgac 44
<210> 804
   <211> 22
   <212> DNA
   <213> Homo sapiens
<400> 804
   tgaacacagt tgtgtcagaa gc 22
<210> 805
   <211> 35
   <212> DNA
   <213> Homo sapiens
<400> 805
   ataaaagtca gggcagagcc atctattgct tac 35
<210> 806
   <211> 30
   <212> DNA
   <213> Homo sapiens
<400> 806
   tcagggcaga gccatctatt gcttacattt 30
<210> 807
   <211> 35
   <212> DNA
   <213> Homo sapiens
<400> 807
   gtcagggcag agccatctat tgcttacatt tgctt 35
<210> 808
   <211> 35
   <212> DNA
   <213> Homo sapiens
<400> 808
   agggcaggag ccagggctgg gcataaaagt caggg 35
<210> 809
   <211> 35
   <212> DNA
   <213> Homo sapiens
<400> 809
   aaagtcaggg cagagccatc tattgcttac atttg 35
<210> 810
   <211> 28
   <212> DNA
   <213> Homo sapiens
<400> 810
   tgggcataaa agtcagggca gagccatc 28
<210> 811
   <211> 35
   <212> DNA
   <213> Homo sapiens
<400> 811
   caggagcagg gagggcagga gccagggctg ggcat 35
<210> 812
   <211> 29
   <212> DNA
   <213> Homo sapiens
<400> 812
   cagggagggc aggagccagg gctgggcat 29
<210> 813
   <211> 27
   <212> DNA
   <213> Homo sapiens
<400> 813
   caggagcagg gagggcagga gccaggg 27
<210> 814
   <211> 25
   <212> DNA
   <213> Homo sapiens
<400> 814
   gactataaac atgctttccg tggca 25